(19) **Europäisches Patentamt**

**European Patent Office**

**Office européen des brevets**

(11) **EP 1 163 363 B1**

(12) **EUROPEAN PATENT SPECIFICATION**

(45) Date of publication and mention
of the grant of the patent:
**19.07.2006 Bulletin 2006/29**

(51) Int Cl.:
*C12Q 1/25* (2006.01)    *C12Q 1/34* (2006.01)
*C12Q 1/42* (2006.01)    *C07K 14/00* (2006.01)

(21) Application number: **00906471.8**

(22) Date of filing: **25.02.2000**

(86) International application number:
**PCT/GB2000/000666**

(87) International publication number:
**WO 2000/050631 (31.08.2000 Gazette 2000/35)**

(54) **COMPOSITIONS AND METHODS FOR MONITORING THE MODIFICATION OF NATURAL BINDING PARTNERS**

VERFAHREN UND ZUSAMMENSETZUNGEN ZUR ÜBERWACHUNG DER MODIFIKATION VON NATÜRLICHEN BINDUNGSPARTNERS

COMPOSITIONS ET METHODES DE CONTROLE DE LA MODIFICATION DE PARTENAIRES DE LIAISON NATURELS

(84) Designated Contracting States:
**AT BE CH CY DE DK ES FI FR GB GR IE IT LI LU MC NL PT SE**

(30) Priority: **25.02.1999 GB 9904392**
**25.02.1999 GB 9904393**

(43) Date of publication of application:
**19.12.2001 Bulletin 2001/51**

(73) Proprietor: **Cyclacel Limited**
**London SW1Y 4RB (GB)**

(72) Inventors:
 • **COLYER, John**
 **Bardsey LS17 9AN (GB)**
 • **CRAIG, Roger Kingdon**
 **Smallwood,**
 **Cheshire CW11 2XB (GB)**

(74) Representative: **Khoo, Chong-Yee**
**D Young & Co**
**120 Holborn**
**London EC1N 2DY (GB)**

(56) References cited:
EP-A- 0 392 808        WO-A-96/20211
WO-A-98/02571          US-A- 5 496 934

• **ZHOU M.M. ET AL.: "Structural basis for IL-4 receptor phosphopetide recognition by the IRS-1 PTB domain" NATURE STRUCTURAL BIOLOGY, vol. 3, no. 4, April 1996 (1996-04), pages 388-393, XP000916504 cited in the application**
• **L. IU. SMITH ET AL.: "Phosphorylated sites within the binding domains of the 100kDa steroid-binding subunit of glucocorticoid receptors" BIOCHEMISTRY, vol. 28, 1989, pages 4490-4498, XP000916440 US**
• **ZINING W ET AL: "Ligand binding analysis of interleukin-2 receptor complexes using surface plasmon resonance" JOURNAL OF IMMUNOLOGICAL METHODS,NL,ELSEVIER SCIENCE PUBLISHERS B.V.,AMSTERDAM, vol. 183, no. 1, 14 June 1995 (1995-06-14), pages 127-130, XP004021031 ISSN: 0022-1759**
• **DATABASE SCISEARCH [Online] WEERNINK P A O ET AL: "DETERMINATION OF SPECIFIC PROTEIN-KINASE ACTIVITIES USING P-33" retrieved from STN Database accession no. 91998 XP002144626 & JOURNAL OF BIOCHEMICAL AND BIOPHYSICAL METHODS, (11 JAN 1996) VOL. 31, NO. 1-2, PP. 49-57. ISSN: 0165-022X., UNIV UTRECHT HOSP, DEPT HAEMATOL, LAB MED ENZYMOL, POB 85500, 3508 GA UTRECHT, NETHERLANDS (Reprint);UNIV UTRECHT HOSP, DEPT HAEMATOL, LAB MED ENZYMOL, 3508 GA UTRECHT, NETHERLANDS**

**Description**

The invention relates to monitoring of the post-translational modification of a protein.

The post-translational modification of proteins has been known for over 40 years and since then has become a ubiquitous feature of protein structure. The addition of biochemical groups to translated polypeptides has wide-ranging effects on protein stability, protein secondary/tertiary structure, enzyme activity and in more general terms on the regulated homeostasis of cells. Such modifications include, but are not limited to, the addition of a carbohydrate (glycosylation), ADP-ribosyl (ADP ribosylation), fatty acid (prenylation, which includes but is not limited to: myrisoylation and pahnitylation), ubiquitin (ubiquitination) protein phosphorylation and dephosphorylation and sentrin (sentrinization; a ubiquitination-like protein modification).

Phosphorylation is a well-studied example of a post-translational modification of proteins. There are many cases in which polypeptides form higher order tertiary structures with like polypeptides (homo-oligomers) or with unalike polypeptides (hetero-oligomers). In the simplest scenario, two identical polypeptides associate to form an active homodimer. An example of this type of association is the natural association of myosin II molecules in the assembly of myosin into filaments.

The dimerization of myosin II monomers is the initial step in seeding myosin filaments. The initial dimerization is regulated by phosphorylation, the effect of which is to induce a conformational change in myosin II secondary structure resulting in the folded 10S monomer subunit extending to a 6S molecule. This active molecule is able to dimerize and subsequently to form filaments. The involvement of phosphorylation of myosin II in this priming event is somewhat controversial. Although in higher eukaryotes the conformational change is dependant on phosphorylation, in *Ancanthonmoeba,* a lower eukaryote, the post-translational addition of phosphate is not required to effect the initial dimerization step. It is of note that the dimerization domains in myosin 11 of higher eukaryotes contain the sites for phosphorylation and it is probable that phosphorylation in this region is responsible for enabling myosin II to dimerize and subsequently form filaments. In *Dicryostelium* this situation is reversed in that the phosphorylation sites are outside the dimerization domain and phosphorylation at these sites is required to effect the disassembly of myosin filaments. In contrast to both these examples, *Acanthoamoeba* myosin II is phosphorylated in the dimerization domain but this modification is not necessary to enable myosin II monomers to dimerize in this species.

By far the most frequent example of post-translational modification is the addition of phosphate to polypeptides by specific enzymes known as protein kinases. These enzymes have been identified as important regulators of the state of phosphorylation of target proteins and have been implicated as major players in regulating cellular physiology. For example, the cell-division-cycle of the eukaryotic cell is primarily regulated by the state of phosphorylation of specific proteins, the functional state of which is determined by whether or not the protein is phosphorylated. This is determined by the relative activity of protein kinases which add phosphate and protein phosphatases which remove the phosphate moiety from these proteins. Clearly dysfunction of either the kinases or phosphatases may lead to a diseased state. This is best exemplified by the uncontrolled cellular division shown by tumor cells. The regulatory pathway is composed of a large number of genes that interact *in vivo* to regulate the phosphorylation cascade that ultimately determines if a cell is to divide or arrest cell division.

Currently there are several approaches to analysing the state of modification of target proteins *in vivo:*

1. *In vivo* incorporation of labeled (for example, radiolabelled) moieties (e.g., fatty acyl (including, but not limited to, myristoyl and palmityl) sentrin, methyl, actyl, hydroxyl, iodine, flavin, phosphate, ubiquitin or ADP- ribosyls), which are added to target proteins. According to one common procedure, intracellular ATP pools are labeled with $^{32}PO_4$. which is subsequently incorporated into protein. Analysis of modified proteins is typically performed by electrophoresis and autoradiography, with specificity enhanced by immunoprecipitation of proteins of interest prior to electrophoresis.

2. Back-labeling. The incorporation of a labeled (including, but not limited to, with a radioactive and fluorescent label) moiety, such as phosphate (e.g., $^{32}P$) into a protein *in vitro* to estimate the state of modification *in vivo.*

3. Detection of alteration in electrophoretic mobility of modified protein compared with unmodified (e.g., glycosylated or ubiquitinated) protein.

4. Thin=layer chromatography of radiolabelled fatty acids extracted from the protein of interest.

5. Partitioning of protein into detergent-rich or detergent-poor layer by phase separation, and the effects of enzyme treatment of the protein of interest on the partitioning between aqueous and detergent-rich environments.

6. The use of cell-membrane-permeable protein-modifying enzyme inhibitors (e.g., Wortmannin, staurosporine) to block modification of target proteins and comparable inhibitors of the enzymes involved in other forms of protein modification (above), for example phosphorylation of target proteins.

7. Antibody recognition of the modified form of the protein (e.g., using an antibody directed at ubiquitin or carbohydrate epitopes), e.g., by Western blotting, of either 1- or 2- dimensional gels bearing test protein samples, in which phosphorylation is detected using antibodies specific for phosphorylated forms of target proteins.

8. Lectin-protein interaction in Western blot format as an assay of the presence of particular carbohydrate groups (defined by the specificity of the lectin in use).

9. The exploitation of eukaryotic microbial systems to identify mutations in protein-modifying enzymes such as protein kinases and/or protein phosphatases.

These strategies have certain limitations. Monitoring states of modification by pulse or steady-state labelling is merely a descriptive strategy to show which proteins are modified when samples are separated by gel electrophoresis and visualized by autoradiography. This is unsatisfactory, due to the inability to identify many of the proteins that are modified. A degree of specificity is afforded to this technique if it is combined with immunoprecipitation; however, this is of course limited by the availability of antibodies to target proteins. Moreover, only highly-expressed proteins are readily detectable using this technique, which may fail to identify many low-abundance proteins, which are potentially important regulators of cellular functions.

The use of enzyme inhibitors to block activity is also problematic. For example, very few enzyme inhibitors have adequate specificity to allow for the unequivocal correlation of a given enzyme with a specific modification reaction. Indeed, many inhibitors have a broad inhibitory range. For example, staurosporine is a potent inhibitor of phospholipid/$Ca^{+2}$ dependant kinases. Wortmannin is some what more specific, being limited to the phosphatidylinositol-3 kinase family. This is clearly unsatisfactory because more than one biochemical pathway may be affected during treatment making the assignment of the effects almost impossible.

Monoclonal antibodies directed against phosphorylated epitopes, except in specific cases, exhibit a limitation of specificity comparable to that observed when *in vivo* labeling is undertaken. Immunological methods can only detect phosphorylated proteins globally (e.g., an anti-phosphotyrosine antibody will detect all tyrosine-phosphorylated proteins) and can only describe a steady state, rather provide a real-time assessment of protein:protein interactions. Such assays also require considerable manpower for processing.

Finally, yeast (*Saccharomyces cervisiae* and *Schizosaccharomyces pombe)* has been exploited as a model organism for the identification of gene function using recessive mutations. It is through research on the effects of these mutations that the functional specificities of many protein-modifying enzymes have been elucidated. However, these molecular genetic techniques are not easily transferable to higher eukaryotes, which are diploid and therefore not as genetically tractable as these lower eukaryotes.

A non-limiting example of post-translational modification is provided by the Ras proteins, which are a conserved group of polypeptides located at the plasma membrane which exist in either a GTP-bound active state or in a GDP-bound inactive state. This family of proteins operates in signal transduction pathways that regulate cell growth and differentiation. In higher eukaryotes, Ras is a key regulator that mediates signal transduction from cell surface tyrosine kinase receptors to the nucleus via activation of the MAP kinase cascade. Recent studies have demonstrated that Ras directly binds a serine/threonine kinase, Raf-1, a product of the c-raf-1 proto-oncogene, and that this association leads to stimulation of the activity of Raf-1 to phosphorylate MAP kinase kinase (MEK).

An important post-translational modification is the addition of ubiquitin to selected polypeptides. This provides a key mechanism by which to control the abundance of important regulatory proteins, for example, G1 and mitotic cyclins and the p53 tumor suppressor protein. Ubiquitin is a highly conserved 76-amino-acid cellular polypeptide. The role of ubiquitin in targeting proteins for degradation involves the specific ligation of ubiquitin to the group of lysine residues in proteins that are to be degraded or internalized from the plasma membrane. The ubiquitin tag determines the fate of the protein and results in its selective proteolysis. Recently a number of factors have been isolated and shown to be involved in the ubiquitination process.

The initial step in the addition of ubiquitin to a protein is the activation of ubiquitin by the ubiquitin activating enzyme, El. This is an ATP-dependent step resulting in the formation of a thioester bond between the carboxyl terminal glycine of ubiquitin and the active site cysteine residue of E1. Activated ubiquitin then interacts with a second factor, the E2 protein. A thioester bond forms between the activated glycine residue of ubiquitin and a cysteine residue in a specific E2 protein. The E2 proteins represent a family of closely-related proteins encoded by different genes that confer specificity in the proteolytic process. The ligation of ubiquitin to target proteins is effected by the involvement of a further factor, a ubiquitin ligase, E3. of which a number are known (in yeast. reviewed by Haas and Siepmann, 1997, FASEB J., 11: 1257-1268; in humans, see Honda et al., 1997, FEBS Lett., 420: 25-27). E3 completes the final step of ubiquitination by attaching ubiquitin *via* the amino group on lysine residues in proteins to be targeted for degradation. Moreover, E3 is able to add ubiquitin to ubiquitin molecules already attached to target proteins, thereby resulting in polyubiquitinated proteins that are ultimately degraded by the multi-subunit proteasome. Recent research into the sites of protein phosphorylation has revealed a number of sequence specific motifs which, when phosphorylated or dephosphorylated, promote interaction with selected target proteins to either induce or inhibit activity of either the phosphorylated polypeptide or the target polypeptide.

For example, and not by way of limitation, many proteins involved in intracellular signal transduction have been shown to contain a domain comprising a sequence of approximately 100 amino acids; this sequence is termed the Src homology two (SH2) domain. SH2 domains bind target polypeptides that contain phosphorylated tyrosine. This binding is dependent on the primary amino acid sequence around the phosphotyrosine in the target protein and several peptide sequences which, when phosphorylated, bind to an SH2 domain have been identified (see e.g., Songyang et al., 1993, Cell, 72: 767-778). Non-limiting examples of such sequences include FLPVPEYINQSV, a sequence found in human ECF receptor, and AVGNPEYLNTVQ, a sequence found in human EGF receptor, both of which are autophosphorylated growth factor receptors which stimulate the biochemical signaling pathways that control gene expression, cytoskeletal architecture and cell metabolism. Both of these sequences interact with SH2 domains found in the Sen-5 adapter protein.

The tumor suppressor protein p53, becomes activated by a transcription factor in response to DNA damage. A DNA-dependent protein kinase (DNA-PK) that is activated in response to breaks in DNA is thought to be a regulator of p53 activity (Woo et al., 1998, Nature, 394: 700-704). The data described by Woo et al. indicate that the phosphorylation of p53 by DNA-PK serves a dual purpose insofar as phosphorylation promotes the binding of p53 to DNA and also prevents p53 inactivation by MDM2. A p53-derived peptide sequence EPPLSQEAFADLWKK is identified as the site of phosphorylation in p53 that (when phosphorylated) prevents the interaction of p53 with MDM2.

An example of heterodimer association is described in patent application number WO92/00388. It describes an adenosine 3: 5 cyclic monophosphate (cAMP) dependent protein kinase which is a four-subunit enzyme being composed of two catalytic polypeptides (C) and two regulatory polypeptides (R). In nature the polypeptides associate in a stoichiometry of $R_2C_2$. In the absence of cAMP the R and C subunits associate and the enzyme complex is inactive. In the presence of cAMP the R subunit functions as a ligand for cAMP resulting in dissociation of the complex and the release of active protein kinase. The invention described in WO92/00388 exploits this association by adding fluorochromes to the R and C subunits.

The polypeptides are labeled (or 'tagged') with fluorophores having different excitation/emission wavelengths. The excitation and emission of one such fluorophore effects a second excitation/emission event in the second fluorophore. By monitoring the fluorescence emission of each fluorophore, which reflects the presence or absence of fluorescence energy transfer between the two, it is possible to derive the concentration of cAMP as a function of the level of association between the R and C subunits. Therefore, the natural affinity of the C subunit for the R subunit has been exploited to monitor the concentration of a specific metabolite, namely cAMP.

The prior art teaches that intact, fluorophore-labeled proteins can function as reporter molecules for monitoring the formation of multi-subunit complexes from protein monomers; however, in each case, the technique relies on the natural ability of the protein monomers to associate.

Tsien *et al.* (WO97/28261) teach that fluorescent proteins having the proper emission and excitation spectra that are brought into physically close proximity with one another can exhibit fluorescence resonance energy transfer ("FRET"). The invention of WO97/28261 takes advantage of that discovery to provide tandem fluorescent protein constructs in which two fluorescent protein labels capable of exhibiting FRET are coupled through a linker to form a tandem construct. In the assays of Tsien *et al.,* protease activity is monitored using FRET to determine the distance between fluorophores controlled by a peptide linker and subsequent hydrolysis thereof. Other applications rely on a change in the intrinsic fluorescence of the protein as in the kinase assays of WO98/06737.

The present invention instead encompasses monitoring of the association of polypeptides, as described herein, which are labelled with fluorescent (protein and chemical) labels. FRET, a non-limiting example of a detection method of use in the invention, indicates the proximity of two labelled polypeptide binding partners, which labelled partners associate either in the presence or absence of a given post-translational modification to a site which is present in the natural binding domain and, optionally, in the binding partner, but not into the fluorophore, reflecting the modification state of one or both of the binding partners and, consequently, the level of activity of a protein-modifying enzyme. We also describe methods which employ non-fluorescent labels including, but not limited to, radioactive labels. In addition, we describe methods which do not employ detectable labels; such methods include, but are not limited to, the detection of the inhibition or reconstitution of enzymatic activity, which inhibition or reconstitution results from modification-dependent binding or dissociation between a natural binding domain and a binding partner therefor.

There is a need in the art for efficient means of monitoring and/or modulating post-translational protein modification. Further, there is a need to develop a technique whereby the addition/removal of a modifying group can be monitored continuously during real time to provide a dynamic assay system that also has the ability to resolve spatial information.

EP-A-0392808 describes a method for determining the presence or level of activity of an enzyme in a sample. A labelled substrate for the enzyme is contacted with an enzyme containing sample to produce a product. A receptor, which binds specifically with either the substrate or the product (but not both) is then contacted with the reaction medium. Binding between the two is then determined by the presence or amount of label in the complex, or uncomplexed. The substrate is labelled with a bioluminescent protein, and EP-A-0392808 states that other types of labels not being bioluminescent proteins, would not work.

We describe natural binding domains, sequences and polypeptides, as well as kits comprising these molecules and assays of enzymatic function in which they are employed as reporter molecules. As used herein in reference to a polypeptide component of assays of the invention, the term "natural" refers both to the existence of such an amino acid sequence, whether contiguous or non-contiguous, in nature as well as to the phosphorylation-dependent binding of that component to a second polypeptide or binding partner, and does not relate to attributes of such a polypeptide other than such binding.

The invention makes use of a natural binding domain and a binding partner therefor, wherein the natural binding domain includes a site for post-translational modification and binds the binding partner therefor in a manner dependent upon modification of the site. At least one of the natural binding domain and the binding partner is labelled with a fluorescent label.

The invention also provides a method for monitoring activity of an enzyme comprising performing a detection step to detect binding of a natural binding domain and a binding partner therefor as a result of contacting one or both of the natural binding domain and the binding partner with the enzyme, wherein the natural binding domain includes a site for post-translational modification and binds the binding partner in a manner dependent upon modification of the site and wherein detection of binding of the natural binding domain and the binding partner as a result of the contacting is indicative of enzyme activity, in which at least one of the natural binding domain and the binding partner is labelled with a fluorescent label.

An enzyme to be assayed according to the invention may, for example, be selected from a carbohydrate transferase, deglycosidase, a ubiquitin activating enzyme E1, a ubiquitin conjugating enzyme E2, a ubiquitin conjugating enzyme Ubc9, a ubiquitin protein ligase E3, a poly (ADP-ribose) polymerase, a fatty acyl transferase, an NAD:Arginine ADP ribosyltransferase, a protease, a protein kinase and a protein phosphatase.

The invention additionally encompasses a method for monitoring activity of an enzyme comprising performing a detection step to detect dissociation of a natural binding domain from a binding partner therefor as a result of contacting one or both of the natural binding domain and the binding partner with the enzyme, wherein the natural binding domain includes a site for post-translational phosphorylation and binds the binding partner in a manner dependent upon phosphorylation of the site and wherein detection of dissociation of the natural binding domain from the binding partner as a result of the contacting is indicative of enzyme activity, in which at least one of the natural binding domain and the binding partner is labelled with a fluorescent label.

Another aspect of the invention is a method for monitoring activity of an enzyme comprising performing a detection step to detect dissociation of a natural binding domain from a binding partner therefor as a result of contacting one or both of the natural binding domain and the binding partner with said enzyme, wherein the natural binding domain includes a site for post-translational modification and binds the binding partner in a manner dependent upon modification of the site and wherein detection of dissociation of the natural binding domain from the binding partner as a result of the contacting is indicative of enzyme activity, in which at least one of the natural binding domain and the binding partner is labelled with a fluorescent label.

As used herein, the term "binding domain" in a three-dimensional sense refers to the amino acid residues of a first polypeptide required for modification-dependent binding between the first polypeptide and its binding partner. The amino acids of a "binding domain" may be either contiguous or non-contiguous and may form a binding pocket for modification-dependent binding. A binding domain must include at least 1 amino acid, and may include 2 or more amino acids, preferably at least 4 amino acids, which are contiguous or non-contiguous, but are necessary for modification-dependent binding to the binding partner. A binding domain will not include a natural full-length polypeptide, but will include a subset of the amino acids of a full-length polypeptide, wherein the subset may include a number of amino acids as high as one fewer than the length of a given natural full-length polypeptide.

A binding domain which is of use in the invention is a "natural binding domain" (i.e., a binding domain that exhibits modification-dependent binding to a binding partner in nature). A natural binding domain of use in the invention may be isolated or may be present in the context of a larger polypeptide molecule (i.e., one which comprises amino acids other than those of the natural binding domain), which molecule may be either naturally-occurring or recombinant and, in the case of the latter, may comprise either natural or non-natural amino acid sequences outside the binding domain,

As used herein with regard to modification of a polypeptide, the terms "site" and "site sufficient for the addition of" refer to an amino acid sequence of a natural binding domain or a binding partner which is recognized by (i.e., a signal for) a modifying enzyme for the purpose of post-translational modification (i.e., addition or removal of a "moiety" as defined below) of the polypeptide or a portion thereof. A "site" additionally refers to the single amino acid which is modified. It is contemplated that a site comprises a small number of amino acids, as few as one but typically from 2 to 10, less often up to 30 amino acids, and further that a site comprises fewer than the total number of amino acids present in the polypeptide.

In an enzymatic assay of the invention, a "site", for post-translational modification may be present on either or both of a natural binding domain and its binding partner. If such sites are present on both the natural binding domain and the binding partner, binding between the natural binding domain and its binding partner may be dependent upon the modification state of either one or both sites. If a single polypeptide chain comprises the natural binding domain and its binding partner (or two natural binding domains), the state of post-translational modification of one or both sites will determine whether binding between the two domains occurs.

As used herein, the term "modification" or "post-translational modification" refers to the addition or removal of a chemical "moiety", as described herein, to/from a site on a polypeptide chain and does not refer to other post-translational events which do not involve addition or removal of such a moiety as described herein, and thus does not include simple cleavage of the reporter molecule polypeptide backbone by hydrolysis of a peptide bond, but does include hydrolysis of an isopeptide bond (e.g., in the removal of ubiquitin).

As used interchangeably herein, the terms "moiety" and "group" refer to one of the post-translationally added or removed groups referred to herein: i.e., one of a ubiquitin moiety, a glycosyl moiety, a fatty acyl moiety, a sentrin moiety, a phosphate moiety or an ADP-ribosyl moiety.

As used herein, the term "binding partner" refers to a polypeptide or fragment thereof (a peptide) that binds to a binding domain, sequence or polypeptide, as defined herein, in a manner which is dependent upon the state of modification of a site for post-translational modification which is, at a minimum, present upon the binding domain, sequence or polypeptide; the binding partner itself may, optionally, comprise such a site and binding between the binding domain, fragment or polypeptide with its corresponding binding partner may, optionally, depend upon modification of that site. Binding between the natural binding domain and the binding partner, or between two natural binding domains, may be dependent upon the phosphorylation or dephosphorylation state of either one or both sites. If a single polypeptide chain comprises the natural binding domain and the binding partner (or two natural binding domains), the state of phosphorylation or dephosphorylation of one or both sites will determine whether binding occurs.

A site suitable for addition or removal of a phosphate moiety is present within a natural binding domain or binding partner thereof at a position such that formation of a complex between the natural binding domain and its binding partner is dependent upon the presence or absence of the phosphate moiety; and preferably does not overlap with an amino acid which is part of a fluorescent tag or quencher.

Similarly, the amino acid that includes a phosphate moiety may be positioned anywhere within the natural binding domain such that binding of the natural binding domain and its binding partner is dependent upon the presence or absence of the phosphate moiety.

As used herein, "phosphorylation" and "dephosphorylation" refer to the addition or removal of a phosphate moiety to/from a polypeptide, respectively. As used herein, the term "post-translational modification" refers to the addition or removal of a chemical moiety but does not include simple cleavage of the reporter molecule polypeptide backbone by hydrolysis of a peptide bond.

As used herein, the term "moiety" includes a post-translationally added or removed phosphate ($PO_4$ group; the terms "moiety" and "group" are used interchangeably.

As used herein, the term "binding partner" refers to a polypeptide or fragment thereof (a peptide) that binds to a binding domain, sequence or polypeptide, as defined herein, in a manner which is dependent upon the state of phosphorylation of a site for phosphorylation or dephosphorylation which is, at a minimum, present upon the binding domain, sequence or polypeptide; the binding partner itself may, optionally, comprise such a site and binding between the binding domain, fragment or polypeptide with its corresponding binding partner may, optionally, depend upon modification of that site. A binding partner does not necessarily have to contain a site for post-translational modification if such a site is not required to be present on it for modification-dependent association between it and a binding domain, sequence or polypeptide. Binding partners of use in the invention are those which are found in nature and exhibit natural modification-dependent binding to a natural binding domain, sequence or polypeptide of the invention as defined herein. In one embodiment of the invention, a binding partner is shorter (i.e., by at least one N-terminal or C-terminal amino acid) than the natural full-length polypeptide.

As used herein, the term "associates" or "binds" refers to a natural binding domain as described herein and its binding partner, having a binding constant sufficiently strong to allow detection of binding by FRET or other detection means, which are in physical contact with each other and have a dissociation constant (Kd) of about $10\mu M$ or lower. The contact region may include all or parts of the two molecules. Therefore, the terms "substantially dissociated" and "dissociated" or "substantially unbound" or "unbound" refer to the absence or loss of contact between such regions, such that the binding constant is reduced by an amount which produces a discemable change in a signal compared to the bound state, including a total absence or loss of contact, such that the proteins are completely separated, as well as a partial absence or loss of contact, so that the body of the proteins are no longer in close proximity to each other but may still be tethered together or otherwise loosely attached, and thus have a dissociation constant greater than 10 μM (Kd). In many cases, the Kd will be in the mM range. The terms "complex". "dimer", "multimer" and "oligomer" as used herein, refer to the natural binding domain and its binding partner in the associated or bound state. More than one molecule of each of the two or more proteins may be present in a complex, dimer, multimer or oligomer according to the methods of the invention.

As used herein in reference to a natural binding domain or other polypeptide, the term "isolated" refers to a molecule or population of molecules that is substantially pure (i.e., free of contaminating molecules of unlike amino acid sequence).

As used herein in reference to the purity of a molecule or population thereof, the term "substantially" refers to that which is at least 50%, preferably 60-75%, more preferably from 80-95% and, most preferably, from 98-100% pure.

"Naturally-occurring" as used herein, as applied to a polypeptide or polynucleotide, refers to the fact that the polypeptide or polynucleotide can be found in nature. One such example is a polypeptide or polynucleotide sequence that is present in an organism (including a virus) that can be isolated form a source in nature.

The term "synthetic", as used herein, is defined as any amino- or nucleic acid sequence which is produced *via* chemical synthesis.

In an assay of the invention, post-translational modification is reversible, such that repeating cycles of addition and removal of a modifying moiety may be observed, although such cycles may not occur in a living cell found in nature.

An advantage of assays of the invention is that they may, if desired, be performed in "real time". As used herein in reference to monitoring, measurements or observations in assays of the invention, the term "real time" refers to that which is performed contemporaneously with the monitored, measured or observed events and which yields a result of the monitoring, measurement or observation to one who performs it simultaneously, or effectively so, with the occurrence of a monitored, measured or observed event. Thus, a "real time" assay or measurement contains not only the measured and quantitated result, such as fluorescence, but expresses this in real time, that is, in hours, minutes, seconds, milliseconds, nanoseconds, picoseconds, etc. Shorter times exceed the instrumentation capability; further, resolution is also limited by the folding and binding kinetics of polypeptides.

As used herein. the term "binding sequence" refers to that portion of a polypeptide comprising at least 4, but up to 8, 10, 100 or even 1000 contiguous (i.e., covalently linked by peptide bonds) amino acid residues, that are sufficient for modification-dependent binding to a binding partner. A binding sequence will not include a natural full-length polypeptide, but will include a subset of the amino acids of a full-length polypeptide, wherein the subset may include a number of amino acids as high as one fewer than the length of a given natural full-length polypeptide.

As used herein in reference to those binding sequences that are of use in the invention, the term "natural binding sequence" refers to a binding sequence, as defined above, which consists of an amino acid sequence which is found in nature and which is naturally dependent upon the modification state of a site for post-translational modification found within it for binding to a binding partner. A "natural binding sequence" may be present either in isolation or in the context of a larger polypeptide molecule, which molecule may be naturally-occurring or recombinant. If present, amino acids outside of the binding sequence may be either natural, i.e.. from the same polypeptide sequence from which the fragment is derived, or non-natural, i.e., from another (different) polypeptide or from a sequence that is not derived from any known polypeptide. In assays of the invention, a binding sequence and its binding partner may exist either on two different polypeptide chains or on a single polypeptide chain.

As used herein, the term "binding polypeptide" refers to a molecule comprising multiple binding sequences, as defined above. A binding polypeptide of use in the invention is a "natural binding polypeptide", in which the component binding sequences are natural binding sequences, as defined above (e.g., wherein the binding sequences are derived from a single, naturally-occurring polypeptide molecule), and are both necessary and, in combination, sufficient to permit modification-state-dependent binding of the binding polypeptide to its binding partner, as defined above, wherein the sequences of the binding polypeptide are either contiguous or are non-contiguous. As used herein in reference to the component binding sequences of a binding polypeptide, the term "non-contiguous" refers to binding sequences which are linked by intervening naturally-occurring, as defined herein, or non-natural amino acid sequences or other chemical or biological linker molecules such are known in the art. The amino acids of a polypeptide that do not significantly contribute to the modification-state-dependent binding of that polypeptide to its binding partner may be those amino acids which are naturally present and link the binding sequences in a binding polypeptide or they may be derived from a different natural polypeptide or may be wholly unknown in nature. In assays of the invention, a binding polypeptide and its binding partner (which may, itself, be a binding domain, sequence or polypeptide, as defined herein) may exist on two different polypeptide chains or on a single polypeptide chain. According to the invention, a natural binding polypeptide, like a polypeptide as defined above, is not a full-length natural polypeptide chain, but instead comprises a subset that encompasses up to one fewer than the total number of amino acids in a natural polypeptide chain.

As used herein, the terms "polypeptide" and "peptide" refer to a polymer in which the monomers are amino acids and are joined together through peptide or disulfide bonds. The terms subunit and domain also may refer to polypeptides and peptides having biological function. A peptide useful in the invention will at least have a binding capability, i.e, with respect to binding as- or to a binding partner, and also may have another biological function that is a biological function of a protein or domain from which the peptide sequence is derived. "Polypeptide" refers to a naturally-occurring amino acid chain comprising a subset of the amino acids of a full-length protein, wherein the subset comprises at least one fewer amino acid than does the full-length protein, or a "fragment thereof" or "peptide", such as a selected region of the polypeptide that is of interest in a binding assay and for which a binding partner is known or determinable. "Fragment thereof" thus refers to an amino acid sequence that is a portion of a full-length polypeptide, between about 8 and about 1000 amino acids in length, preferably about 8 to about 300, more preferably about 8 to about 200 amino acids, and even more preferably about 10 to about 50 or 100 amino acids in length. "Peptide" refers to a short amino acid sequence that is 10-40 amino acids long, preferably 10-35 amino acids. Additionally, unnatural amino acids, for example, alanine, phenyl glycine and homoarginine may be included. Cornmonly-encountered amino acids which are not gene-encoded may also be used in the present invention. All of the amino acids used in the present invention may be either the D- or L- optical isomer. The L-isomers are preferred. In addition, other peptidomimetics are also useful, e.g. in linker sequences of polypeptides of the present invention (see Spatola, 1983, in Chemistry and Biochemistry of Amino Acids. Peptides and Proteins, Weinstein, ed., Marcel Dekker, New York, p. 267).

As used herein, the terms "protein", "subunit" and "domain" refer to a linear sequence of amino acids which exhibits biological function. This linear sequence does not include full-length amino acid sequences (e.g. those encoded by a full-length gene or polynucleotide), but does include a portion or fragment thereof, provided the biological function is maintained by that portion or fragment. The terms "subunit" and "domain" also may refer to polypeptides and peptides having biological function. A peptide useful in the invention will at least have a binding capability, i.e, with respect to binding as or to a binding partner, and also may have another biological function that is a biological function of a protein or domain from which the peptide sequence is derived.

"Polynucleotide" refers to a polymeric form of nucleotides of at least 10 bases in length and up to 1,000 bases or even more, either ribonucleotides or deoxyribonucleotides or a modified form of either type of nucleotide. The term includes single and double stranded forms of DNA.

Preferably, with regard to the natural binding domain and/or binding partner therefor that the site comprises a sequence which directs modification by one or more of the following enzymes: a carbohydrate transferase (e.g., a UDP-N-Acetylglucosamine-Dolichyl-phosphate-N-acetylsglucosamine phosphotransferase or an O-GlcNAc transferase), a ubiquitin activating enzyme E1, a ubiquitin conjugating enzyme E2, a ubiquitin conjugating enzyme Ubc9, a ubiquitin protein ligase E3, a poly (ADP-ribose) polymerase, a fatty acyl transferase (e.g., a glycylpeptide-N-tetradecanoyltransferase (peptide N-myristoyltransferase)) and an NAD:Arginine ADP ribosyltransferase.

It is preferred that phosphorylation of the site prevents binding of the natural binding domain to the binding partner.

As used herein, the term "prevents" refers to a reduction of at least 10%, preferably 20-40%, more preferably 50-75% and, most preferably, 80-100% of binding of the natural binding domain to the binding partner therefor.

It is additionally preferred that the site permits addition of a chemical moiety which may be: a ubiquitin moiety, a glycosyl moiety, an ADP-ribosyl moiety, a phosphate moiety, a fatty acid-moiety and a sentrin moiety, and the addition prevents binding of the natural binding domain to the binding partner.

As used herein the term "prevents binding" or "prevents association" refers to the ability of at least one of a ubiquitin moiety, a glycosyl moiety, a fatty acyl moiety, a sentrin moiety, a phosphate moiety or an ADP-ribosyl moiety to inhibit the association, as defined above, of a natural binding domain and a binding partner thereof by at least 10%, preferably by 25-50%, highly preferably by 75-90% and, most preferably, by 95-100% relative the association observed in the absence of such a modification under the same experimental conditions.

According to another preferred embodiment, the site permits addition of a chemical moiety which may be: a ubiquitin moiety, a glycosyl moiety, an ADP-ribosyl moiety, a phosphate moiety, a fatty acid moiety and a sentrin moiety, and the addition promotes binding of the natural binding domain to the binding partner.

As used herein, the term "promotes binding" refers to that which causes an increase in binding of the natural binding domain and its binding partner of at least two-fold, preferably 10- to 20-fold, highly preferably 50- to 100-fold, more preferably from 200- to 1000-fold, and, most preferably, from 200 to 10,000-fold.

Preferably, the site permits removal of a chemical moiety which may be: a ubiquitin moiety, a glycosyl moiety, an ADP-ribosyl moiety, a phosphate moiety, a fatty acid moiety and a sentrin moiety, and the removal prevents binding of the natural binding domain to the binding partner.

It is additionally preferred that the site permits removal of a chemical moiety which may be: a ubiquitin moiety, a glycosyl moiety, an ADP-ribosyl moiety, a fatty acid moiety, a phosphate moiety and a sentrin moiety, and the removal promotes binding of the natural binding domain to the binding partner.

The natural binding partner and/or binding domain may comprise more than one site as referred to above. - Thus for example, the natural binding partner and/or binding domain may comprise two or three sites that are subject to post-translational modification.

At least one of the natural binding domain and the binding partner comprises a detectable label which is a fluorescent label. At least one, preferably both, of the binding domain and the binding partner comprise a fluorescent label.

It is preferred that one of the natural binding domain and the binding partner thereof comprises a quencher for the detectable label.

A "fluorescent label", "fluorescent tag" or "fluorescent group" refers to either a fluorophore or a fluorescent protein or fluorescent fragment thereof. "Fluorescent protein" refers to any protein which fluoresces when excited with appropriate electromagnetic radiation. This includes a protein whose amino acid sequence is either natural or engineered. A "fluorescent protein" is a full-length fluorescent protein or fluorescent fragment thereof . By the same token, the term "linker" refers to that which is coupled to both the donor and acceptor protein molecules, such as an amino acid sequence joining two natural binding domains or a disulfide bond between two polypeptides.

It is contemplated that with regard to fluorescent labels employed in FRET, the reporter labels are chosen such that the emission wavelength spectrum of one (the "donor") is within the excitation wavelength spectrum of the other (the "acceptor"). With regard to a fluorescent label and a quencher employed in a single-label detection procedure in an assay of the invention, it is additionally contemplated that the fluorophore and quencher are chosen such that the emission wavelength spectrum of the fluorophore is within the absorption spectrum of the quencher, such that when the fluorophore' and the quencher with which it is employed are brought into close proximity by binding of the natural binding domain, sequence or polypeptide upon which one is present with the binding partner comprising the other, detection of the fluorescent signal emitted by the fluorophore is reduced by at least 10%, preferably 20-50%, more preferably 70-90% and, most preferably, by 95-100%. A typical quencher reduces detection of a fluorescent signal by approximately 80%.

We describe further a kit comprising a natural binding domain and a binding partner therefor, wherein the natural binding domain includes a site for post-translational modification and binds the binding partner in a manner dependent upon modification of the site, and packaging material therefor.

It is preferred that the kit further comprises a buffer which permits modification-dependent binding of the natural binding domain and the binding partner.

As used herein, the term "buffer" refers to a medium which permits activity of the protein-modifying enzyme used in an assay of the invention, and is typically a low-ionic-strength- or other biocompatible solution (e.g., water, containing one or more of physiological salt, such as simple saline, and/or a weak buffer, such as Tris or phosphate, or others as described hereinbelow), a cell culture medium, of which many are known in the art, or a whole or fractionated cell lysate. Such a buffer permitsdimerization of a non-ubiquitinated and/or non-prenylated and/or non-sentrinated and/or non-ADP-ribosylated and/or non- glycosylated and/or non-phosphorylated natural binding domain and a binding partner therefor and, preferably, inhibits degradation and maintains biological activity of the reaction components. Inhibitors of degradation, such as protease inhibitors (e.g., pepstatin, leupeptin, etc.) and nuclease inhibitors (e.g., DEPC) are well known in the art. Lastly, a buffer may comprise a stabilizing substance such as glycerol, sucrose or polyethylene glycol.

As used herein, the term "physiological buffer" refers to a liquid medium that mimics the salt balance and pH of the cytoplasm of a cell or of the extracellular milieu, such that post-translational protein modification reactions and protein:protein binding are permitted to occur in the buffer as they would *in vivo.*

Preferably, the buffer additionally permits modification of the site for protein modification by one or more of the following enzymes: a kinase, a phosphatase, a carbohydrate transferase (e.g., a UDP-N-Acetylglucosamine-Dolichyl-phosphate-N-acetylsglucosamine phosphotransfer-ase or an O-GlcNAc transferase), a ubiquitin activating enzyme E1, a ubiquitin conjugating enzyme E2, a ubiquitin conjugating enzyme Ubc9, a ubiquitin protein ligase E3, a poly (ADP-ribose) polymerase, a fatty acyl transferase (e.g., a glycylpeptide-N-tetradecanoyltransferase (peptide N-myristoyltransferase)) and an NAD:Arginine ADP ribosyltransferase.

It is preferred that the kit further comprises one or more of the following enzymes: carbohydrate transferase (e.g., a UDP-N-Acetylglucosamine-Dolichyl-phosphate-N-acetylsglucosamine phosphotransferase or an O-GlcNAc transferase), a kinase, a phosphatase, a ubiquitin activating enzyme E1, a ubiquitin conjugating enzyme E2, a ubiquitin conjugating enzyme Ubc9, a ubiquitin protein ligase E3, a poly (ADP-ribose) polymerase, a fatty acyl transferase (e.g., a glycylpeptide-N-tetradecanoyltransferase (peptide N-myristoyltransferase)) and an NAD:Arginine ADP ribosyltransferase.

It is additionally preferred that the kit further comprises a substrate for the enzyme which may be: ubiquitin, sentrin, nicotinamide adenine dinucleotide (NAD$^+$), uridine-diphosphate-N-acetylglucosamine-dolichyl-phosphate (LTDP-N-acetylglucosamine-dolichyl-phosphate), palmytyl CoA, myristoyl CoA, UDP-N-acetylglucosamine or MgATP, .

It is contemplated that at least a part of a substrate of an enzyme of use in an assay of the invention is transferred to a modification site on an isolated polypeptide. As used herein, the term "at least a part of a substrate" refers to a portion (e.g., a fragment of an amino acid sequence, a moiety or a group, as defined above) which comprises less than the whole of the substrate for the enzyme, the transfer of which portion to a modification site on an isolated polypeptide, both as defined above, is catalyzed by the enzyme.

It is additionally preferred that the kit further comprises a cofactor for one or both of the kinase or phosphatase. Cofactors of use in the invention include, but are not limited to, cAMP, phosphotidylserine, diolein and Mn$^{2+}$. Preferably, the kit further comprises a cofactor for said enzyme.

An enzyme (e.g., a protein kinase or phosphatase) of use in the invention may be natural or recombinant or, alternatively, may be chemically synthesized. If either natural or recombinant, it may be substantially pure (i.e., present in a population of molecules in which it is at least 50% homogeneous), partially purified (i.e., represented by at least 1% of the molecules present in a fraction of a cellular lysate) or may be present in a crude biological sample.

As used herein, the term "sample" refers to a collection of inorganic, organic or biochemical molecules which is either found in nature (e.g., in a biological- or other specimen) or in an artificially-constructed grouping, such as agents which might be found and/or mixed in a laboratory. Such a sample may be either heterogeneous or homogeneous.

As used herein, the interchangeable terms "biological specimen" and "biological sample" refer to a whole organism or a subset of its tissues, cells or component parts (e.g. body fluids, including but not limited to blood, mucus, lymphatic fluid, synovial fluid, cerebrospinal fluid, saliva, amniotic fluid, amniotic cord blood, urine, vaginal fluid and semen). "Biological sample" and "biological specimen" further refer to a homogenate, lysate or extract prepared from a whole organism or a subset of its tissues, cells or component parts, or a fraction or portion thereof. Lastly, "biological sample" refers to a medium, such as a nutrient broth or gel in which an organism has been propagated, which contains cellular components, such as proteins or nucleic acid molecules.

As used herein, the term "organism" refers to all cellular life-forms, such as prokaryotes and eukaryotes, as well as non-cellular, nucleic acid-containing entities, such as bacteriophage and viruses.

In the method as described above, at least one of the natural binding domain and the binding partner is labeled with a fluorescent label.

In a preferred embodiment, the detection step is to detect a change in signal emission by the fluorescent label.

It is preferred that the method further comprises exciting the fluorescent label and monitoring fluorescence emission.

Preferably, the enzyme is one of the following enzymes: a kinase, a phosphatase, a carbohydrate transferase (e.g., a UDP-N-Acetylglucosamine-Dolichyl-phosphate-N-acetylsglucosamine phosphotransfer-ase or an O-GlcNAc transferase), a ubiquitin activating enzyme E1, a ubiquitin conjugating enzyme E2, a ubiquitin conjugating enzyme Ubc9, a ubiquitin protein ligase E3, a poly (ADP-ribose) polymerase, a fatty acyl transferase (e.g., a glycylpeptide-N-tetradecanoyltransferase (peptide N-myristoyltransferase)) and an NAD:Arginine ADP ribosyltransferase.

It is additionally preferred that the method further comprises the step, prior to or after the detection step, of contacting the natural binding domain and the binding partner with an agent which modulates the activity of the enzyme.

As used herein with regard to a biological or chemical agent, the term "modulate" refers to enhancing or inhibiting the activity of a protein-modifying enzyme in an assay of the invention; such modulation may be direct (e.g. including, but not limited to, cleavage of-or competitive binding of another substance to the enzyme) or indirect (e.g. by blocking the initial production or, if required, activation of the modifying enzyme). "Modulation" refers to the capacity to either increase or decease a measurable functional property of biological activity or process (e.g., enzyme activity or receptor binding) by at least 10%, 15%, 20%, 25%, 50%, 100% or more; such increase or decrease may be contingent on the occurrence of a specific event, such as activation of a signal transduction pathway, and/or may be manifest only in particular cell types.

The term "modulator" refers to a chemical compound (naturally occurring or non-naturally occurring), such as a biological macromolecule (e.g., nucleic acid, protein, non-peptide, or organic molecule), or an extract made from biological materials such as bacteria, plants, fungi, or animal (particularly mammalian) cells or tissues, or even an inorganic element or molecule. Modulators are evaluated for potential activity as inhibitors or activators (directly or indirectly) of a biological process or processes (e.g., agonist, partial antagonist, partial agonist, antagonist, antineoplastic agents, cytotoxic agents, inhibitors of neoplastic transformation or cell proliferation, cell proliferation-promoting agents, and the like) by inclusion in screening assays described herein. The activities (or activity) of a modulator may be known, unknown or partially-known. Such modulators can be screened using the methods described herein.

The term "candidate modulator" refers to a compound to be tested by one or more screening method(s) of the invention as a putative modulator. Usually, various predetermined concentrations are used for screening such as 0.01 $\mu$M, 0.1 $\mu$M, 1.0 $\mu$M, and 10.0 $\mu$M, as described more fully hereinbelow. Test compound controls can include the measurement of a signal in the absence of the test compound or comparison to a compound known to modulate the target.

The invention additionally provides a method of screening for a candidate modulator of enzymatic activity of one or more of the following enzymes: a carbohydrate transferase (e.g., a UDP-N-Acetylglucosamine-Dolichyl-phosphate-N-acetylsglucosamine phospho-transferase or an O-GlcNAc transferase), a ubiquitin activating enzyme E1, a ubiquitin conjugating enzyme E2, a ubiquitin conjugating enzyme Ubc9, a ubiquitin protein ligase E3, a poly (ADP-ribose) polymerase, a fatty acyl transferase (e.g, a glycylpeptide-N-tetradecanoyltransferase (peptide N-myristoyltransferase)) an NAD:Arginine ADP ribosyltransferase, and a kinase or a phosphatase, the method comprising contacting a natural binding domain, a binding partner therefor and an enzyme with a candidate modulator of the modifying enzyme, wherein the natural binding domain includes a site for post-translational modification and binds the binding partner in a manner that is dependent upon modification of the site by the enzyme and wherein at least one of the natural binding domain and the binding partner comprises a detectable label, and monitoring the binding of the natural binding domain to the binding partner, wherein binding or dissociation of the natural binding domain and the binding partner as a result of the contacting is indicative of modulation of enzymatic activity by the candidate modulator of the enzyme. The label comprises a fluorescent label.

It is preferred that the monitoring comprises measuring a change in energy transfer between a detectable label present on the natural binding domain and a detectable label present on the binding partner.

A final aspect of the invention is a method of screening for a candidate modulator of enzymatic activity of one or more of the following enzymes: a UDP-N-Acetylglucosamine-Dolichyl-phosphate-N-acetylsglucosamine phospho-transferase, an O-GlcNAc transferase, a ubiquitin activating enzyme E1, a ubiquitin conjugating enzyme E2, a ubiquitin conjugating enzyme Ubc9, a ubiquitin protein ligase E3, a poly (ADP-ribose) polymerase, a glycylpeptide-N-tetradecanoyltransferase (peptide N-myristoyltransferase), an NAD:Arginine ADP ribosyltransferase, and a kinase or a phosphatase, the method comprising contacting an assay system with a candidate modulator of enzymatic activity of such an enzyme, and monitoring binding of a natural binding domain and a binding partner therefor in the assay system, wherein the natural binding domain includes a site for post-translational modification and binds the binding partner in a manner that is dependent upon modification of the site by at least one such enzyme in the assay system, wherein at least one of the natural binding domain and the binding partner comprises a detectable fluorescent label, and wherein binding or dissociation of the natural binding domain and the binding partner as a result of the contacting is indicative of modulation of enzymatic activity by the candidate modulator of such an enzyme.

It is highly preferred that in any of the above methods, the method comprises real-time observation of association of a natural binding domain and its binding partner.

Further features and advantages of the invention will become more fully apparent in the following description of the embodiments and drawings thereof, and from the claims.

Detailed description

The invention is based upon the discovery that a natural binding domain, sequence or polypeptide, as defined above, associates with a binding partner to form a complex or dissociates from a binding partner, in a manner that is dependent upon the presence or absence of a chemical moiety, and that is detectable and measurable in a highly sensitive manner that may be observed in real time.

Polypeptides of use in the invention

Reporter molecules are use in the assays for measuring the activity of protein modifying enzymes. These reporter molecules are naturally occurring polypeptides which include natural binding domains, natural binding sequences and natural binding polypeptides, each as defined above, which are used in assays of the invention in combination with polypeptide binding partners, also as defined above.

Minimally, such a reporter molecule comprises or consists of a natural binding domain. The amino acids of a natural binding domain are those which are necessary for phosphorylation-dependent binding of the molecule comprising or consisting of the natural binding domain with a binding partner, whether such a partner is present on the same or a different polypeptide chain as the natural binding domain. Such amino acids may include points of direct contact between the domain and the binding partner, those which are recognized and/or modified (e.g., phosphorylated or dephosphorylated) by an enzyme and those which maintain the three-dimensional structure or charge of the binding domain in a manner which permits modification and dependent binding of the domain to the binding partner. The amino acids of a natural binding domain may be contiguous or may be separated by non-domain amino acids; such non-domain residues may be either those which are naturally present between the amino acids of the natural binding domain or which are non-natural. In cases in which non-natural amino acids are found interspersed with those of a natural binding domain, such non-natural residues will be residues which do not substantially (that is, measurably) alter the natural phosphorylation-dependent binding of the natural binding domain to its binding partner.

A second reporter molecule of use in the invention is that which comprises or consists of a naturally-occurring stretch of contiguous amino acids sufficient for phosphorylation-dependent binding to a binding partner, as defined above, i.e., at least the minimum number of contiguous amino acids required to encompass a natural binding domain. The phosphorylation-dependence of such a molecule, referred to herein as a "natural binding sequence", is, itself natural. Such a reporter molecule may either consist of or comprise a natural binding sequence. In the latter case, amino acids outside of the natural binding sequence do not substantially influence phosphorylation-dependent binding of the natural binding domain to the binding partner.

Lastly, a reporter molecule of use in the invention may be a "natural binding polypeptide", as defined above. Such a polypeptide molecule comprises or consists of multiple natural binding domains (above), which domains are, either individually or in concert with one another, sufficient to permit natural, phosphorylation-dependent binding of the natural binding polypeptide to a binding partner.

By monitoring the association or dissociation of a natural binding domain, sequence or polypeptide and its binding partner in the presence of a known or candidate protein modifying enzyme, the activity of such an enzyme can be measured. In such assays, one or both of the natural binding domain, sequence or polypeptide and its binding partner comprises a detectable label which is a fluorescent label, which may be either chemical or proteinaceous. By measuring changes in signal emission before and after addition to the mixture comprising the natural binding domain, sequence or polypeptide and its binding partner of the enzyme to be assayed, the extent of modification can be calculated. An important feature of the invention is that such measurements (e.g., of a shift in FRET or other signal emitted by a detectable label) can be performed in real-time. This allows for sensitive assessment of enzyme reaction kinetics based upon the rate of change of the protein-binding-dependent signal emission or absorption by the label(s).

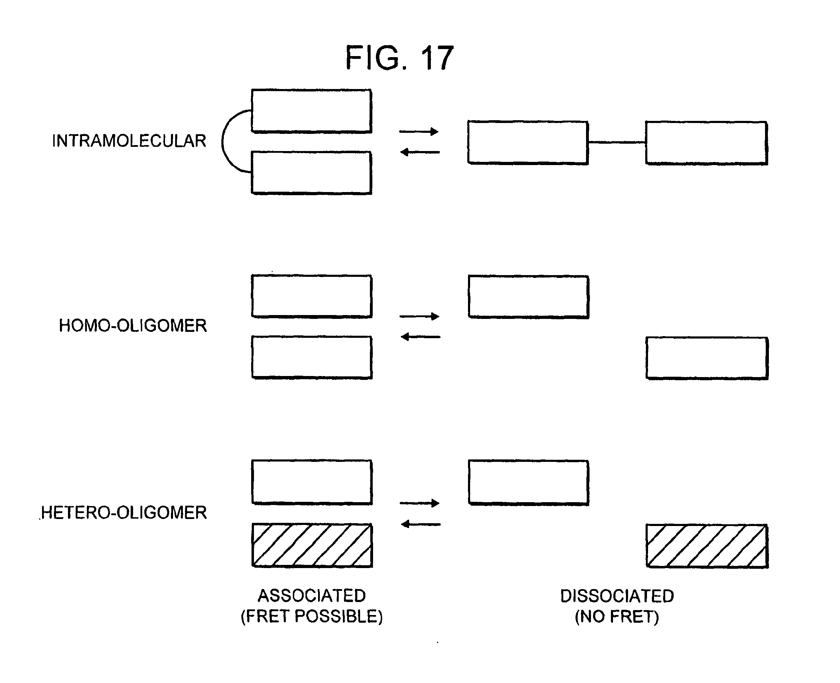

Assays in which the above reporter molecules are used according to the invention may be performed either in double- or single-chain format (Figure 17). In double-chain format, natural binding domain, sequence or polypeptide is comprised by a different polypeptide chain from that comprising or consisting of the binding partner and is not otherwise covalently linked to it. In single-chain format, the natural binding domain, sequence or polypeptide is covalently linked to its binding partner, either through an intervening amino acid sequence or a chemical linker.

The binding partner of a natural binding domain, sequence or polypeptide may, itself, be a natural binding domain, sequence or polypeptide as defined herein. If so, binding of the two molecules may depend upon the modification state of one or both in a manner that is comparable to that found in nature.

Methods by which assays of the invention are performed are described in detail in the following sections and in the Examples.

Unless defined otherwise, all technical and scientific terms used herein have the same meaning as commonly understood by one of ordinary skill in the art (e.g, in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual. 2d ed. (1989) Cold Spring Harbor. Laboratory Press, Cold Spring Harbor, N.Y., which is incorporated herein by reference), chemical methods, pharmaceutical formulation and delivery and treatment of patients.

Methods by which to detect protein:protein binding in assays of the invention

According to the invention, the activity of a modifying enzyme is assayed by measuring the formation or destruction of protein:protein complexes when the modifying enzyme is present with a natural binding domain and its corresponding binding partner under conditions which permit modifying activity. Methods which enable the detection of protein:protein complexes (i.e., methods which allow one of skill in the art to discriminate between polypeptide pairing partners which are bound and those which are unbound) are known in the art.

Methods of detection without use of label are known in the art. These include detection using surface plasmon resonance to detect changes in the mass of the immobilised polypeptide, which would occur if binding of the partner polypeptide increased or decreased. Such measurements may be made for example using a BIAcore machine. Alternatively, mass spectroscopy may be used to detect the presence and mass of a bound species interacting with an immobilised peptide.

However, according to the invention, labelled binding domains and binding partners and therefore techniques which measure physical parameters arising from such labels are used. Thus, typically, at least one of the binding domain and the binding partner comprise a detectable fluorescent label. Examples of other labels include optical labels, an absorptive coloured particle or a dye, radioactive, such as may be applicable for use in a scintillation proximity assay, or a scintillation emulsion, enzymatic, such as a glucose oxidase sensor or other redox system or an immobilised chemical cascade, or based on mass, as might be applicable in a surface plasmon resonance-based assay, in which case either the protein or the binding partner must be of high molecular weight. Fluorescent labels are described in detail below

"Detectable label" means a moiety, proteinaceous or otherwise whose physical characteristics alter in a detectable manner depending on its molecular context, such as when the polypeptide to which it is linked binds to another polypeptide. The detectable label may be detectable in a non-invasive manner that does not require destruction of cellular material. Thus for example labels that do not require the extraction and resolution of cellular components and allow in situ measurements may be contemplated.

The detectable label may comprise subunits or fragments of enzymes that are functional when associated. For example, β-galactosidase may be cleaved into two fragments ($\Delta\alpha$ and $\Delta\omega$) which when expressed together form a functional enzyme (see Sambrook et al., ibid). Such a system has been used in intact yeast to monitor protein-protein interactions (Rossi et al., 1997, Proc. Natl. Acad. Sci. USA 94: 8405-8410). Also, bacterial luciferase can be produced as two fragments which when expressed together and associate, form a functional enzyme (Almashanu et al., 1996, Protein Eng. 9: 803-9).

Detectable labels also include moieties that may serve to increase the size of the reporter polypeptide for use in detection techniques that measure an increase in the size of a complex. Thus for example, a suitable moiety includes streptavidin which is attached to the reporter polypeptide via a biotin moiety. Biotinylation of peptide is well known in the art. A biotin/streptavidin moiety is described in the Examples where the measurement technique used was fluorescent polarisation which measures the rate of tumbling of macromolecules. When the reporter polypeptide coupled to biotin/streptavidin binds to a fluorescently labelled binding partner, the polarisation value of the fluorescent labelled binding partner changes as a result of the large increase in size of the complex.

Where, for example, the binding domain-label and binding partner-label polypeptides will be expressed by living cells the label will typically be in the form of a polypeptide whose amino acid constituents are naturally occurring amino acids, including those arising from post-translational modifications. The nucleic acid constructs encoding said polypeptides may be introduced into the genome of the organism using standard techniques. Typically, the binding domain and binding partner is linked to the detectable label by being expressed in the form of a fusion protein. However, the binding domain and/or binding partner may be linked to their detectable label by any physiological applicable means. For example they may be part of a multi-protein complex joined by disulphide bonding or by non-covalent bonding, such as electrostatic interactions, hydrophobic interactions and/or van der Waal's forces.

The invention relies on those methods which entail fluorescent labelling of the natural binding domain and/or its binding partner, and subsequent detection of changes in fluorescence, whether in frequency or level, following incubation of the labeled assay components with the candidate modifying enzyme. Several such procedures are briefly summarized below. Likewise, methods of detecting the phosphorylation-dependent binding of a natural binding domain, sequence or polypeptide and a binding partner in an assay of the invention most usefully, although not exclusively, are those which employ light-emitting labels. Several such techniques are described below.

Fluorescence energy resonance transfer (FRET)

A tool with which to assess the distance between one molecule and another (whether protein or nucleic acid) or between two positions on the same molecule is provided by the technique of fluorescence resonance energy transfer (FRET), which is now widely known in the art (for a review, see Matyus, 1992, J. Photochem. Photobiol. B: Biol., 12: 323-337. which is herein incorporated by reference). FRET is a radiationless process in which energy is transferred from an excited donor molecule to an acceptor molecule: the efficiency of this transfer is dependent upon the distance between the donor and acceptor molecules, as described below. Since the rate of energy transfer is inversely proportional to the sixth power of the distance between the donor and acceptor, the energy transfer efficiency is extremely sensitive to distance changes. Energy transfer is said to occur with detectable efficiency in the 1-10 nm distance range, but is typically 4-6 nm for favorable pairs of donor and acceptor.

Radiationless energy transfer is based on the biophysical properties of fluorophores. These principles are reviewed elsewhere (Lakowicz, 1983, Principles of Fluorescence Spectroscopy, Plenum Press, New York; Jovin and Jovin, 1989, Cell Structure and Function by Microspectrofluorometry, eds. E. Kohen and J.G. Hirschberg, Academic Press, both of which are incorporated herein by reference). Briefly, a fluorophore absorbs light energy at a characteristic wavelength. This wavelength is also known as the excitation wavelength. The energy absorbed by a flurochrome is subsequently released through various pathways, one being emission of photons to produce fluorescence. The wavelength of light being emitted is known as the emission wavelength and is an inherent characteristic of a particular fluorophore. Radiationless energy transfer is the quantum-mechanical process by which the energy of the excited state of one fluorophore is transferred without actual photon emission to a second fluorophore. That energy may then be subsequently released at the emission wavelength of the second fluorophore. The first fluorophore is generally termed the donor (D) and has an excited state of higher energy than that of the second fluorophore, termed the acceptor (A). The essential features of the process are that the emission spectumi of the donor overlap with the excitation spectrum of the acceptor, and that the donor and acceptor be sufficiently close. The distance over which radiationless energy transfer is effective depends on many factors including the fluorescence quantum efficiency of the donor, the extinction coefficient of the acceptor, the degree of overlap of their respective spectra, the refractive index of the medium, and the relative orientation of the transition moments of the two fluorophores. In addition to having an optimum emission range overlapping the excitation wavelength of the other fluorophore, the distance between D and A must be sufficiently small to allow the radiationless transfer of energy between the fluorophores.

FRET may be performed either *in vivo* or *in vitro.* Proteins are labeled either *in vivo* or *in vitro* by methods known in the art. According to the invention, a natural binding domain, sequence or polypeptide and its binding partner, comprised either by the same or by different polypeptide molecules, are differentially labeled, one with a donor and the other with an acceptor, and differences in fluorescence between a test assay, comprising a protein modifying enzyme, and a control, in which the modifying enzyme is absent, are measured using a fluorimeter or laser-scanning microscope. It will be apparent to those skilled in the art that excitation/detection means can be augmented by the incorporation of photomultiplier means to enhance detection sensitivity. The differential labels may comprise either two different fluorescent labels (e.g., fluorescent proteins as described below or the fluorophores rhodamine, fluorescein, SPQ, and others as are known in the art) or a fluorescent label and a molecule known to quench its signal; differences in the proximity of the natural binding domain, sequence or polypeptide with its binding partner with and without the protein-modifying enzyme can be gauged based upon a difference in the fluorescence spectrum or intensity observed.

This combination of protein-labeling methods and devices confers a distinct advantage over prior art methods for determining the activity of protein-modifying enzymes, as described above, in that results of all measurements are observed in real time (i.e., as a reaction progresses). This is significantly advantageous, as it allows both for rapid data collection and yields information regarding reaction kinetics under various conditions.

A sample, whether *in vitro* or *in vivo,* assayed according to the invention therefore comprises a mixture at equilibrium of the labeled natural binding domain, sequence or polypeptide and its binding partner which, when disassociated from one another, fluoresce at one frequency and, when complexed together, fluoresce at another frequency or, alternatively, of molecules which either do or do not fluoresce or show reduced fluorescence, depending upon whether or not they are associated.

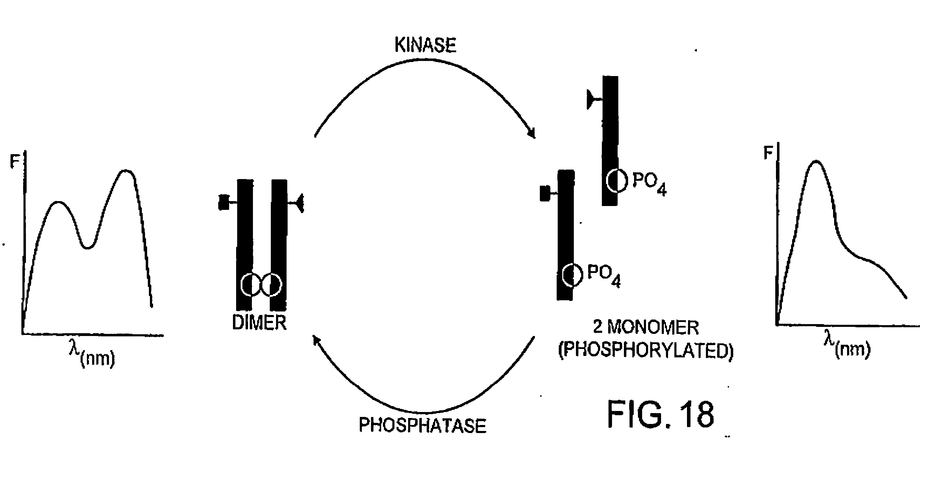

The natural binding domain, sequence or polypeptide is modified to allow the attachment of a fluorescent label to the surface of that molecule or is fused in-frame with a fluorescent protein, as described below. The choice of fluorescent label will be such that upon excitation with light, labeled peptides which are associated will show optimal energy transfer between fluorophores. In the presence of a protein modifying enzyme that recognizes the site for protein modification present on the natural binding domain and, optionally, the binding partner, the natural binding domain and its binding partner dissociate due to a structural or electrostatic change which occurs as a consequence of addition or removal of a chemical moiety, as described herein, to/from the enzyme recognition site, thereby leading to a decrease in energy transfer and increased emission of light by the donor fluorophore. In this way, the state of polypeptide modification can be monitored and quantitated in real-time. This scheme, which represents the broadest embodiment of the invention, is shown in Figure 18.

As used herein, the terms "fluorophore" and "fluorochrome" refer interchangeably to a molecule which is capable of absorbing energy at a wavelength range and releasing energy at a wavelength range other than the absorbance range. The term "excitation wavelength" refers to the range of wavelengths at which a fluorophore absorbs energy. The term "emission wavelength" refers to the range of wavelength that the fluorophore releases energy or fluoresces.

A non-limiting list of chemical fluorophores of use in the invention, along with their excitation and emission wavelengths, is presented in Table 1.

Table 1

| Fluorophore | Excitation (nm) | Emission (nm) | Color |
|---|---|---|---|
| PKH2 | 490 | 504 | green |
| PKH67 | 490 | 502 | green |
| Fluorescein (FITC) | 495 | 525 | green |
| Hoechst 33258 | 360 | 470 | blue |
| R-Phycoerythrin (PE) | 488 | 578 | orange-red |
| Rhodamine (TRITC) | 552 | 570 | red |
| Quantum Red™ | 488 | 670 | red |
| PKH26 | 551 | 567 | red |
| Texas Red | 596 | 620 | red |
| Cy3 | 552 | 570 | red |

It should also be noted that where the fluorescent technique used requires both a donor molecule and an acceptor molecule, naturally occurring or engineered tryptophan residues within the binding domain and/or partner are suitable as a donor and therefore in this situation, only one of the molecules need be labelled with a fluorescent molecule.

Examples of fluorescent proteins which vary among themselves in excitation and emission maxima are listed in Table 1 of WO 97/28261. These (each followed by [excitation max./emission max.] wavelengths expressed in nanometers) include wild-type Green Fluorescent Protein [395(475)/508] and the cloned mutant of Green Fluorescent Protein variants P4 [383/447], P4-3 [381/445], W7 [433(453)/475(501)], W2 [432(453)/480], S65T [489/511], P4-1 [504(396)/480], S65A [471/504], S65C [479/507], S65L [484/510], Y66F [360/442], Y66W [458/480], IOc [513/527], W1B [432(453)/476(503)], Emerald [487/508] and Sapphire [395/511]. This list is not exhaustive of fluorescent proteins known in the art; additional examples are found in the Genbank and SwissProt public databases. Further examples are described in Matz *et al.,* 1999 (Nature Biotech 17: 969-973) and include red fluorescent protein from *Discosoma sp.* (drFP583).

A number of parameters of fluorescence output are envisaged including

1) measuring fluorescence emitted at the emission wavelength of the acceptor (A) and donor (D) and determining the extent of energy transfer by the ratio of their emission amplitudes;
2) measuring the fluorescence lifetime of D;
3) measuring the rate of photobleaching of D;
4) measuring the anisotropy of D and/or A; or
5) measuring the Stokes shift monomer; excimer fluorescence.

Certain of these techniques are presented below.

Alternative fluorescent techniques suitable for monitoring protein:protein binding in assays of the invention

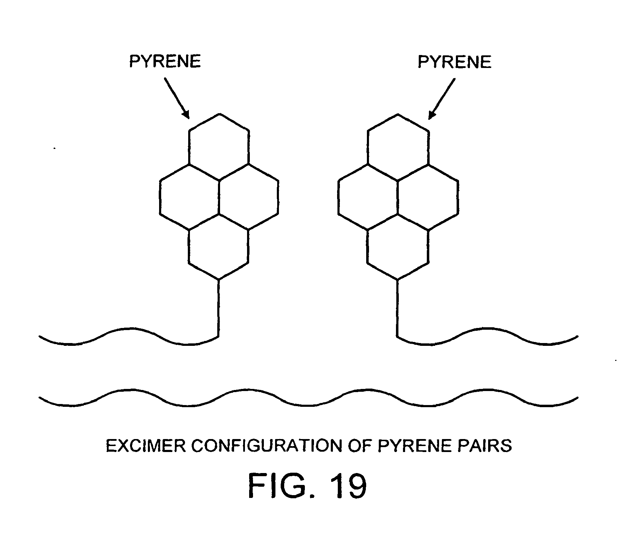

One embodiment of the technology can utilize monomer:excimer fluorescence as the output. The association of a natural binding domain with a binding partner in this format is shown in Figure 19.
The fluorophore pyrene when present as a single copy displays fluorescent emission of a particular wavelength significantly shorter than when two copies of pyrene form a planar dimer (excimer), as depicted. As above, excitation at a single wavelength (probably 340 nm) is used to review the excimer fluorescence (-470 nm) over monomer fluorescence (~375 nm) to quantify assembly:disassembly of the reporter molecule.
Additional embodiments of the present invention are not dependent on FRET. For example the invention can make use of fluorescence correlation spectroscopy (FCS), which relies on the measurement of the rate of diffusion of a label (see Elson and Magde, 1974 Biopolymers, 13: 1-27; Rigler et al., 1992, in Fluorescence Spectroscopy: New Methods and Applications, Springer Verlag, pp.13-24; Eigen and Rigler, 1994, Proc. Natl, Acad. Sci. U.S.A., 91: 5740-5747; Kinjo and Rigler, 1995, Nucleic Acids Res., 23: 1795-1799).
In FCS, a focused laser beam illuminates a very small volume of solution, of the order of $10^{-15}$ liter, which at any given point in time contains only one molecule of the many under analysis. The diffusion of single molecules through the illuminated volume, over time, results in bursts of fluorescent light as the labels of the molecules are excited by the laser. Each individual burst, resulting from a single molecule, can be registered.
A labeled polypeptide will diffuse at a slower rate if it is large than if it is small. Thus, multimerized polypeptides will display slow diffusion rates, resulting in a lower number of fluorescent bursts in any given timeframe, while labeled polypeptides which are not multimerized or which have dissociated from a multimer will diffuse more rapidly. Binding of polypeptides according to the invention can be calculated directly from the diffusion rates through the illuminated volume.
Where FCS is employed, rather than FRET, it is not necessary to label more than one polypeptide. Preferably, a single polypeptide member of the multimer is labelled. The labelled polypeptide dissociates from the multimer as a result of modification, thus altering the FCS reading for the fluorescent label.
A further detection technique which may be employed in the method of the present invention is the measurement of time-dependent decay of fluorescence anisotropy. This is described, for example, in Lacowicz, 1983, Principles of Fluorescence Spectroscopy, Plenum Press, New York, incorporated herein by reference (see, for example, page 167).
Fluorescence anisotropy relies on the measurement of the rotation of fluorescent groups. Larger multimers of polypeptides rotate more slowly than monomers, allowing the formation of multimers to be monitored.

Non-fluorescent detection methods

We describe methods which use a number of non-fluorescent labels. The natural binding domain and binding partner therefor may therefore form, when bound, an active enzyme which is capable of participating in an enzyme-substrate reaction which has a detectable endpoint. The enzyme may comprise two or more polypeptide chains or regions of a single chain, such that upon binding of the natural binding domain to the binding partner, which are present either on two different polypeptide chains or in two different regions of a single polypeptide, these components assemble to form a functional enzyme. Enzyme function may be assessed by a number of methods, including scintillation counting and photospectroscopy. In a further embodiment, the assay may be configured such that the label is a redox enzyme, for example glucose oxidase, and the signal generated by the label is an electrical signal.
Modification of the natural binding domain and, optionally, its binding partner is required to inhibit binding and, consequently, enzyme component assembly, thus reducing enzyme activity.
In another assay format, an enzyme is used together with a modulator of enzyme activity, such as an inhibitor or a cofactor. In such an assay, one of the enzyme and the inhibitor or cofactor is an natural binding domain, the other its binding partner. Binding of the enzyme to its inhibitor or cofactor results in modulation of enzymatic activity, which is detectable by conventional means (such as monitoring for the conversion of substrate to product for a given enzyme).

Fluorescent protein labels in assays of the invention

The methods described here may employ FRET. In a FRET assay, the fluorescent protein labels are chosen such that the excitation spectrum of one of the labels (the acceptor) overlaps with the emission spectrum of the excited fluorescent label (the donor). The donor label is excited by light of appropriate intensity within the donor's excitation spectrum. The donor then emits some of the absorbed energy as fluorescent light and dissipates some of the energy by FRET to the acceptor fluorescent label. The fluorescent energy it produces is quenched by the acceptor fluorescent protein label. FRET can be manifested as a reduction in the intensity of the fluorescent signal from the donor, reduction in the lifetime of its excited state, and re-emission of fluorescent light at the longer wavelengths (lower energies) characteristic of the acceptor. When the donor and acceptor labels become spatially separated, FRET is diminished or eliminated.

One can take advantage of the FRET exhibited by a natural binding domain, sequence or polypeptide and its binding partner labeled with different fluorescent proteins, wherein one is linked to a donor and the other to an acceptor fluorescent protein, in monitoring protein modification according to the present invention. A single polypeptide may comprises a blue fluorescent protein donor and a green fluorescent protein acceptor, wherein each is fused to a different assay component (i.e., in which one is fused to the natural binding domain, sequence or polypeptide and the other to its binding partner): such a construct is herein referred to as a "tandem" fusion protein. Alternatively, two distinct polypeptides ("single" fusion proteins) one comprising a natural binding domain, sequence or polypeptide and the other its binding partner may be differentially labeled with the donor and acceptor fluorescent proteins, respectively. The construction and use of tandem fusion proteins in the invention can reduce significantly the molar concentration of peptides necessary to effect an association between differentially-labeled polypeptide assay components relative to that required when single fusion proteins are instead used. The labeled natural binding domain, sequence or polypeptide and/or its binding partner may be produced via the expression of recombinant nucleic acid molecules comprising an in-frame fusion of sequences encoding a such a polypeptide and a fluorescent protein label either *in vitro* (e.g., using a cell-free transcription/translation system, as described below, or instead using cultured cells transformed or transfected using methods well known in the art) or *in vivo,* for example in a transgenic animal including, but not limited to, insects, amphibians and mammals. A recombinant nucleic acid molecule of use in the invention may be constructed and expressed by molecular methods well known in the art, and may additionally comprise sequences including, but not limited to, those which encode a tag (e.g., a histidine tag) to enable easy purification, a secretion signal, a nuclear localization signal or other primary sequence signal capable of targeting the construct to a particular cellular location, if it is so desired.

The means by which a natural binding domain, sequence or polypeptide and its binding partner are assayed for association using fluorescent protein labels according to the invention may be briefly summarized as follows:

Whether or not the natural binding domain, sequence or polypeptide and its binding partner are present on a single polypeptide molecule, one is labeled with a green fluorescent protein, while the other is preferably labeled with a red or, alternatively, a blue fluorescent protein. Useful donor:acceptor pairs of fluorescent proteins (see Tsien et al., 1997, supra) include, but are not limited to:

Donor: S72A, K79R, Y145F, M153A and T203I (excitation 395nm; emission 511)
Acceptor: S65G, S72A, K79R and T203Y (excitation 514 nm; emission 527 nm), or T203Y/S65G, V68L, Q69K or S72A (excitation 515nm; emission 527nm).

An example of a blue:green pairing is P4-3 (shown in Table 1 of Tsien et al., 1997, supra) as the donor label and S65C (also of Table 1 of Tsien et al., 1997, supra) as the acceptor label. The natural binding domain, sequence or polypeptide and corresponding binding partner are exposed to light at, for example, 368 nm, a wavelength that is near the excitation maximum of P4-3. This wavelength excites S65C only minimally. Upon excitation, some portion of the energy absorbed by the blue fluorescent protein donor is transferred to the acceptor through FRET if the natural binding domain, sequence or polypeptide and its binding partner are in close association. As a result of this quenching, the blue fluorescent light emitted by the blue fluorescent protein is less bright than would be expected if the blue fluorescent protein existed in isolation. The acceptor (S65C) may re-emit the energy at longer wavelength, in this case, green fluorescent light.

After modification of one or both of the natural binding domain, sequence or polypeptide and its binding partner by a protein modifying enzyme, the natural binding domain, sequence or polypeptide and its binding partner (and, hence, the green and red or, less preferably, green and blue fluorescent proteins) physically separate or associate, accordingly inhibiting or promoting FRET. For example, if activity of the modifying enzyme results in dissociation of a protein:protein dimer, the intensity of visible blue fluorescent light emitted by the blue fluorescent protein increases, while the intensity of visible green light emitted by the green fluorescent protein as a result of FRET, decreases.

Such a system is useful to monitor the activity of enzymes that modify a site for post-translational modification of a natural binding domain, sequence or polypeptide and, optionally, its binding partner to which the fluorescent protein labels are fused, as well as the activity of protein modifying enzymes or candidate modulators of those enzymes.

In particular, we contemplate assays in which the amount- or activity of a modifying enzyme in a sample is determined by contacting the sample with a natural binding domain, sequence or polypeptide and its binding partner, differentially-labeled with fluorescent proteins, as described above, and measuring changes in fluorescence of the donor label, the acceptor label or the relative fluorescence of both. Fusion proteins, as described above, which comprise either one or both of the labeled natural binding domain, sequence or polypeptide and its binding partner of an assay of the invention can be used for, among other things, monitoring the activity of a protein modifying enzyme inside the cell that expresses the recombinant tandem construct or two different recombinant constructs.

Advantages of single- and tandem fluorescent protein/polypeptides comprising a natural - binding domain, sequence or polypeptide fused to a fluorescent protein include the potential to express the natural binding domain, sequence or polypeptide in the cell (providing a convenient experimental format), the greater extinction coefficient and quantum yield of many of these proteins compared with those of the Edans fluorophore. Also, the acceptor in such a construct or pair of constructs is, itself, a fluorophore rather than a non-fluorescent quencher like Dabcyl. Alternatively, in single-label assays whether involving use of a chemical fluorophore or a single fluorescent fusion construct, such a non-fluorescent quencher may be used. Thus, the enzyme's substrate (i.e., the natural binding domain and, optionally, the corresponding binding partner), and reaction products (i.e., the natural binding domain and, optionally, the corresponding binding partner after modification) are both fluorescent but with different fluorescent characteristics.

In particular, the substrate and modified products exhibit different ratios between the amount of light emitted by the donor and acceptor labels. Therefore, the ratio between the two fluorescences measures the degree of conversion of substrate to products, independent of the absolute amount of either, the optical thickness of the sample, the brightness of the excitation lamp, the sensitivity of the detector, etc. Furthermore, *Aequorea*-derived or -related fluorescent protein labels tend to be protease resistant. Therefore, they are likely to retain their fluorescent properties throughout the course of an experiment.

Reporter polypeptide fusion construct

As stated above, recombinant nucleic acid constructs which may be used in the invention are those which comprise in-frame fusions of sequences encoding a natural binding domain, sequence or polypeptide or a binding partner therefor and a fluorescent protein. If a natural binding domain, sequence or polypeptide and its binding partner are to be expressed as part of a single polypeptide, the nucleic acid molecule additionally encodes, at a minimum, a donor fluorescent protein fused to one, an acceptor fluorescent protein label fused to the other, a linker that couples the two and is of sufficient length and flexibility to allow for folding of the polypeptide and pairing of the natural binding domain, sequence or polypeptide with the binding partner, and gene regulatory sequences operatively linked to the fusion coding sequence. If single fusion proteins are instead encoded (whether by one or more nucleic acid molecules), each nucleic acid molecule need only encode a natural binding domain, sequence or polypeptide or a binding partner therefor, fused either to a donor or acceptor fluorescent protein label and operatively linked to gene regulatory sequences.

"Operatively-linked" refers to polynucleotide sequences which are necessary to effect the expression of coding and non-coding sequences to which they are ligated. The nature of such control sequences differs depending upon the host organism; in prokaryotes, such control sequences generally include promoter, ribosomal binding site, and transcription termination sequence; in eukaryotes, generally, such control sequences include promoters and transcription termination sequence. The term "control sequences" is intended to include, at a minimum, components whose presence can influence expression, and can also include additional components whose presence is advantageous, for example, leader sequences and fusion partner sequences.

As described above, the donor fluorescent protein label is capable of absorbing a photon and transferring energy to another fluorescent label. The acceptor fluorescent protein label is capable of absorbing energy and emitting a photon. If needed, the linker connects the natural binding domain, sequence or polypeptide and its binding partner either directly or indirectly, through an intermediary linkage with one or both of the donor and acceptor fluorescent protein labels. Regardless of the relative order of the natural binding domain, sequence or polypeptide, its binding partner and the donor and acceptor fluorescent protein labels on a polypeptide molecule, it is essential that sufficient distance be placed between the donor and acceptor by the linker and/or the natural binding domain, sequence or polypeptide and its binding partner to ensure that FRET does not occur unless the natural binding domain, sequence or polypeptide and its binding partner bind. It is desirable, as described in greater detail in WO97/28261, to select a donor fluorescent protein with an emission spectrum that overlaps with the excitation spectrum of an acceptor fluorescent protein. In some embodiments of the invention the overlap in emission and excitation spectra will facilitate FRET. Such an overlap is not necessary, however, if intrinsic fluorescence is measured instead of FRET. A fluorescent protein of use in the invention includes, in addition to those with intrinsic fluorescent properties, proteins that fluoresce due intramolecular rearrangements or the addition of cofactors that promote fluorescence.

For example, green fluorescent proteins ("GFPs") of cnidarians, which act as their energy-transfer acceptors in bioluminescence, can be used in the invention. A green fluorescent protein, as used herein, is a protein that fluoresces green light, and a blue fluorescent protein is a protein that fluoresces blue light. GFPs have been isolated from the Pacific Northwest jellyfish, *Aequorea victoria,* from the sea pansy, *Renilla reniformis,* and from *Phialidium gregarium.* (Ward et al., 1982, Photochem. Photobiol., 35: 803-808; Levine et al., 1982, Comp. Biochem. Physiol., 72B: 77-85).

A variety of *Aequorea*-related GFPs having useful excitation and emission spectra have been engineered by modifying the amino acid sequence of a naturally occurring GFP from Aequorea victoria. (Prasher et al., 1992, Gene, 111: 229-233; Heim et al., 1994, Proc. Natl. Acad. Sci. U.S.A., 91: 12501-12504; PCT/US95/14692). As used herein, a fluorescent protein is an Aequorea-related fluorescent protein if any contiguous sequence of 200 amino acids of the fluorescent protein has at least 95% sequence identity with an amino acid sequence, either contiguous or non-contiguous, from the wild type *Aequorea* green fluorescent protein of SwissProt Accession No. P42212. Similarly, the fluorescent protein may be related to *Renilla* or *Phialidium* wild-type fluorescent proteins using the same standards.

*Aequorea*-related fluorescent proteins include, for example, wild-type (native) *Aequorea victoria* GFP, whose nucleotide and deduced amino acid sequences are presented in Genbank Accession Nos. L29345, M62654, M62653 and others Aequorea-related engineered versions of GFP, of which some are listed above. Several of these, i.e., P4, P4-3, W7 and W2 fluoresce at a distinctly shorter wavelength than wild type.

Recombinant nucleic acid molecules encoding single- or tandem fluorescent protein/polypeptide comprising a natural binding domain, sequence or polypeptide or a binding partner therefor fused to a fluorescent protein useful in the invention may be expressed for *in vivo* assay of the activity of a modifying enzyme on the encoded products. Alternatively, the encoded fusion proteins may be isolated prior to assay, and instead assayed in a cell-free *in vitro* assay system, as described elsewhere herein.

Protein modifications

We describe reagents and methods for assaying the activity of enzymes which perform post-translational modification of proteins. Table 2 lists non-limiting examples of post-translational modifications.

Table 2

| Modification | Protein Source | Consensus Sequence/ Sequence | Reference/ GenBank No. |
|---|---|---|---|
| | | **Modified residues** indicated in bold. Residues forming part of the recognition site are shown in italics. | |
| ADP-Ribosylation | B-50 | [1]MLCCMRRTKQVE KND DD | Coggins et al., 1993, J. Neurochem., 60: 368-71 |
| | subunit of cGMP phosphodiesterase | [30]FKQRQTRQFK | X04270 |
| Ubiquitination | IκB | [1]MFQAAERPQEWA MEG PRDGLKKERLLDD RH | M69043 |
| | -Galactosidase | HGSGAWLLPVSLV KR KTTLAP | Johnson et al., 1990, Nature, 346: 287-291 |
| N-Myristoylation | Src | [1]GSSKSKPKD | Resh, 1994, Cell, 76: 411-413 |
| | Lyn | [1]GCIKSKPKD | Resh, 1994, supra |
| | Yes | [1]GCIKSKEDK | Resh, 1994, supra |
| | Fyn | [1]GCVQCKDKE | Resh, 1994, supra |
| | Gα | [1]GCTLSAEDK | Resh, 1994, supra |
| Palmitylation | Lyn | [1]GCIKSKRKD | M64608 |
| | Fyn | [1]GCVQCKDKE | M14676 |
| | Gαi2 | [1]GCTLSAEDK | Milligan et al., 1995, Trends Biochem. Sci., 20: 181-186 |
| N-Glycosylation | | *NXS*/*T*- X can be any amino acid except P | Shakineshleman, 1996, Trends in Glycoscience and Glycotechnology, 8: 115-130 |
| O-Glycosylation | p67[SRF] | [274]GTTSTIQTAP [313]SAVSSADGTVLK [374]DSSTDLTQTSSSG TVTLP | J03161 |
| Sentrinization | RanGAP1 | | Johnson and Hochstrasser, 1997, Trends Cell.Biol., 7: 408-413 |
| | PML | | Kamitani et al., 1998, J. Biol. Chem., 273: 3117-3120 |

Phosphorylation - kinase and phosphatases

A particularly important post-translational modification for which a large number of enzymes and targets have been identified is phoshorylation and dephosphorylation. The art is replete with references to said enzymes, i.e. protein kinases and phosphatases, and their targets, including consensus phosphorylation motifs (such as -SQ- or -TQ- for the DNA dependent protein kinase (DNA-PK).

Some non-limiting examples of kinases and their sites for post-translational modification are presented in Table 2B (phosphorylation/dephosphorylation)

Table 2B

| Kinase | Consensus Sequence | GenBank No./Reference |
|---|---|---|
| cAMP-dependent protein kinase | -RRXRRXS/T- | C$\alpha$ subunit M34181 RII$\beta$ subunit M31158 |
| Myosin Heavy Chain kinase | -KXXSX- | Trends in Biochem. Sci. (1990) 15 342-346. |
| Myosin Heavy Chain kinase | -RXT- | M93393 |
| Calmodulin-Dependent protein kinase II | -RXXSX- | $\alpha$ chain J02942 $\beta$ chain M16112 $\gamma$ chain J05072 $\delta$ chain J04063 |
| cGMP-dependent protein kinase | -XSRX- | $\beta$ isozyme Y07512 |
| Protein kinase C | -XRXXSXRX- | Trends in Biochem. Sci.(1990) 15 342-346. |
| S6 kinase II | -XRXXSX- | $\alpha$2 isozyme L07599, L07601 |
| dsRNS kinase pp68 | -SELSRR- | Trends in Biochem. Sci.(1990) 15 342-346. |
| Casein kinase I | -XSXXSX- | $\alpha$ isoform X80693 |
| Mammary gland casein kinase | -XSXEX- | Trends in Biochem. Sci.(1990) 15 342-346. |
| Glycogen synthase kinase 3 | -XSXXSX | $\alpha$ isoform L40027 |

X signifies any amino acid. Consensus sequences are taken from Trends Biochem. Sci. (1990) 15: 342-346.

Further examples of protein kinases identified to date include the protein tyrosine kinase subfamily (such as PDGF receptors, EGF receptors, src family kinases (see Brown and Cooper, 1996, Biochimica and Biophysica Acta 1287: 121-149 for a review), the JAK kinase family (such as JAK1, JAK2 and tyk2), Erb B2, Bcr-Abl, Alk, Trk, Res/Sky- for a detailed review see Al-Obeidi *et al.* 1998, Biopolvmers (Peptide Science), Vol 47: 197-223), the MAP kinase pathway subfamily (such as the MAP family, the ERK family, the VIEI< family, the MEKK. family, RAF-1 and JNK), the cvclin-dependent kinase subfamily (such as p34[cdc2] and cdk2 - see Nigg, 1995, Bioessays 17: 471-480 for a review). Weel/Mytl, polo-like kinases (such as p1kl, P1x1. POLO, Snk, Fnk/Prk Sak-a, Sak-b - see Lane and Nigg, 1997, Trends in Cell Biol. 7: 63-68), the receptor serine kinase subfamily, protein kinase C (PK-C), cyclic-AMP dependent kinase (PK-A), cyclic-GMP dependent kinase. Ca2+/calmodulin dependent kinases (such as CaM kinase I, II and IV), DNA dependent protein kinase,), phosphoinositide 3-kinases, PDK-1, the p21-activated protein kinase family (PAKs), such as Pak1, Pak2 and Pak3- see Sells and Chernoff, 1997, Trends in Cell Biol. 7: 162-167), p70 S6 kinase, IkB kinase, casein kinase II, glycogen-synthase kinases.

A discussion of particular kinase pathways involved in signal transduction is given in chapter 35 of Lewin, 1997, Gene VI, Oxford University Press.

Details of recognition and binding domains for a variety of kinases are given in Kuriyan and Cowburn, 1997, Annu. Rev. Biophys. Biomol. Struc. 26:259-288.

Some specific examples of kinases whose activity may be studied using the methods of the invention include the src family tyrosine kinases Lck and Fyn. that phosphorylate the TCR $\zeta$ chain, and are known to be involved in signal transduction associated with T cell receptor stimulation. The TCR $\zeta$ chain comprises specific tyrosine residues present in immunoreceptor tyrosine-based activation motifs (ITAMs) that are phosphorylatd by Lck and Fyn (Kuriyan and Cowbum, 1997, *ibid*.). The SH2 domain of another tyrosine kinase, ZAP70 binds to phosphorylated TCR $\zeta$. Thus TCR $\zeta$ ITAM and ZAP70 SH2 represent binding domains and binding partners that may be of interest in studying the activity of the kinases Lck and Fyn (see Elder et al., 1994. Science 264: 1596-1599 and Chan et al., 1994, Science 264: 1599-1601.

Another example is the IgE receptor $\gamma$ subunit and the SH2 domain of Syk that may be used to study the activity of the Lyn kinase.

Examples of phosphatase identified to date fall into three main families (for review see Barford et al., 1998, Annu. Rev. Biophys. Biomol. Struc. 27: 133-164). The PPP family includes the following catalytic subunits: PPlc, PP2Ac, PP2B, PPP1, PPP2A and PPP5 and the following regulatory subunits: MPP-1, RIPP-1, p53BP2, γ,34.6, PR65, PR55. PR72, PTPA, SV40 small T antigen, PPY, PP4, PP6 and PP5.

The PPM family includes pyruvate dehydrogenase phosphatase and *Arabidopsis* ABI1.

The protein tyrosine phosphatase family includes PTP1B, SHP-1, SHP-2 (cytosolic non-receptor forms), CD45 (see Thomas and Brown, 1999, Trends in Immunol, 20: 406 and Ashwell and D'Oro, 1999, Trends in Immunol, 20: 412 for further details), RPTP (receptor-like, transmembrane forms) and cdc25, kinase-associated phosphatase and MAP kinase phosphatase-1 (dual-specificity phosphatases). PTP1B is known to associate with the insulin receptor *in vivo* (Bandyopadhyay *et al.,* 1997, J. Biol. Chem. 272: 1639-1645).

A simple FRET assay based upon these modifications to site for post-translational modification present on a natural binding domain may be performed as presented below. It is contemplated that other light-based detection assays, such as those involving single labels, labels and corresponding quenchers, etc. can be employed.

$$(\text{F1-NBD})(\text{F2-partner}) + \text{substrate} \rightarrow \text{F1-M-NBD} + \text{F2-partner} + \text{byproduct}$$
$$(\text{FRET}) \qquad\qquad (\text{No FRET})$$

where: NBD = natural binding domain, M = modification
F 1 = donor fluorophore, F2 = acceptor fluorophore

Alternatively, a FRET-based assay may follow a format such as:

$$\text{F1-NBD} + \text{F2-partner} + \text{substrate} \rightarrow (\text{F1-M-NBD})(\text{F2-partner}) + \text{byproduct}$$
$$(\text{No FRET}) \qquad\qquad (\text{FRET})$$

Table 3 provides a non-limiting list of enzymes that are representative of the classes of modifying enzymes discussed herein as being amenable to assay according to the invention.

**Table 3**

| Modification | Enzyme | Specific Action | GenBank No./ Reference |
|---|---|---|---|
| Mono-ADP-Ribosylation | NAD:ArginineADP-ribosyl transferase | | Zolkiewska et al., 1992, <u>PNAS</u> B2: 11352-6 |
| Poly- ADP-Ribosylation | *Drosophila* PARP | | D13806, D13807, D13808 |
| Ubiquitination | E1 E2(UBC8) E3(RSP5) | Ubiquitination of large subunit of RNA pol II (Rpb 1) (NB, E2 and E3 confer substrate specificity on the ubiquitination) | X55386, X56507, M65083, U18916, L11119, L11120, U00092, U75972 |
| N-Nlyristoylation | Glycylpeptide-N-tetradecanoyltransferase (peptide N-myristoyltransferase) | | M86707 |

(continued)

| Modification | Enzyme | Specific Action | GenBank No./ Reference |
|---|---|---|---|
| N-Glycosylation | UDP-N-acetylalucosamine-dolichyl-phosphate N-acetylglucosamine phosphotransferase | Initial step in synthesis of dolichol-P-P-olieosacharides | X65603, S41875 |
| O-Glycosylation | O-GlcNAc transferase | | Kreppel et al., 1997. J. Biol. Chem., 272: 9308-9315 |
| Sentrinization | Ubc9 | | Gong et al.. 1997, J. Biol. Chem., 272. 28198-28201 |

The several types of post-translational modification presented above will be discussed in some detail below, along with assays to test the enzymes that perform such modifications using natural binding domains and binding partners therefor according to the invention.

ADP-ribosylation

Mono-ADP-ribosylation is a post-translational modification of proteins which is currently thought to play a fundamental role in cellular signalling. A number of mono-ADP-ribosyl-transferases have been identified, including endogenous enzymes from both bacterial and eukaryotic sources and bacterial toxins. A mono-ADP-ribosylating enzyme, using as substrates the protein to be modified and nicotinamide adenine dinucleotide ($NAD^+$), is NAD:Arginine ADP ribosyltransferase (Zolkiewska et al., 1992, Proc. Natl. Acad. Sci. U.S.A., 89: 11352-11356). The reactions catalyzed by bacterial toxins such as cholera and pertussis toxin are well understood; the activities of these toxins result in the permanent modification of heterotrimeric G proteins. Endogenous transferases are also thought to modify G proteins and therefore to play a role in signal transduction in the cell (de Murcia et al., 1995, Trends Cell Biol., 5: 78-81). The extent of the effects that ADP-ribosylation can mediate in the cell is illustrated by the example of brefeldin A, a fungal toxin metabolite of palmitic acid. This toxin induces the mono-ADP-ribosylation of BARS-50 (a G protein involved in membrane transport) and glyceraldehyde-3-phosphate dehydrogenase. The cellular effects of brefeldin A include the blocking of constitutive protein secretion and the extensive disruption of the Golgi apparatus. Inhibitors of the brefeldin A mono-ADP-ribosyl-transferase reaction have been shown to antagonise the disassembly of the Golgi apparatus induced by the toxin (Weigert et al., 1997, J. Biol. Chem., 272: 14200-14207). A number of amino acid residues within proteins have been shown to function as ADP-ribose acceptors. Bacterial transferases have been identified which modify arginine, asparagine, cysteine and diphthamide residues in target proteins. Endogenous eukaryotic transferases are known which also modify these amino acids, in addition there is evidence that serine, threonine, tyrosine, hydroxyproline and histidine residues may act as ADP-ribose acceptors but the relevant transferases have not yet been identified (Cervantes-Laurean et al., 1997, Methods Enzymol., 280: 275-287 and references therein).

Poly-ADP-ribosylation is thought to play an important role in events such as DNA repair, replication, recombination and packaging and also in chromosome decondensation. The enzyme responsible for the poly-ADP-ribosylation of proteins involved in these processes is poly (ADP-ribose) polymerase (PARP; for *Drosophila melanogaster* PARP, see Genbank Accession Nos. D13806, D13807 and D13808). The discovery of a leucine zipper in the self-poly(ADP-ribosyl)ation domain of the mammalian PARP (Uchida et al., 1993, Proc. Natl. Acad. Sci. U.S.A., 90: 3481-3485) suggested that this region may be important for the dimerization of PARP and also its interaction with other proteins (Mendoza-Alvarez et al., 1993, J. Biol. Chem., 268: 22575-22580).

Specific examples of ADP ADP-ribosylation sites are those found at $Cys_3$ and $Cys_4$ (underlined) of the B-50 protein (Coggins et al., 1993, J. Neurochem., 60: 368-371; SwissProt Accession No. P06836):
MLCCMRRTKQVEKNDDD
and Pγ (the γ subunit of cycylic CMP phophodiesterase; Bondarenko et al., 1997, J. Biol. Chem., 272: 15856-15864; Genbank Accession No. X04270):
FKQRQTRQFK.

Two non-limiting examples of assays of enzymatic activity according to the invention which assays are based upon the detection of changes in ADP-ribosylation-dependent protein:protein binding are briefly summarized as follows:

Assay 1:

This assay employs as reporter molecules the following:

Retinal rod cGMP phosphodiesterase $\gamma$ subunit (P$\gamma$; whole protein, or as little as amino acids 19-87. mutated to remove the phosphorylation site at Thr$_{22}$; Bondarenko, 1997, J. Biol. Chem., 272: 15856-15864; Tsuboi et al., 1994, J. Biol. Chem., 269: 15016-15023; OWL accession no. P04972; Genbank accession no. X04270)

Transducin $\alpha$ subunit (T$\alpha$; whole protein, or as little as amino acids 293-314, acetyl-EDAGNYIKVQFLELNMRRDVKE-amide; Rarick et al., 1992, Science, 256: 1031-1033; OWL accession no. P04695; Genbank no. K03254).

These proteins are components of the vertebrate light-response system, which includes transducin (a heterotrimeric G protein), a cGMP-specific phosphodiesterase (PDE) and rhodopsin. Analogous components can be identified in a number of G protein-coupled signalling systems. T$\alpha$-GTP activates its effector, cGMP-PDE, by binding to the inhibitory subunits of that protein and thereby relieving inhibition its enzymatic activity (Stryer, 1986, Ann. Rev. Cell Biol., 2: 391-419). It has been shown that the ADP-ribosylation of P$\gamma$ at Arg$_{33}$ or Arg$_{36}$ occurs when P$\gamma$ is complexed with P$\alpha\beta$ but not when it is complexed with T$\alpha$ (Bondarenko et al., 1997, supra). It has been suggested that the sites of ADP-ribosylation are masked in the P$\gamma$ -T$\alpha$ complex. This assay for ADP-ribosylation can be adapted to detect the de-ADP-ribosylation of P$\gamma$. Problems arise because the affinity of P$\gamma$ for its alternative partners is affected by other factors including the nucleotide bound to T$\alpha$ (T$\alpha$ -GTP has higher affinity for P$\gamma$ than does T$\alpha$ -GDP) and the phosphorylation state of P$\gamma$. This can be overcome by using a T$\alpha$ peptide to avoid the effects of nucleotide exchange and by using a P$\gamma$ peptide lacking the relevant phosphorylation site (Thr$_{22}$; Tsuboi et al., 1994, supra). An 4/ 6 loop peptide (amino acids 293-314) has a high affinity for P$\gamma$ (Noel et al., 1993, Nature, 366: 654-663). The ability of this system to be used as an assay for the ADP-ribosylation reaction depends upon the affinity of the T$\alpha$ peptide (amino acids 293-314) for P$\gamma$ (amino acids 19-87). If binding is too tight, a shorter peptide which has also been reported to stimulate PDE activity (i.e. associate with P$\gamma$; Rarick et. al., 1992, supra) can be used instead.

The design for such an assay is:

$$(T\alpha\text{ -F1})(P\gamma\text{ -F2}) + NAD^+ \;\rightarrow\; T\alpha\text{ -F1} + (ADP\text{-ribose})P\gamma\text{ -F2}$$

$$\text{FRET} \qquad\qquad\qquad\qquad \text{No FRET}$$

where F1 is the donor fluorophore and F2 is the acceptor.

Placement of chemical fluorophores:

There are several lysine residues close to the modified arginine residues in the P$\gamma$ sequence. Candidates for labelling include K$_{25}$, K$_{41}$, K$_{44}$ and K$_{45}$. The protein has a single C residue, C$_{68}$. Labelling here may simplify the addition of a single fluorophore at a known site.

The T$\alpha$ peptide has two lysine residues which can be labeled, K$_{300}$ and K$_{313}$. The most appropriate labelling site pairings can be selected based upon such structural information as is available and/or by the empirical testing of labels at various combinations of sites.

Placement of a fluorescent protein label:

GFP of another fluorescent protein can be fused to a natural binding domain, sequence or polypeptide and/or its binding partner at either of the C- and N-termini of the two molecules followed by empirical detection of the labeled polypeptides in control protein binding reactions.

Assay 2:

The following component is employed in a homodimerization assay:

*Drosophila* PARP (whole protein or amino acids 369-994, lacking the zinc finger DNA binding domain; Uchida et al., 1993, Proc. Natl. Acad. Sci. U.S.A., 90: 3481-3485; OWL accession no. P35875; Genbank accession nos. D13806, D13807 and D13808).

*Drosophila* PARP possesses a leucine zipper motif in the self-poly(ADP-ribosyl)ation domain which is also found in the bovine, mouse, chicken and human sequences. Two conserved glutamate residues are predicted to be poly(ADP-ribosylation) automodification sites. It has been suggested that poly(ADP-ribosylation) of these sites results in dissociation of the dimer due to the large negative charge of the polymer (Mendoza-Alvarez et al., 1993, supra). A catalytic dimer is required for the reaction to proceed as the automodification reaction has been shown to be intermolecular (Mendoza-Alvarez et al., 1993, supra). The leucine zipper domain is predicted to also mediate heterodimerization of PARP; thus, other poly(ADP-ribosylating) binding partners may be useful in this assay of the invention.

Thus the assay would be:

$$(\text{PARP-F1})(\text{PARP-F2}) \ (\text{PARP-F1}) \rightarrow (\text{poly ADP-R}) + (\text{PARP-F2})(\text{poly ADP-R})$$

FRET                No FRET

Active dimer              Inactive monomers

where F1 is the donor fluorophore and F2 is the acceptor.

In reporter molecules of such an assay, chemical fluorophores are located on residue $K_{416}$, just outside the leucine zipper, or on residues in the b, c or f positions of the leucine zipper (e.g. $K_{389}$). If a fluorescent protein (e.g., GFP) is used, it is placed at the N-terminus of the truncated PARP molecule.

Ubiquitination

Ubiquitination of a protein targets the protein for destruction by the proteosome. This process of destruction is very rapid ($t_{1/2} \sim$ 60 seconds), and many proteins with rapid turnover kinetics are destroyed *via* this route. These include cyclins, p53, transcription factors and transcription regulatory factors, among others. Thus, ubiquitination is important in processes such as cell cycle control, cell growth, inflammation, signal transduction; in addition, failure to ubiquitinate proteins in an appropriate manner is implicated in malignant transformation. Ubiquitin is a 76-amino-acid protein which is covalently attached to a target protein by an isopeptide bond, between the -amino group of a lysine residue and the C-terminal glycine residue of ubiquitin. Such modification is known as mono-ubiquitination, and this can occur on multiple Lys residues within a target protein. Once attached, the ubiquitin can itself be ubiquitinated, thus forming extended branched chains of polyubiquitin. It is this latter state which signals destruction of the target protein by the proteosome. In the process of destruction, it appears that the polyubiquitinated protein is taken to the proteosome *via* a molecular chaperone protein, the ubiquitin molecules are removed undamaged (and recycled) and the target is degraded.

Ubiquitination is a complex process, which requires the action of three enzymes: Ubiquitin activating enzyme E1 (for human, Genbank Accession No. X56976), ubiquitin conjugating enzyme E2, also referred to as the ubiquitin carrier protein, (for human 17kDa form, Genbank Accession No. X78140) and Ubiquitin protein ligase E3 (UBR1; human, Genbank Accession No. AF061556). There are multiple forms of each of these enzymes in the cell, and the above examples are, therefore, non-limiting.

Two examples of ubiquitination sites from natural proteins, IκB (Dai et al., 1998, J. Biol. Chem, 273: 3562-3573: Genbank Accession No. M69043) and β-galactosidase (Johnson et al., 1990, Nature, 346: 287-291) are as follows:
IκB NH$_3$-MFQAAERPQEWAMEGPRDGLKKERLLDDRH-COOH
β-galactosidase NH$_3$-HGSGAWLLPVSLVKRKTTLAP-COOH
where the ubiquitinated lysine residue is underlined for each (e.g., $Lys_{15}$ and $Lys_{17}$ for β-galactosidase).

According to the invention, a ubiquitination assay measures the addition of ubiquitin to-, rather than the destruction of-, a natural binding domain, sequence or polyeptpide.

Such an assay is summarized briefly below.

The assay components are as follows:

NFκB IκB and VCP

NFκB is a transcription factor held in the cytoplasm by the tight association with an inhibitor protein IκB ., or other members of this protein family (Baldwin, 1996, Ann. Rev. Biochem., 14: 648-681). A variety of signals prompt the release of NFκB from IκB and the subsequent movement of NFκB to the nucleus, where it functions as a transcription factor. IκB is the first phosphorylated on two residues ($Ser_{32}$ and $Ser_{36}$). which prompts the ubiquitination of IκB on $Lys_{21}$ and other residues; such modification marks IκB for destruction by the proteosome (Dai et al., 1998, J. Biol. Chem.. 273: 3562-3573). It has been suggested that following ubiquitination of IκB. a molecular chaperone protein VCP binds to IκB and displaces NFκB (Dai et al., 1998, supra), after which it is surmised that VCP transports IκB to the proteosome for destruction. A fragment of IκB (amino acids 1-242) can participate in the early stages of the above process (i.e., it becomes phosphorylated and ubiquitinated, and binds VCP), but is not then destroyed by proteolysis (Dai, et al., 1998, supra).

An assay for ubiquitination, in this non-limiting example using the natural binding domains found in this pathway, would be:

$$F1\text{-}(NF\kappa B)(I\kappa B)\text{-}F2 + VCP + ATP + Ub \rightarrow F1\text{-}(NF\kappa B) + P,Ub\text{-}(I\kappa B)\text{-}F2(VCP) + ADP$$

F1 is the donor fluorophore, F2 the acceptor, P is the phosphorylation and Ub is ubiquitin.

If the assay is to be constructed using fragments of the full length proteins, those which comprise natural binding domains (i.e., those which are "binding sequences", as defined above) then the regions of NFκB and IκB of interest are:

For the NFκB p65:p50 heterodimer:

p65 (amino acids 12-317) as described as capable of binding IκB (Jaffray et al., 1995, Mol. Cell. Biol., 15: 2166-2172). p50 (amino acids 39-364; Ghosh et al., 1995, Nature, 373: 303-310). This represents the murine sequence (Accession No. M57999, M37732). As an alternative, the human p50 (amino acids 2-366; Muller et al., 1995, Nature, 373: 311-317) can be employed.

For IκB and VCP:

IκB (amino acids 1-305: as deduced from the data of Bell et al.. 1996. Mol. Cell. Biol., 16: 6477-6485). The amino acid sequence of IκB has been described (Davis et al., 1991. Science, 253: 1268-1271). The acidic domain of IκB, which includes residues 275-300, is required for effective binding to NFκB . VCP, which is an optically inactive part of this assay (i.e. not fluorescently labeled), is used as a whole protein, as functional dissection of VCP has not been described to date.

Placement of fluorophores (chemical):

Prior to use in an assay of the invention, a natural binding domain and binding partner used to examine ubiquitination should be labeled at a number of positions and the distance between F1 and F2 optimized empirically. In order to do this, existing residues which can be conjugated with fluorescent dyes may be used. Alternatively, new residues may be introduced by site-directed mutagenesis for the purpose of conjugation with fluorescent dyes; however, such alterations must be made in residues which are not a part of the natural binding domain.

Potential labelling sites on p50 (human sequence) include: $K_{278}$, $K_{275}$, $K_{252}$, $K_{148}$. These residues appear to be close to the NLS site (Bell et al., 1996, supra), which is believed to form part of the IκB binding feature, but they are not protected by IκB binding, thus modification at these positions to accommodate a label should not interfere with binding.

Potential labelling sites on IκB : Binding sites for NFκB appear to be present within the region of amino acids 200-300 of IκB (Jaffray et al., 1995, supra). Cys or Lys residues within this region may be used for label attachment, providing they are not within the acidic domain (residues 275-300), which is instrumental in protein:protein binding. Good candidate sites include $K_{242}$, $K_{249}$ and $C_{219}$.

Where to attach a fluorescent protein:

For P50 of NFκB (murine amino acids 39-364, human amino acids 2-366 or alternatively, an intact human or murine protein), the fluorescent protein is fused at the C-terminus of P50. As IκB binds at the C-terminus of NFκB, the fluorescent protein (e.g., GFP) needs to be in close proximity to the binding site.

For IκB (full protein or amino acids 1-305) the fluorescent protein is placed at the C-terminus. NFκB binds at the C-terminus of IκB ; therefore, the fluorescent protein should be close to the binding site.

A second assay is configured as follows:

$$\text{NF}\kappa\text{B (I}\kappa\text{B )-F2 + VCP-F1} \rightarrow \text{NF}\kappa\text{B +P, Ub-(I}\kappa\text{B )-F2(VCP)-F1}$$

$$\text{No FRET} \qquad\qquad \text{FRET}$$

In this instance, the NFκB is either an intact protein or a partial protein, as described above (see first assay). It is not fluorescently labeled in this assay.

The portion of IκB of use in the assay is that encompassing amino acids 1-242, as a polypeptide of this sequence can be ubiquitinated, but will not proceed to proteosome destruction (see above; Dai et al., 1998, supra).

The full-length VCP protein is used.

Positioning of the fluorophores (chemical): IκB is labeled as discussed above in Assay 1. For VCP, the optimal K or C residues for label attachment must be determined empirically.

Position of a fluorescent protein: The C terminus of IκB is fused to the fluorescent protein, as above.

For VCP, the relative suitability of N-terminal and C-terminal attachment of a fluorescent protein (e.g., GFP or a variant thereof) is determined empirically. Alternatively, as structural analysis of VCP is further advanced, the fluorescent protein can be attached to sites outside of the binding domain.

A third format by which to assay ubiquitination according to the invention involves the binding of $(\text{SINA})_2$, and PHYL to TTK and the subsequent ubiquitination of TTK (and degradation; Li et al., 1997, <u>Cell</u>, 90: 469-478). These proteins, which are located in the nucleus, are used whole in assays of the invention.

$$(\text{SINA})_2\text{PHYL-F1 + TTK-F2 + ATP + UB} \rightarrow (\text{SINA})_2\text{PHYL-F1:TTK-F2-Ub + ADP}$$

$$\text{No FRET} \qquad\qquad \text{FRET}$$

Glycosylation

N-linked glycosylation is a post-translational modification of proteins which occurs in the endoplasmic reticulum and Golgi apparatus and is utilized with some proteins *en route* for secretion or destined for expression on the cell surface or in another organelle. The carbohydrate moiety is attached to Asn residues in the non-cytoplasmic domains of the target proteins, and the consensus sequence (Shakineshleman, 1996, <u>Trends Glycosci. Glycotech.</u>, 8: 115-130) for a glycosylation site is NxS/T, where x cannot be proline or aspartic acid. N-linked sugars have a common five-residue core consisting of two GlcNAc residues and three mannose residues due to the biosynthetic pathway. This core is modified by a variety of Golgi enzymes to give three general classes of carbohydrate known as oligomannosyl, hybrid and lactosamine-containing or complex structures (Zubay, 1998, <u>Biochemistry,</u> Wm. C. Brown Publishers). An enzyme known to mediate N-glycosylation at the initial step of synthesis of dolichyl-P-P-oligosaccharides is UDP-N-Acetylglucosamine-Dolichyl-phosphate-N-acetylsglucosamine phosphotransferase (for mouse, Genbank Accession Nos. X65603 and S41875).

Oxygen-linked glycosylation also occurs in nature with the attachment of various sugar moieties to Ser or Thr residues (Hansen et al., 1995, <u>Biochem. J.</u>, 308: 801-813). Complex O-linked glycosylation can be broken into at least six classes - mucin type, ser-1-GlcNAc; proteoglycan type, ser-Gal-Gal-Xyl core; collagen type hydroxylys-Gal-Glc; clotting factor type, ser-Xyl-Glc or ser-Xyl-Xyl-Glc core; fungal type, ser-Man; plant type, hydroxypro-Ara or ser-Gal (where GlcNAc = N-acetylglucosamine. Gal = galactose. Xyl = Xylose; Glc = glucose, Man = mannose and Ara = arabinose: Hansen et al., 1995. supra). Intracellular proteins are among the targets for O-glycosylation through the dynamic attachment and removal of O-N-Acetyl-D-glucosamine (O-GlcNAc: reviewed by Hart, 1997, <u>Ann. Rev. Biochem.,</u> 66: 315-335). Proteins known to be O-glycosylated include cytoskeletal proteins, transcription factors, the nuclear pore protein complex, and tumor-suppressor proteins (Hart, 1997, supra). Frequently these proteins are also phosphoproteins, and there is a suggestion that O-GlcNAc and phosphorylation of a protein play reciprocal roles. Furthermore, it has been proposed that the glycosylation of an individual protein regulates protein:protein interactions in which it is involved.

Specific sites for the addition of O-GlcNAc are found, for example, at $\text{Ser}_{277}$, $\text{Ser}_{316}$ and $\text{Ser}_{383}$ of p67[SRF] (Reason et al., 1992, <u>J. Biol. Chem.,</u> 267: 16911-16921; Genbank Accession No. J03161). The recognition sequences encompassing these residues are shown below:

[274] GTT<u>S</u>TIQTAP
[313] SAV<u>S</u>SADGTVLK
[374]DSS<u>T</u>DLTQTSSSGTVTLP

The identity of sites of O-GlcNAc is additionally known for a small number of proteins including c-myc ($Thr_{58}$, also a phosphorylation site; Chou et al., 1995, J. Biol. Chem., 270: 18961-18965), the nucleopore protein p62 (see Reason et al., 1992, supra):
MAGGPADTSDPL and band 4.1 of the erythrocyte (see Reason et al., 1992, supra):
AQTITSETPSSTT.
The site at which modification occurs is, in each case, underlined. The reaction is mediated by O-GlcNAc transferase (Kreppel et al., 1997, J. Biol. Chem., 272: 9308-9315).
Several non-limiting examples of assay formats useful in the monitoring of glycoslating enzymes according to the invention may be summarized as follows:

Assay 1:

The reporter polypeptides of a first glycosylation assay are:

Chicken hepatic lectin (amino acids 49-207, predicted extracellular domain; Burrows et al., 1997, Biochem. J., 324: 673-680; OWL accession no. P02707; Genbank accession no. M63230)

c-Myc (amino acids 1-143, N-terminal transactivation domain; Chou et al., 1995. J. Biol. Chem., 270: 18961-18965; OWL accession no. PO1107; Genbank accession no. V00568)

Chicken hepatic lectin (CHL) is a type II transmembrane protein which shows almost complete specificity for N-acetylglucosamine, which residue it binds by the C-terminal, extracellular carbohydrate-recognition domain (CRD). The intact receptor probably consists of a trimer of polypeptides stabilized by a coiled-coil structure formed by the transmembrane region and the stalk immediately N-terminal to the (CRD). Molecular modeling suggests, however, that the sugar-binding site is formed by a single polypeptide (Burrows at al., 1997, supra). It is likely that glycosylated c-Myc will bind to the O-GlcNAc at $Thr_{58}$.
c-Myc is a proto-oncogene product playing a role in the control of gene transcription. Mutation or deregulation of the expression of this protein can contribute to malignant transformation of cells. The O-GlcNAcylation of c-Myc at $Thri_{58}$ is thought to be reciprocal to phosphorylation at this site which is also a site of frequent mutation in human lymphomas (Chou et al., 1995, supra).
In addition to its utility in enzymatic assays of the invention, this assay for O-Glc-NAcylation can be adapted to monitor the modification of the large number of cytoplasmic and nuclear proteins thought to undergo O-Glc-NAcylation (Hart, 1997, Ann. Rev. Biochem., 66: 315-35).

Thus, the assay would be:

$$\text{c-Myc-F1} + \text{CHL-F2} + \text{UDP-N-acetylglucosamine} \longrightarrow (\text{GlcNAc-c-Myc-F1})(\text{CHL-F2}) + \text{UDP}$$

No FRET                    FRET

where F I is the donor fluorophore, and F2 the acceptor fluorophore.

Placement of chemical fluorophores:

From the molecular model of interaction between CHL and N-acetylglucosamine (based on the MBP-C crystal structure, Burrows et al., 1997, supra; Weis et al., 1992, Nature, 360: 127-134), the binding site is thought to be in the C-terminal region of the domain. Possible labelling sites include $K_{166}$, $E_{167}$, $E_{202}$ and $K_{207}$.
In the absence of complete structural data for c-Myc, labelling sites must be assayed emprically. $K_{51}$ and $K_{52}$ are close to the glycosylated site, and so labelling at these sites may interfere with the interaction between c-myc and CHL. Other candidate sites include $E_{39}$, $E_{47}$, $R_{66}$, $R_{83}$, $K_{126}$ and $K_{143}$.

Placement of a fluorescent protein:

In reporter molecules of this glycosylation assay, a fluorescent protein (e.g., Green Fluorescent Protein, GFP) would be placed at the C-terminus of the CHL domain and probably at the N-terminus of the c-Myc transactivation domain. Because in the c-Myc transactivation domain the site of glycosylation is almost equidistant in primary structure from the termini, the most appropriate site would need to be determined empirically in the absence of structural information.

Assay 2:

A second glycosylation assay of use in the invention comprises the following reporter molecules:

Tau (Wang et al., 1996, Nature Medicine, 2: 871-875; OWL accession no. P10636; Genbank accession no. X14474)
*Galanthus nivalis* agglutinin (Wang et al., 1996, supra; Hester et al., 1995, Nature *Structural* Biology, 2: 472-479; OWL accession no. P30617; Genbank accession no. M55556)

Tau from the brains of patients with Alzheimer's Disease (AD) has been shown to be glycosylated (determined by lectin binding on Western blots), whereas tau from normal brain tissue shows no sign of such glycosylation. This abnormal post-translational modification has been shown to play a role in the maintenance of the helical twists in the paired helical filament (PHF) structures formed by tau in the AD neuron (Wang et al., 1996, supra); various lectins were used in this study to identify different carbohydrate moieties on the tau protein. *Galanthus nivalis* agglutinin (GNA) primarily recognizes terminally-linked mannose residues. The use of this protein as an assay for the modification of Tau permits monitoring of the addition of a terminal mannose residue to a carbohydrate chain on this protein. According to certain embodiments of this assay of the invention, the addition of other residues can be monitored by substituting other lectins, with different sugar recognition specificities than that of GNA, for GNA.

The crystal structure of GNA has been determined (Hester at al., 1995, surpa), this indicates that the protein consists of a dimer of dimers with the high affinity mannose binding site formed at the interface between the A and D or, alternatively, B and C subunits. This binding site consists of -strands donated from the N- and C-terminal regions of one subunit (residues 1-6 and 82-101) and also the C-terminal -strand from the partner subunit (residues 102-109). It is suggested that the domain structure is indicative of a covalent link between subunits during the evolution of this tetramer (Hester at al., 1995, surpa). The assay can, therefore, be configured using the whole tetrameric assembly with a fluorescent label on only one subunit or potentially as a covalent dimer of subunits, again with one label.

An assay of this type is diagrammed as follows:

$$\text{GNA-F1} + \text{CHO-tau-F2} + \text{mannose} \rightarrow (\text{GNA-F1})(\text{mannose-CHO-tau-F2})$$
$$\text{No FRET} \qquad\qquad \text{FRET}$$

where F1 is the donor fluorophore and F2 is the acceptor. CHO represents the glycosylation of tau prior to addition of the terminal mannose.

Tau protein contains a number of residues which can be labeled, of which the most favorable must be determined empirically.

A number of potential labelling sites exist within the GNA subdomain which participates in high-affinity mannose binding. These include $D_1$, $K_{90}$ (which residue is very close to the site of interaction with the mannose residue), $D_{100}$ and $R_{101}$. Alternatively, a short C-terminal extension can bear a labelling site.

As above, the site for fusion of GFP to tau is to be determined empirically. Labelling of the GNA subdomain on either the C- or N-terminus is equally effective.

Prenylation (fatty acylation)

The post-translational modification of proteins with fatty acids includes the attachment of myristic acid to the primary amino group of an N-terminal glycine residue (Johnson et al., 1994, Ann. Rev. Biochem., 63: 869-914) and the attachment of palmitic acid to cysteine residues (Milligan et al., 1995, Trends Biochem. Sci., 20: 181-186).

Fatty acylation of proteins is a dynamic post-translational modification which is critical for the biological activity of many proteins, as well as their interactions with other proteins and with membranes. Thus, for a large number of proteins, the location of the protein within a cell can be controlled by its state of prenylation (fatty acid modification) as can its ability to interact with effector enzymes (ras and MAP kinase, Itoh et al., 1993, J. Biol. Chem., 268: 3025-; ras and adenylate cyclase (in yeast; Horiuchi et al., 1992, Mol. Cell. Biol., 12: 4515-) or with regulatory proteins (Shirataki et al., 1991, J. Biol. Chem., 266: 20672-20677). The prenylation status of ras is important for its oncogenic properties (Cox, 1995, Methods Enzymol., 250: 105-121) thus interference with the prenylation status of ras is considered a valuable anti-cancer strategy (Hancock, 1993, Curr. Biol.. 3: 770).

Sentrinization

Sentrin is a novel 101-amino acid protein which has 18 % identity and 48% similarity with human ubiquitin (Okura et al., 1996, J. Immunol., 157: 4277-4281 ). This protein is known by a number of other names including SUMO-1, UBL1, PIC1, GMP1 and SMT3C and is one of a number of ubiquitin-like proteins that have recently been identified. Sentrin is expressed in all tissues (as shown by Northern blot analysis), but mRNA levels are higher in the heart, skeletal muscle, testis, ovary and thymus.

The sentrinization of proteins is thought to involve the Ubiquitin-conjugating enzyme Ubc9 (Gong et al., 1997. J. Biol. Chem., 272: 28198-28201). The interaction between these two proteins in the yeast two-hybrid screen is very specific, suggesting that this is a biologically relevant phenomenon. The interaction is dependent upon the presence of the conserved.C-terminal Gly-Gly residues present in sentrin (Gong et al., 1997, supra). The conjugation of sentrin to other proteins *via* $Gly_{97}$ requires the cleavage of the C-terminal four amino acids of the protein, His-Ser-Thr-Val.

One important protein shown to be modified by the addition of sentrin is the Ran-specific GTPase-activating protein, RanGAPI, which is involved in nuclear import of proteins bearing nuclear-localization signals (Johnson and Hochstrasser, 1997, Trends Cell Biol., 7: 408-413). Conjugation of RanGAP1 and sentrin is essential both for the targeting of RanGAP 1 to its binding partner on the nuclear pore complex (NPC) and for the nuclear import of proteins. Sentrin itself does not bind with high affinity to the NPC and it is, therefore, likely that it either provokes a conformational change in RanGAP1 that exposes a binding site or, alternatively, that the binding site is formed using both sentrin and RanGAP1 sequences. There is evidence to suggest that the conjugation of sentrin to RanGAP1 is necessary for the formation of other sentrinized proteins (Kamitani et al., 1997, J. Biol. Chem., 272: 14001-14004) and that the majority of these sentrinized proteins are found in the nucleus.

Sentrin has been shown in yeast two-hybrid screens to interact with a number of other proteins, including the death domains of Fas/APO1 and the TNF receptors, PML, RAD51 and RAD52 (Johnson and Hochstrasser, 1997, supra). These interactions implicate sentrin in a number of important processes. Fas/APO1 and TNF receptors are involved in transducing the apoptosis signal *via* their death domains. Ligation of Fas on the cell surface results in the formation of a complex *via* death domains and death-effector domains, triggering the induction of apoptosis. The overexpression of sentrin protects cells from both anti-Fas/APO and TNF-induced cell death (Okura et al., 1996, supra). It is not clear whether this protection is achieved simply by preventing the binding of other proteins to these death domains or whether a more complex process is involved, possibly one involving the ubiquitin pathway.

The interaction of sentrin with PML (a RING finger protein) is important, as it points to a disease state in which this protein may play a role. In normal myeloid cells, PML is found in a nuclear multiprotein complex known as a nuclear body. These nuclear bodies are disrupted in acute promyelocytic leukaemia, where a chromosomal translocation generates a fusion between regions of the retinoic acid receptor and PML. This disruption can be reversed by treatment with retinoic acid. It has been shown that PML is covalently modified at multiple sites by members of the sentrin family of proteins (but not by ubiquitin or NEDD8). Two forms of the aberrant fusion protein have been identified, neither of which is modified by sentrin. It is, therefore, thought that differential sentrinization of the normal and aberrant forms of PML may be important in the processes underlying acute promyelocytic leukaemia and may help in the understanding of the biological role of the PML protein (Kamitani et al., 1998, J. Biol. Chem., 273: 3117-3120).

Methods for detection of protein modification in real time

i. *In vitro* protein modification and detection thereof

Modifying enzymes

The invention requires the presence of a modifying enzyme which catalyzes either the addition or removal of a modifying group. A range of kinases, phosphatases and other modifying enzymes are available commercially (e.g. from Sigma, St. Louis, MO; Promega, Madison, WI; Boehringer Mannheim Biochemicals, Indianapolis, IN; New England Biolabs, Beverly, MA; and others). Alternatively, such enzymes may be prepared in the laboratory by methods well known in the art.

The catalytic sub-unit of protein kinase A (c-PKA) can be purified from natural sources (e.g. bovine heart) or from cells/organisms engineered to heterologously express the enzyme. Other isoforms of this enzyme may be obtained by these procedures. Purification is performed as previously described from bovine heart (Peters et al., 1977, Biochemistry, 16: 5691-5697) or from a heterologous source (Tsien et al., WO92/00388), and is in each case briefly summarized as follows:

Bovine ventricular cardiac muscle (2 kg) is homogenized and then centrifuged. The supernatant is applied to a strong anion exchange resin (e.g. Q resin, Bio-Rad) equilibrated in a buffer containing 50 mM Tris-HCl, 10 mM NaCl, 4 mM EDTA pH 7.6 and 0.2 mM 2-mercaptoethanol. The protein is eluted from the resin in a second buffer containing 50 mM Tris-HCl, 4 mM EDTA pH 7.6, 0.2 mM 2-mercaptoethanol, 0.5 M NaCl. Fractions containing PKA are pooled and ammonium sulphate added to 30% saturation. Proteins precipitated by this are removed by centrifugation and the ammonium sulphate concentration of the supernatant was increased to 75% saturation. Insoluble proteins are collected by centrifugation (included c-PKA) and are dissolved in 30 mM phosphate buffer pH 7.0, 1 mM EDTA, 0.2 mM 2-mercaptoethanol. These proteins are then dialysed against the same buffer (500 volume excess) at 4 C for two periods of 8 hours each. The pH of the sample is reduced to 6.1 by addition of phosphoric acid, and the sample is mixed sequentially with 5 batches of CM-Sepharose (Pharmacia, ~80 ml resin each) equilibrated in 30 mM phosphate pH 6.1. l mM EDTA, 0.2 mM 2-mercaptoethanol. Cyclic AMP (10 M) is added to the material which fails to bind to the CM-Sepharose, and the sample-cAMP mix is incubated with a fresh resin of CM-Sepharose (~100 ml) equilibrated as before. c-PKA is eluted from this column following extensive washing in equilibration buffer by addition of 30 mM phosphate pH 6.1, 1 mM EDTA, 1M KCl, 0.2 mM 2-mercaptoethanol. Fractions containing c-PKA are pooled and concentrated by filtration through a PM-30 membrane (or similar). The c-PKA sample is then subjected to gel-filtration chromatography on a resin such as Sephacryl 200HR (Pharmacia).

The purification of recombinant c-PKA is as described in WO 92/00388. General methods of preparing pure and partially-purified recombinant proteins, as well as crude cellular extracts comprising such proteins, are well known in the art. Molecular methods useful in the production of recombinant proteins, whether such proteins are the enzymes to be assayed according to the invention or the labeled reporter polypeptides of the invention (i.e., the natural binding domain, sequence or polypeptide and its binding partner), are well known in the art (for methods of cloning, expression of cloned genes and protein purification, see Sambrook et al., 1989, Molecular Cloning. A Laboratory Manual., 2nd Edition, Cold Spring Harbor Laboratory Press, Cold Spring Harbor, NY; Ausubel et al., Current Protocols in Molecular Biology, copyright 1987-1994, Current Protocols, copyright 1994-1998, John Wiley & Sons, Inc.). The sequences of the catalytic subunit of several PKA molecules are found in the Genbank database (see PKA C, bovine, Genbank Accession Nos. X67154 and S49260; PKA C 1, bovine, Genbank Accession No. J02647; PKA C 2, bovine, M60482, the form most likely purified from bovine heart by the protocol described above).

According to the invention, assays of the activity of protein-modifying enzymes may be performed using crude cellular extracts, whether to test the activity of a recombinant protein or one which is found in nature, such as in a biological sample obtained from a test cell line or animal or from a clinical patient. In the former case, use of a crude cell extract enables rapid screening of many samples, which potentially finds special application in high-throughput screening methods, e.g. of candidate modulators of protein-modifying enzyme activity. In the latter case, use of a crude extract with the labeled reporter polypeptide comprising a natural binding domain, sequence or polypeptide of the invention facilitates easy and rapid assessment of the activity of an enzyme of interest in a diagnostic procedure, e.g.; one which is directed at determining whether a protein- modifying enzyme is active at an a physiologically-appropriate level, or in a procedure designed to assess the efficacy of a therapy aimed at modulating the activity of a particular enzyme.

Production of a natural binding domain sequence or polypeptide

Polypeptides comprising or consisting of a natural binding domain, sequence or polypeptide or a binding partner thereof may be synthesized by Fmoc or Tboc chemistry according to methods known in the art (e.g., see Atherton et al., 1981, . Chem. Soc. Perkin I, 1981(2): 538-546; Merrifield, 1963, J.Am. Chem.Soc., 85: 2149-2154, respectively). Following deprotection and cleavage from the resin, peptides are desalted by gel filtration chromatography and analysed by mass spectroscopy, HPLC, Edman degradation and/or other methods as are known in the art for protein sequencing using standard methodologies.

Alternatively, nucleic acid sequences encoding such peptides may be expressed either in cells or in an *in vitro* transcription/translation system (see below) and, as with enzymes to be assayed according to the invention, the proteins purified by methods well known in the art.

Labelling of polypeptides with fluorophores

Polypeptides comprising or consisting of natural binding domains, sequences or polypeptides or a binding partner therefor are labeled with thiol reactive derivatives of fluorescein and tetramethylrhodamine (isothiocyanate or iodoacetamide derivatives, Molecular Probes, Eugene, OR, USA) or other fluorophores as are known in the art using procedures described by Hermanson G.T., 1995, Bioconjugate Techniques, Academic Press, London. Alternatively, primary-amine-directed conjugation reactions can be used to label lysine sidechains or the free peptide N-terminus (Hermason, 1995, supra).

Purification of fluorescent natural binding domains and/or binding partners therefor

Fluorescent peptides are separated from unreacted fluorophores by gel filtration chromatography or reverse phase HPLC.

Phosphorylation of natural binding domains and, optionally, binding partners therefor *in vitro*

Natural binding domains and, optionally, binding partners therefor (0.01-100.0 $\mu$M) are phosphorylated by purified c-PKA in 50 mM Histidine buffer pH 7.0, 5 mM $MgSO_4$, 1 mM EGTA, 0.1-10.0 $\mu$M c-PKA, and 0.2 mM [32P]-ATP (specific activity ~2Bq/pmol) at 15-40 C for periods of time ranging from 0 to 60 minutes. Where the chemistry of the peptide is appropriate *(i.e.* having a basic charge) the phosphopeptide is captured on a cation exchange filter paper (e.g. phosphocellulose P81 paper; Whatman), and reactants are removed by extensive washing in 1 % phosphoric acid (see Casnellie, 1991, Methods Enzymol., 200: 115-120). Alternatively, phosphorylation of samples is terminated by the addition of SDS-sample buffer (Laetnmli, 1970, Nature, 227: 680-685) and the samples analysed by SDS-PAGE electrophoresis, autoradiography and scintillation counting of gel pieces.

Dephosphorylation of a natural binding domain or binding partner therefor *in vitro*

The dephosphorylation of natural binding domains and, optionally, binding partners therefor, phosphorylated as above is studied by removal of ATP (through the addition of 10 mM glucose and 30 U/ml hexokinase; Sigma, St. Louis, MO) and addition of protein phosphatase-1 (Sigma). Dephosphorylation is followed at 15-40°C by quantitation of the remaining phosphopeptide component at various time points, determined as above.

Fluorescence measurements of protein modification *in vitro* in real time

Donor and acceptor fluorophore-labeled polypeptides comprising or consisting of natural binding domains, sequences or polypeptides (molar equivalents of fluorophore-labeled polypeptide or molar excess of acceptor-labeled polypeptide) are first mixed (if the natural binding domains, sequence or polypeptide and its binding partner are present on separate polypeptides). Samples are analyzed in a fluorimeter using excitation wavelengths relevant to the donor fluorescent label and emission wavelengths relevant to both the donor and acceptor labels. A ratio of emission from the acceptor over that from the donor following excitation at a single wavelength is used to determine the efficiency of fluorescence energy transfer between fluorophores, and hence their spatial proximity. Typically, measurements are performed at 0-37°C as a function of time following the addition of the modifying enzyme (and, optionally, a modulator or candidate modulator of function for that enzyme, as described below) to the system in 50 mM histidine pH 7.0, 120 mM KCI, 5 mM $MgSO_4$, 5 mM NaF, 0.05 mM EGTA and 0.2 mM ATP. The assay may be performed at a higher temperature if that temperature is compatible with the enzyme(s) under study.

Alternative cell-free assay system

A cell-free assay system is required to permit binding of an unmodified, labeled natural binding domain, sequence or polypeptide and its binding partner to occur. As indicated herein, such a system may comprise a low-ionic-strength buffer (e.g., physiological salt, such as simple saline or phosphate- and/or Tris-buffered saline or other as described above), a cell culture medium, of which many are known in the art, or a whole or fractionated cell lysate. The components of an assay of post-translational modification of a polypeptide molecule may be added into a buffer, medium or lysate or may have been expressed in cells from which a lysate is derived. Alternatively, a cell-free transcription-and/or translation system may be used to deliver one or more of these components to the assay system. Nucleic acids of use in cell-free expression systems are as described for *in vivo* assays, below.

Such an assay may be performed in a standard in vitro transcription/translation system under conditions which permit expression of a recombinant or other gene. The TNT® T7 Quick Coupled Transcription/Translation System (Cat. # L1170; Promega) contains all reagents necessary for *in vitro* transcription/translation except the DNA of interest and the detection label; as discussed below, polypeptides comprising natural binding domains, sequences or polypeptides or their binding partners may be encoded by expression constructs in which their coding sequences are fused in-frame to those encoding fluorescent proteins. The TNT® Coupled Reticulocyte Lysate Systems (comprising a rabbit reticulocyte lysate) include: TNT® T3 Coupled Reticulocyte Lysate System (Cat. # L4950; Promega); TNT® T7 Coupled Reticulocyte Lysate System (Cat. # L4610; Promega); TNT® SP6 Coupled Reticulocyte Lysate System (Cat. # L4600; Promega); TNT® T7/SP6 Coupled Reticulocyte Lysate System (Cat. # L5020; Promega); TNT® T7/T3 Coupled Reticulocyte Lysate System (Cat. # L5010; Promega).

An assay involving a cell lysate or a whole cell (see below) may be performed in a cell lysate or whole cell preferably eukaryotic in nature (such as yeast, fungi, insect, e.g., *Drosophila),* mouse, or human). An assay in which a cell lysate is used is performed in a standard *in vitro* system under conditions which permit gene expression. A rabbit reticulocyte lysate alone is also available from Promega, either nuclease-treated (Cat. # L4960) or untreated (Cat. # L4151).

In a preferred *in vitro* embodiment, at least one of the binding domain or binding partner is immobilised to a solid support. Suitable supports include beads, "chips", resins, matrices, gels, and the material forming the walls of a vessel. Matrices, and in particular gels, such as agarose gels, may conveniently be packed into columns. A particular advantage of solid phase immobilisation is that the reagents may be removed from contact with the polypeptide(s) with facility.

Sets of reporter polypeptides may be immobilised to solid substrates to produce peptide arrays. The reporter polypeptides are typically immobilised onto or in discrete regions of a solid substrate. The substrate may be porous to allow immobilisation within the substrate or substantially non-porous, in which case the sensing elements are typically immobilised on the surface of the substrate. The solid substrate may be made of any material to which polypeptides can bind, either directly or indirectly. Examples of suitable solid substrates include flat glass, silicon wafers, mica, ceramics and organic polymers such as plastics, including polystyrene and polymethacrylate. It may also be possible to use semi-permeable membranes such as nitrocellulose or nylon membranes, which are widely available. The semi-permeable membranes may be mounted on a more robust solid surface such as glass. The surfaces may optionally be coated with a layer of metal, such as gold, platinum or other transition metal. A particular example of a suitable solid substrate is the commercially available BIACoire™ chip (Pharmacia Biosensors). A further example is a microtitre plate or similar configuration.

Candidate modulators of protein-modifying enzymes to be screened

Whether *in vitro* or in an *in vivo* system (see below), we describe methods by which to screen compositions which may enhance, inhibit or not affect (e.g., in a cross-screening procedure in which the goal is to determine whether an agent intended for one purpose additionally affects general cellular functions, of which protein modification is an example) the activity of a protein-modifying enzyme.

Candidate modulator compounds from large libraries of synthetic or natural compounds can be screened. Numerous means are currently used for random and directed synthesis of saccharide, peptide, and nucleic acid based compounds. Synthetic compound libraries are commercially available from a number of companies including Maybridge Chemical Co. (Trevillet, Cornwall, UK), Comgenex (Princeton, NJ), Brandon Associates (Merrimack, NH), and Microsource (New Milford, CT). A rare chemical library is available from Aldrich (Milwaukee, WI). Combinatorial libraries are available and can be prepared. Alternatively, libraries of natural compounds in the form of bacterial, fungal, plant and animal extracts are available from e.g., Pan Laboratories (Bothell, WA) or MycoSearch (NC), or are readily produceable by methods well known in the art.

Additionally, natural and synthetically produced libraries and compounds are readily modified through conventional chemical, physical, and biochemical means.

Useful compounds may be found within numerous chemical classes, though typically they are organic compounds, including small organic compounds. Small organic compounds have a molecular weight of more than 50 yet less than about 2,500 daltons, preferably less than about 750, more preferably less than about 350 daltons. Exemplary classes include heterocycles, peptides, saccharides, steroids, and the like. The compounds may be modified to enhance efficacy, stability, pharmaceutical compatibility, and the like. - Structural identification of an agent may be used to identify, generate, or screen additional agents. For example, where peptide agents are identified, they may be modified in a variety of ways to enhance their stability, such as using an unnatural amino acid, such as a D-amino acid, particularly D-alanine, by functionalizing the amino or carboxylic terminus, e.g. for the amino group, acylation or alkylation, and for the carboxyl group, esterification or amidification, or the like.

Candidate modulators which may be screened include receptors, enzymes, ligands, regulatory factors, and structural proteins. Candidate modulators also include nuclear proteins, cytoplasmic proteins, mitochondrial proteins, secreted proteins, plasmalemma-associated proteins, serum proteins, viral antigens, bacterial antigens, protozoal antigens and parasitic antigens. Candidate modulators additionally comprise proteins, lipoproteins, glycoproteins, phosphoproteins and nucleic acids (e.g., RNAs such as ribozymes or antisense nucleic acids). Proteins or polypeptides which can be screened include hormones, growth factors, neurotransmitters, enzymes, clotting factors, apolipoproteins, receptors, drugs, oncogenes, tumor antigens, tumor suppressors, structural proteins, viral antigens, parasitic antigens, bacterial antigens and antibodies (see below).

Candidate modulators which may be screened also include substances for which a test cell or organism might be deficient or that might be clinically effective in higher-than-normal concentration as well as those that are designed to eliminate the translation of unwanted proteins. Nucleic acids of use not only may encode the candidate modulators described above, but may eliminate or encode products which eliminate deleterious proteins. Such nucleic acid sequences are antisense RNA and ribozymes, as well as DNA expression constructs that encode them. Note that antisense RNA molecules, ribozymes or genes encoding them may be administered to a test cell or organism by a method of nucleic acid delivery that is known in the art, as described below. Inactivating nucleic acid sequences may encode a ribozyme or antisense RNA specific for the a target mRNA. Ribozymes of the hammerhead class are the smallest known, and lend themselves both to *in vitro* production and delivery to cells (summarized by Sullivan, 1994, J. Invest. Dermatol., 103: 85S-98S; Usman et al., 1996, Curr. Opin. Struct. Biol., 6: 527-533).

As stated above, antibodies are of use as modulators (specifically, as inhibitors) of protein-modifying enzymes. Methods for the preparation of antibodies are well known in the art, and are briefly summarized as follows:

Either recombinant proteins or those derived from natural sources can be used to generate antibodies using standard techniques, well known to those in the field. For example, the proteins are administered to challenge a mammal such as a monkey, goat, rabbit or mouse. The resulting antibodies can be collected as polyclonal sera, or antibody-producing cells from the challenged animal can be immortalized (e.g. by fusion with an immortalizing fusion partner) to produce monoclonal antibodies.

1. Polyclonal antibodies.

The antigen protein may be conjugated to a conventional carrier in order to increases its immunogenicity, and an antiserum to the peptide-carrier conjugate is raised. Coupling of a peptide to a carrier protein and immunizations may be performed as described (Dymecki et al., 1992, J. Biol. Chem., 267: 4815-4823). The serum is titered against protein antigen by ELISA (below) or alternatively by dot or spot blotting (Boersma and Van Leeuwen, 1994, J. Neurosci. Methods, 51: 317). At the same time, the antiserum may be used in tissue sections prepared as described below. The serum is shown to react strongly with the appropriate peptides by ELISA, for example, following the procedures of Green et al., 1982, Cell, 28: 477-487.

2. Monoclonal antibodies.

Techniques for preparing monoclonal antibodies are well known, and monoclonal antibodies may be prepared using a candidate antigen whose level is to be measured or which is to be either inactivated or affinity-purified, preferably bound to a carrier, as described by Arnheiter et al., Nature, 294, 278-280 (1981).

Monoclonal antibodies are typically obtained from hybridoma tissue cultures or from ascites fluid obtained from animals into which the hybridoma tissue is introduced. Nevertheless, monoclonal antibodies may be described as being "raised to" or "induced by" a protein.

Monoclonal antibody-producing hybridomas (or polyclonal sera) can be screened for antibody binding to the target protein. By antibody, we include constructions using the binding (variable) region of such an antibody, and other antibody modifications. Thus, an antibody useful in the invention may comprise a whole antibody, an antibody fragment, a polyfunctional antibody aggregate, or in general a substance comprising one or more specific binding sites from an antibody. The antibody fragment may be a fragment such as an Fv, Fab or $F(ab')_2$ fragment or a derivative thereof, such as a single chain Fv fragment. The antibody or antibody fragment may be non-recombinant, recombinant or humanized. The antibody may be of an immunoglobulin isotype, e.g., IgG, IgM, and so forth. In addition, an aggregate, polymer, derivative and conjugate of an immunoglobulin or a fragment thereof can be used where appropriate.

Determination of activity of candidate modulator of a protein-modifying enzyme

A candidate modulator of the activity of a protein-modifying enzyme may be assayed as described herein, is determined to be effective if its use results in a difference of about 10% or greater relative to controls in which it is not present (see below) in FRET or other signal emanating from a detectable label of use in the invention resulting from the association of a natural binding domain, sequence or polypeptide and its binding partner in the presence of a protein-modifying enzyme.

The level of activity of a candidate modulator may be quantified using any acceptable limits, for example, via the following formula:

$$\text{Percent Modulation} = \frac{(\text{Index}_{Control} - \text{Index}_{Sample})}{(\text{Index}_{Control})} \times 100$$

where Index$_{Control}$ is the quantitative result (e.g., amount of- or rate of change in fluorescence at a given frequency, rate of molecular rotation, FRET, rate of change in FRET or other index of modification, including, but not limited to, enzyme inhibition or activation) obtained in assays that lack the candidate modulator (in other words, untreated controls), and Index$_{Sample}$ represents the result of the same measurement in assays containing the candidate modulator. As described below, control measurements are made with differentially labeled natural binding domains, sequences or polypeptides and their binding partners only, and then with these molecules plus a protein-modifying enzyme which recognizes a natural site for post-translational protein modification present on the natural binding domain and, optionally, on the binding partner.

Such a calculation is used in either *in vitro* or *in vivo* assays.

ii. *In vivo* assays of enzymatic activity

Reporter group protein modification in living cells

Differentially-labeled natural binding domains, sequences or polypeptides and their corresponding binding partners of the invention are delivered (e.g., by microinjection) to cells, such as smooth muscle cells (DDT1) or ventricular cardiac myocytes as previously described (Riabowol et al., 1988, Cold Spring Harbor Symposia on Quantitative Biology, 53: 85-90). The ratio of emission from the labeled molecule(s) is measured as described above *via* a photomultiplier tube focused on a single cell. As described elsewhere herein, an ADP ribosylating enzyme may be stimulated with cholera toxin (G-protein recognition feature) or with brefeldin A.

Activation of a kinase (e.g., PKA by the addition of dibutyryl cAMP or -adrenergic agonists) is performed, subsequent inhibition is performed by removal of stimulus and by addition of a suitable antagonist (e.g., cAMP antagonist Rp-cAMPS).

Heterologous expression of peptides

Natural binding domains, sequences or polypeptides and/or their binding partners can be produced from the heterologous expression of DNA sequences that encode them or by chemical synthesis of the same. Expression can be in procaryotic or eukaryotic cells using a variety of plasmid vectors capable of instructing hetemlogous expression. Purification of these products is achieved by destruction of the cells (e.g. French Press) and chromatographic purification of the products. This latter procedure can be simplified by the inclusion of an affinity purification tag at one extreme of the peptide, separated from the peptide by a protease cleavage site if necessary.

The use of cells or whole organisms

When performed using cells, the assays described here are broadly applicable to a host cell susceptible to transfection or transformation including, but not limited to, bacteria (both gram-positive and gram-negative), cultured- or explanted plant (including, but not limited to, tobacco, arabidopsis, carnation, rice and lentil cells or protoplasts), insect (e.g., cultured *Drosophila* or moth cell lines) or vertebrate cells (e.g., mammalian cells) and yeast.

Organisms are currently being developed for the expression of agents including DNA, RNA, proteins, non-proteinaceous compounds, and viruses. Such vector microorganisms include bacteria such as *Clostridium* (Parker et al., 1947, Proc. Soc. Exp. Biol. Med., 66: 461-465; Fox et al., 1996, Gene Therapy, 3: 173-178; Minton et al., 1995, FEMS Microbiol. Rev.. 17: 357-364), *Salmonella* (Pawelek et al., 1997, Cancer Res., 57: 4537-4544; Saltzman et al., 1996, Cancer Biother. Radiopharm., 11: 145-153; Carrier et al., 1992, J. Immunol., 148: 1176-1181; Su et al., 1992, Microbiol. Pathol., 13: 465-476; Chabalgoity et al., 1996, Infect. Immunol., 65: 2402-2412), *Listeria* (Schafer et al., 1992, J. Immunol., 149: 53-59; Pan et al., 1995, Nature Med., 1: 471-477) and *Shigella* (Sizemore et al., 1995, Science, 270: 299-302), as well as yeast, mycobacteria, slime molds (members of the taxa Dictyosteliida - such as of the genera *Polysphondylium* and *Dictystelium, e.g. Dictyostelium discoideum* - and Myxomycetes - e.g. of the genera *Physarum* and *Didymium)* and members of the Domain Arachaea - (including, -but not limited to, archaebacteria), which have begun to be used in recombinant nucleic acid work, members of the phylum Protista, or other cell of the algae, fungi, or any cell of the animal or plant kingdoms.

Plant cells useful in expressing polypeptides of use include, but are not limited to, tobacco *(Nicotiana plumbaginifolia* and *Nicotiana tabacum),* arabidopsis *(Arabidopsis thaliana), Aspergillus niger, Brassica napus, Brassica nigra, Datura innoxia, Vicia narbonensis, Viciafaba,* pea *(Pisum sativum),* cauliflower, carnation and lentil *(Lens culinaris).* Either whole plants, cells or protoplasts may be transfected with a nucleic acid of choice. Methods for plant cell transfection or stable transformation include inoculation with *Agrobacterium tumefaciens* cells carrying the construct of interest (see, among others, Turpen et al., 1993, J. Virol. Methods. 42: 227-239), administration of liposome-associated nucleic acid molecules (Maccarrone et al., 1992, Biochem. Biophys. Res. Commun., 186: 1417-1422) and microparticle injection (Johnston and Tang, 1993, Genet. Eng. (NY), 15: 225-236), among other methods. A generally useful plant transcriptional control element is the cauliflower mosaic virus (CaMV) 35S promoter (see, for example, Saalbach et al., 1994, Mol. Gen. Genet., 242: 226-236). Non-limiting examples of nucleic acid vectors useful in plants include pGSGLUC1 (Saalbach et al., 1994, supra), pGA492 (Perez et al., 1989, Plant Mol. Biol., 13: 365-373), pOCA18 (Olszewski et al., 1988, Nucleic Acids Res., 16: 10765-10782), the Ti plasmid (Roussell et al., 1988, Mol. Gen. Genet., 211: 202-209) and pKR612B1 (Balazs et al., 1985, Gene, 40: 343-348).

Mammalian cells are also of use. Such cells include, but are not limited to, neuronal cells (those of both primary explants and of established cell culture lines) cells of the immune system (such as T-cells, B-cells and macrophages), fibroblasts, hematopoietic cells and dendritic cells. Using established technologies, stem cells (e.g. hematopoietic stem cells) may be used for gene transfer after enrichment procedures. Alternatively, unseparated hematopoietic cells and stem cell populations may be made susceptible to DNA uptake. Transfection of hematopoietic stem cells is described in Mannion-Henderson et al., 1995, Exp. Hematol., 23: 1628; Schiffmann et al., 1995, Blood, 86: 1218; Williams, 1990, Bone Marrow Transplant. 5: 141; Boggs, 1990, Int. J. Cell Cloning, 8: 80; Martensson et al., 1987, Eur. J. Immunol. 17: 1499; Okabe et al., 1992, Eur. J. Immunol., 22: 37-43; and Banerji et al., 1983, Cell. 33: 729. Such methods may advantageously be used..

Nucleic acid vectors for the expression of assay comnonents of the invention in cells or multicellular organisms

A nucleic acid of use according to the methods of the invention may be either double- or single stranded and either naked or associated with protein, carbohydrate, proteoglycan and/or lipid or other molecules. Such vectors may contain modified and/or unmodified nucleotides or ribonucleotides. In the event that the gene to be transfected may be without its native transcriptional regulatory sequences, the vector must provide such sequences to the gene, so that it can be expressed once inside the target cell. Such sequences may direct transcription in a tissue-specific manner, thereby limiting expression of the gene to its target cell population, even if it is taken up by other surrounding cells. Alternatively, such sequences may be general regulators of transcription, such as those that regulate housekeeping genes, which will allow for expression of the transfected gene in more than one cell type; this assumes that the majority of vector molecules will associate preferentially with the cells of the tissue into which they were injected, and that leakage of the vector into other cell types will not be significantly deleterious to the recipient organism. It is also possible to design a vector that will express the gene of choice in the target cells at a specific time, by using an inducible promoter, which will not direct transcription unless a specific stimulus, such as heat shock, is applied.

A gene encoding a component of the assay system or a candidate modulator of protein-modifying enzyme activity may be transfected into a cell or organism using a viral or non-viral DNA or RNA vector, where non-viral vectors include, but are not limited to, plasmids, linear nucleic acid molecules, artificial chromomosomes and episomal vectors. Expression of heterologous genes in mammals has been observed after injection of plasmid DNA into muscle (Wolff J. A. et al., 1990, Science, 247: 1465-1468; Carson D. A. et at, US Patent No. 5,580,859), thyroid (Sykes-et al., 1994, Human Gene Ther., 5: 837-844), melanoma (Vile et al., 1993, Cancer Res.. 53: 962-967), skin (Hengge et al., I995, Nature Genet., 10: 161-166), liver (Hickman et al., 1994, Human Gene Therapy, 5: 1477-1483) and after exposure of airway epithelium (Meyer et al., 1995, Gene Therapy. 2: 450-460).

In addition to vectors of the broad classes described above and fusion gene expression construct encoding a natural binding domain, sequence or polypeptide fused in-frame to a fluorescent protein, as described above (see "Fluorescent resonance energy transfer"), microbial plasmids, such as those of bacteria and yeast, are of use.

Bacterial plasmids:

Of the frequently used origins of replication, pBR322 is useful, and pUC is preferred. Although not preferred, other plasmids which are useful are those which require the presence of plasmid encoded proteins for replication, for example, those comprising pT181, FII, and FI origins of replication.

Examples of origins of replication which are useful in assays of the invention in *E. coli* and *S. typhimurium* include but are not limited to, pHETK (Garapin et al., 1981, Proc. Natl. Acad. Sci. U.S.A.. 78: 815-819), p279 (Talinadge et al., 1980, Proc. Natl. Acad. Sci. U.S.A. 77: 3369-3373), p5-3 and p21A-2 (both from Pawalek et al., 1997, Cancer Res., 57: 4537-4544), pMB1 (Bolivar et al., 1977, Gene, 2: 95-113), ColE1 (Kahn et al., 1979, Methods Enzymol., 68:

268-280), p15A (Chang et al., 1978, <u>J. Bacteriol.,</u> 134: 1141-1156); pSC101 (Stoker et al., 1982, <u>Gene,</u> 18: 3;5-341); R6K (Kahn et al., 1979. supra); RI (temperature dependent origin of replication, Uhlin et al., 1983, <u>Gene,</u> 22: 255-265); lambda dv (Jackson et al., 1972, <u>Proc. Nat. Aca. Sci. U.S.A.,</u> 69: 2904-2909); pYA (Nakayama et al., 1988, infra). An example of an origin of replication that is useful in Staphylococcus is pT181 (Scott, 1984, <u>Microbial Reviews</u> 48: 1-23). Of the above-described origins of replication, pMB1, pl5A and ColEI are preferred because these origins do not require plasmid-encoded proteins for replication.

Yeast plasmids:

Three systems are used for recombinant plasmid expression and replication in yeasts:

1. Integrating. An example of such a plasmid is YIp, which is maintained at one copy *per* haploid genome, and is inherited in Mendelian fashion. Such a plasmid, containing a gene of interest, a bacterial origin of replication and a selectable gene (typically an antibiotic-resistance marker), is produced in bacteria. The purified vector is linearized within the selectable gene and used to transform competent yeast cells. Regardless of the type of plasmid used, yeast cells are typically transformed by chemical methods (e.g. as described by Rose et al., 1990, <u>Methods in Yeast Genetics,</u> Cold Spring Harbor Laboratory Press, Cold Spring Harbor, NY). The cells are treated with lithium acetate to achieve transformation efficiencies of approximately $10^4$ colony-forming units (transformed cells)/$\mu$g of DNA. Yeast perform homologous recombination such that the cut, selectable marker recombines with the mutated (usually a point mutation or a small deletion) host gene to restore function. Transformed cells are then isolated on selective media.

2. Low copy-number ARS-CEN, of which YCp is an example. Such a plasmid contains the autonomous replicating sequence (ARS1), a sequence of approximately 700 bp which, when carried on a plasmid, permits its replication in yeast, and a centromeric sequence (CEN4), the latter of which allows mitotic stability. These are usually present at 1-2 copies *per* cell. Removal of the CEN sequence yields a YRp plasmid, which is typically present in 100-200 copes *per* cell; however, this plasmid is both mitotically and meiotically unstable.

3. High-copy-number 2$\mu$ circles. These plasmids contain a sequence approximately I kb in length, the 2$\mu$ sequence, which acts as a yeast replicon giving rise to higher plasmid copy number; however, these plasmids are unstable and require selection for maintenance. Copy number is increased by having on the plasmid a selection gene operatively linked to a crippled promoter. This is usually the LEU2 gene with a truncated promoter (LEU2-d), such that low levels of the Leu2p protein are produced; therefore, selection on a leucine-depleted medium forces an increase in copy number in order to make an amount of Leu2p sufficient for cell growth.

As suggested above, examples of yeast plasmids useful in the invention include the YRp plasmids (based on autonomously-replicating sequences, or ARS) and the YEp plasmids (based on the 2$\mu$ circle), of which examples are YEp24 and the YEplac series of plasmids (Gietz and Sugino, 1988, <u>Gene,</u> 74: 527-534). (See Sikorski, "Extrachromosomal cloning vectors of Saccharomyces cerevisiae", in <u>Plasmids, A Practical Approach,</u> Ed. K.G. Hardy, IRL Press, 1993; and <u>Yeast Cloning Vectors and Genes, Current Protocols in Molecular Biology,</u> Section II, Unit 13.4, Eds., Ausubel et al., 1994).

In addition to a yeast origin of replication, yeast plasmid sequences typically comprise an antibiotic resistance gene, a bacterial origin of replication (for propagation in bacterial cells) and a yeast nutritional gene for maintenance in yeast cells. The nutritional gene (or "auxotrophic marker") is most often one of the following (with the gene product listed in parentheses and the sizes quoted encompassing the coding sequence, together with the promoter and terminator elements required for correct expression):

*TRPI 1* (PhosphoADP-ribosylanthranilate isomerase, which is a component of the tryptophan biosynthetic pathway).

*URA3* (Orotidine-5'-phosphate decarboxylase, which takes part in the uracil biosynthetic pathway).

*LEU2* (3-Isopropylmalate dehydrogenase, which is involved with the leucine biosynthetic pathway).

*HIS3* (Imidazoleglycerolphosphate dehydratase, or IGP dehydratase).

*LYS2* (-aminoadipate-semialdehyde dehydrogenase, part of the lysine biosynthetic pathway).

Alternatively, the screening system may operate in an intact, living multicellular organism, such as an insect or a mammal. Methods of generating transgenic *Drosophila,* mice and other organisms, both transiently and stably, are well known in the art; detection of fluorescence resulting from the expression of Green Fluorescent Protein in live *Drosophila* is well known in the art. One or more gene expression constructs encoding one or more of a labeled natural binding domain, sequence or polypeptide, a binding partner, a protein-modifying enzyme and, optionally, a candidate modulator thereof are introduced into the test organism by methods well known in the art (see also below). Sufficient time is allowed to pass after administration of the nucleic acid molecule to allow for gene expression, for binding of a natural binding domain, sequence or polypeptide to its binding partner and for chromophore maturation, if necessary (e.g., Green Fluorescent Protein matures over a period of approximately 2 hours prior to fluorescence) before fluorescence or other emission from a detectable label is measured. A reaction component (particularly a candidate modulator of enzyme function) which is not administered as a nucleic acid molecule may be delivered by a method selected from those described below.

Dosage and administration of a labeled natural binding domain, sequence or polypeptide, binding partner therefor, protein-modifying enzyme or candidate modulator thereof for use in an *in vivo* assay

Dosage

For example, the amount of each labeled natural binding domain or binding partner therefor must fall within the detection limits of the fluorescence-measuring device employed. The amount of an enzyme or candidate modulator thereof will typically be in the range of about 1 μg - 100 mg/kg body weight. Where the candidate modulator is a peptide or polypeptide, it is typically administered in the range of about 100 - 500 μg/ml per dose. A single dose of a candidate modulator, or multiple doses of such a substance, daily, weekly, or intermittently, is contemplated.

A candidate modulator is tested in a concentration range that depends upon the molecular weight of the molecule and the type of assay. For example, for inhibition of protein/protein or protein/DNA complex formation or transcription initiation (depending upon the level at which the candidate modulator is thought or intended to modulate the activity of a protein modifying enzyme according to the invention), small molecules (as defined above) may be tested in a concentration range of lpg - 100 μg/ml, preferably at about 100 pg - 10 ng/ml; large molecules, e.g., peptides, may be tested in the range of 10 ng - 100 μg/ml, preferably 100 ng - 10 μg/ml.

Administration

Generally, nucleic acid molecules are administered in a manner compatible with the dosage formulation, and in such amount as will be effective. In the case of a recombinant nucleic acid encoding a natural binding domain and/or binding partner therefor, such an amount should be sufficient to result in production of a detectable amount of the labeled protein or peptide, as discussed above. In the case of a protein modifying enzyme, the amount produced by expression of a nucleic acid molecule should be sufficient to ensure that at least 10% of natural binding domains or binding partners therefor will undergo modification if they comprise a target site recognized by the enzyme being assayed. Lastly, the amount of a nucleic acid encoding a candidate modulator of a protein modifying enzyme of the invention must be sufficient to ensure production of an amount of the candidate modulator which can, if effective, produce a change of at least 10% in the effect of the target protein modifying enzyme on FRET or other label emission resulting from binding of a natural binding domain to its binding partner or, if administered to a patient, an amount which is prophylactically and/or therapeutically effective.

When the end product (e.g. an antisense RNA molecule or ribozyme) is administered directly, the dosage to be administered is directly proportional to the amount needed *per* cell and the number of cells to be transfected, with a correction factor for the efficiency of uptake of the molecules. In cases in which a gene must be expressed from the nucleic acid molecules, the strength of the associated transcriptional regulatory sequences also must be considered in calculating the number of nucleic acid molecules per target cell that will result in adequate levels of the encoded product. Suitable dosage ranges are on the order of, where a gene expression construct is administered, 0.5- to 1μg, or 1- 10μg, or optionally 10- 100 μg of nucleic acid in a single dose. It is conceivable that dosages of up to 1 mg may be advantageously used. Note that the number of molar equivalents per cell vary with the size of the construct, and that absolute amounts of DNA used should be adjusted accordingly to ensure adequate gene copy number when large constructs are injected.

If no effect (e.g., of a protein modifying enzyme or an inhibitor thereof) is seen within four orders of magnitude in either direction of the starting dosage, it is likely that a protein modifying enzyme does not recognize the target site of the natural binding domain (and, optionally, its binding partner) according to the invention, or that the candidate modulator thereof is not of use according to the invention. It is critical to note that when high dosages are used, the concentration must be kept below harmful levels, which may be known if an enzyme or candidate modulator is a drug that is approved for clinical use. Such a dosage should be one (or, preferably, two or more) orders of magnitude below the $LD_{50}$ value that is known for a laboratory mammal, and preferably below concentrations that are documented as producing serious, if non-lethal, side effects.

Components of screening assays of the invention may be formulated in a physiologically acceptable diluent such as water, phosphate buffered saline, or saline, and further may include an adjuvant. Adjuvants such as incomplete Freund's adjuvant, aluminum phosphate, aluminum hydroxide, or alum are materials well known in the art. Administration of labeled polypeptides comprising a natural binding domain, sequence, polypeptide or a binding partner therefor, a protein modifying enzyme or a candidate modulator as described herein may be either localized or systemic.

Localized administration:

Localized administration of a component of an assay of the invention is preferably by *via* injection or by means of a drip device, drug pump or drug-saturated solid matrix from which the labeled natural binding domain, sequence or polypeptide, binding partner therefor, modifying enzyme or candidate modulator therefor, or nucleic acid encoding any of these can diffuse implanted at the target site. When a tissue that is the target of delivery according to the invention is on a surface of an organism, topical administration of a pharmaceutical composition is possible.

Compositions comprising a composition of- or of use in the invention which are suitable for topical administration can take one of several physical forms, as summarized below:

(i) A liquid, such as a tincture or lotion, which may be applied by pouring, dropping or "painting" (i.e. spreading manually or with a brush or other applicator such as a spatula) or injection.

(ii) An ointment or cream, which may be spread either manually or with a brush or other applicator (e.g. a spatula), or may be extruded through a nozzle or other small opening from a container such as a collapsible tube.

(iii) A dry powder, which may be shaken or sifted onto the target tissue or, alternatively, applied as a nebulized spray.

(iv) A liquid-based aerosol, which may be dispensed from a container selected from the group that comprises pressure-driven spray bottles (such as are activated by squeezing), natural atomizers (or "pump-spray" bottles that work without a compressed propellant) or pressurized canisters.

(v) A carbowax or glycerin preparation, such as a suppository, which may be used for rectal or vaginal administration of a therapeutic composition.

In a specialized instance, the tissue to which a candidate modulator of a protein modifying enzyme is to be delivered for assay (or, if found effective, for therapeutic use) is the lung. In such a case the route of administration is *via* inhalation, either of a liquid aerosol or of a nebulized powder of. Drug delivery by inhalation, whether for topical or systemic distribution, is well known in the art for the treatment of asthma, bronchitis and anaphylaxis. In particular, it has been demonstrated that it is possible to deliver a protein *via* aerosol inhalation such that it retains its native activity *in vivo* (see Hubbard et al., 1989, J. Clin. Invest., 84: 1349-1354).

Systemic administration:

Systemic administration of a protein, nucleic acid or other agent according to the invention may be performed by methods of whole-body drug delivery are well known in the art. These include, but are not limited to, intravenous drip or injection, subcutaneous, intramuscular, intraperitoneal, intracranial and spinal injection, ingestion *via* the oral route, inhalation, trans-epithelial diffusion (such as *via* a drug-impregnated, adhesive patch) or by the use of an implantable, time-release drug delivery device, which may comprise a reservoir of exogenously-produced protein, nucleic acid or other material or may, instead, comprise cells that produce and secrete a natural binding domain and/or a binding partner therefor, protein modifying enzyme or candidate modulator thereof. Note that injection may be performed either by conventional means (i.e. using a hypodermic needle) or by hypospray (see Clarke and Woodland, 1975, Rheumatol. Rehabil., 14: 47-49). Components of assays of the invention can be given in a single- or multiple dose.

Delivery of a nucleic acid may be performed using a delivery technique selected from the group that includes, but is not limited to, the use of viral vectors and non-viral vectors, such as episomal vectors, artificial chromosomes, liposomes, cationic peptides, tissue-specific cell transfection and transplantation, administration of genes in general vectors with tissue-specific promoters, etc.

Kits

A kit for assaying the activity of a protein-modifying enzyme

In order to facilitate convenient and widespread use of the methods described here, a kit is described which contains the essential components for screening the activity of a an enzyme which mediates a change in protein modification, as described above. A natural binding domain, sequence or polypeptide, as defined above, and its corresponding binding partner are provided, as is a suitable reaction buffer for *in vitro* assay or, alternatively, cells or a cell lysate. A reaction buffer which is "suitable" is one which is permissive of the activity of the enzyme to be assayed and which permits modification dependent binding of the natural binding domain to the binding partner. The labeled polypeptide components are provided as peptide/protein or a nucleic acid comprising a gene expression construct encoding the one or more of a peptide/protein, as discussed above. Natural binding domains, sequences and polypeptides, as well as their corresponding binding partners, are supplied in a kit either in solution (preferably refrigerated or frozen) in a buffer which inhibits degradation and maintains biological activity, or are provided in dried form, i.e., lyophilized. In the latter case, the components are resuspended prior to use in the reaction buffer or other biocompatible solution (e.g. water, containing one or more of physiological salts, a weak buffer, such as phosphate or Tris, and a stabilizing substance such as glycerol, sucrose or polyethylene glycol); in the latter case, the resuspension buffer should not inhibit modification-dependent protein binding when added to the reaction buffer in an amount necessary to deliver sufficient protein for an assay reaction. Natural binding domains, sequences or polypeptides or their binding partners provided as nucleic acids are supplied- or resuspended in a buffer which permits either transfection/transformation into a cell or organism or *in vitro* transcription/translation, as described above. Each of these components is supplied separately contained or in admixture with one or more of the others in a container selected from the group that includes, but is not limited to, a tube, vial, syringe or bottle.

Optionally, the kit includes cells. Eukaryotic or prokaryotic cells, as described above, are supplied in- or on a liquid or solid physiological buffer or culture medium (e.g. in suspension, in a stab culture or on a culture plate, e.g. a Petri dish). For ease of shipping, the cells are typically refrigerated, frozen or lyophilized in a bottle, tube or vial. Methods of cell preservation are widely known in the art; suitable buffers and media are widely known in the art, and are obtained from commercial suppliers (e.g., Gibco/LifeTechnologies) or made by standard methods (see, for example Sambrook et al., 1989, Molecular Cloning. A Laboratory Manual., 2nd Edition, Cold Spring Harbor Laboratory Press, Cold Spring Harbor, NY).

An enzyme being assayed according to the invention is added to the assay system either as a protein (isolated, partially-purified or present in a crude preparation such as a cell extract or even a living cell) or a recombinant nucleic acid. Methods of expressing a nucleic acid comprising an enzyme or other protein are well known in the art (see again above).

An assay described here may be carried out using the kit according to the methods described above and in the Examples.

A kit for screening a candidate modulator of protein-modifying enzyme activity

A candidate modulator of post-translational modification may be assayed using a kit. A kit as described above is used for this application, with the assay performed further comprising the addition of a candidate modulator of the modifying enzyme which is present to the reaction system. Optionally, a protein-modifying enzyme is supplied with the kit, either as a protein or nucleic acid as described above.

Assays of protein activity are performed as described above. At a minimum, three detections are performed, one in which the natural binding domain and binding partner are present without the modifying enzyme or candidate modulator thereof (control reaction A), one in which the polypeptides are incubated with the modifying enzyme under conditions which permit the modification reaction to occur (control reaction B) and one in which the modifying enzyme and candidate modulator are both incubated with the labeled polypeptides under conditions which permit the modification reaction to occur (test reaction). In each case, conditions are suitable to permit modification-dependent association of the natural binding domain, sequence or polypeptide and the binding partner. The result of the last detection procedure is compared with those of the two controls; the candidate modulator is judged to be efficacious if there is a shift in either of the observed amount of signal (i.e., total amount- or rate of change of fluorescence, FRET, mass of a protein complex or inhibition or activation of an enzyme) of at least 10% away from that observed in control reaction B toward that observed in control reaction A.

The present invention will now be described with reference to the following examples that are intended to be illustrative only and non-limiting.

Description of the figures

Figure 1.    FRET between ZAP-GFP and rhodamine labelled TCRζ derived peptide.
                TCR peptide added after 9.5 minutes, reversal of FRET initiated by addition of YOP after 25 minutes.
Figure 2.    Dependence of YOP activity on different concentrations of TCRζ peptide.
Figure 3.    Measurement of inhibition of YOP by sodium orthovanadate using FRET assay. Calculated $IC_{50} = 0.25 \mu M$.
Figure 4     Fluorescence polarisation assay to detect Chk 1 phosphorylation of fluorescein-labelled Chktide binding to 14-3-3 ζ.
Figure 5     Inhibition of Chk1 phosphorylation of Chktide by EDTA.
Figure 6     Assay of Chk1 activity in real time monitoring the fluorescence polarisation of fluorescein-labelled Chktide substrate binding to 14-3-3ζ protein.
Figure 7     Chk1 activity measured by Chktide-14-3-3 ζ binding is dependent on ATP and the presence of 14-3-3ζ protein.
Figure 8     Inhibition of Chk1 phosphorylation of fluorescein labelled Chktide by EDTA.
Figure 9     Assay of Chk1 phosphorylation of Chktide by 14-3-3s binding.
Figure 10    Measurement of phosphatase λ activity by dephosphorylation of fluorescein labelled Chktide and decrease in binding to 14-3-3ζ.
Figure 11    Measurement of phosphatase λ activity by dephosphorylation of fluorescein labelled Chktide and decrease in binding to 14-3-3ε.
Figure 12    Detection of TCRζ peptide phosphorylation by Src kinase, using FRET assay between ZAP-GFP and rhodamine labelled TCRζ peptide.
Figure 13    Detection of SHPS-1 derived peptide phosphorylation by Src, via binding to SHP2-GFP partner.
Figure 14    Reversal of FRET between SHP2-GFP and rhodamine labelled, phosphorylated SBPS-1 peptide by YOP.
Figure 15    Detection of Src inhibition by staurosporine using a FRET assay between rhodamine labelled SHPS-1 and SHP2-GFP.
Figure 16    Assay of Src phosphorylation of SHPS-1 peptide in real time using FRET.
Figure 17    diagrams double- and single-chain enzymatic assay formats of the invention.
Figure 18    presents a schematic overview of FRET in an assay of the invention.
Figure 19    presents monomer:excimer fluorescence.

EXAMPLES

**Example 1- Use of a polypeptide comprising a natural binding domain as a phosphorylation reporter**

Assay 1

An assay of this type involves the following components:

v-Src SH2 domain (amino acids 148-246; Waksman et al., 1993, Cell. 72: 779-790; OWL database accession no. M33292), and
Hamster polyomavirus middle T antigen (Ag, below) (321-331. EPQYEEIPIYL: Waksman et al., 1993, supra; OWL database accession no. P03079).

SH2 domains are found in proteins involved in a number of signalling pathways and their binding to specific phosphorylated tyrosine residues is key in mediating the transmission of signals between tyrosine kinases and the proteins in the cell which respond to tyrosine phosphorylation (Waksman et al., 1993, supra and references therein). Individual SH2 domains recognize specific sequences, and the sequence specificity of a number of SH2 domains has been determined (Songyang et al., 1993, supra) using a phosphopeptide library. These data provide a number of possible domain/peptide pairs which are useful in assays of enzymatic activity according to the invention. The crystal structure of the Src SH2 domain complexed with a peptide containing its specific recognition motif from the hamster middle-T antigen (target tyrosine for phosphorylation shown in bold above) has been determined by Waksman et. al. (Cell 72, 779-790).

Thus, the assay is:

$$SH2\text{-}F1 + Ag\text{-}F2 \rightarrow (SH2\text{-}F1)(PAg\text{-}F2)$$

No FRET        FRET

F1 is the donor fluorophore, F2 the acceptor fluorophore and P denotes the addition of a phosphate group to the target tyrosine residue.

The peptide as used in the crystallization described above does not contain suitable residues for convenient labelling, and a label within this short sequence is too close to the phosphorylation site. A short linker (e.g., Gly-Gly) is, therefore, added to either the C- or N-terminus of the peptide with a residue such as Lys for labelling on the end. The location of this linker will depend upon the location of F 1 in the SH2 domain.

A number of potential locations for the fluorophore in the SH2 domain have been identified based upon crystal structure:

| SH2 Domain | Middle T Ag. peptide |
|---|---|
| K 232 | C-terminal extension (G-G-K or similar) |
| R 217 | C-terminal extension |
| K 181 | N-terminal extension |
| R 156** | N-terminal extension |
| **this site is close to the site of peptide interaction | |

If a fluorescent protein (e.g., Green Fluorescent Protein, GFP) is used instead of a chemical fluorophore, it is placed at the N-termini of both the SH2 domain and the peptide

**Example 2 - Use of a polypeptide comprising a natural binding domain as a phosphorylation reporter**

Assay 2

This assay involves the following components:

PTB domain of IRS-1 (amino acids 157-267) (Zhou et al., 1996, Nature Structural Biology, 3: 388-393; OWL accession no. P35568), and

Interleukin 4 Receptor (IL-4R) (amino acids 489-499, LVIAGNPAYRS; Zhou et.

al., 1996, supra; OWL accession no. P24394)

Phosphotyrosine binding (PTB) domains are found in a number of proteins involved in signalling pathways utilizing tyrosine phosphorylation. The PTB domain has functional similarities to the SH2 domain but differs in its mechanism of action and structure, as well as in sequence recognition (Laminet et al., 1996, J. Biol. Chem., 271: 264-269; Zhou et. al., 1996, supra and references therein). These two classes of domain have little sequence identity. NMR structural analysis of the PTB domain of lltS-1 complexed with the IL-4 receptor peptide has been performed (Zhou et al., 1996, supra).

The assay format is as follows:

$$PTB\text{-}F1 + IL\text{-}4R\text{-}F2 \rightarrow (PTB\text{-}F1)(PIL\text{-}4R\text{-}F2)$$

No FRET        FRET

F1 is the donor fluorophore, F2 the acceptor fluorophore, and P denotes the addition of a phosphate group to the target tyrosine residue.

The peptide of the NMR study described above does not contain suitable residues for convenient labelling except the arginine next to the phosphorylation site, and a label within this short sequence may be too close to the target site for phosphorylation. A short linker may be added to either the C- or N-terminus of the peptide with a residue for labelling on the end. The location of such a linker depends upon the location of F1 in the PTB domain.

Several potential locations for the fluorophore in the PTB sequence have been identified from the NMR structure:

| PTB domain | IL-4R peptide |
|---|---|
| K161 | N-terminal extension (G-G-K or similar) |
| K190 | N-terminal extension |
| N-terminal extension | N-terminal extension |
| C-terminal extension | C-terminal extension |

Again, if GFP is used in lieu of a chemical fluorophore, it can be fused in-frame to either the N- or C-terminus of both the PTB sequence and the binding partner.

**Example 3**

An assay analogous to that in Example 2 can be configured according to the invention using the PTB domain of the proto-oncogene product Cbl and a peptide derived from the Zap-70 tyrosine kinase. The Cbl phosphotyrosine-binding domain selects a D(N/D)XpY motif and binds to the $Tyr_{292}$ negative regulatory phosphorylation site of ZAP-70 (Lupher et al., 1997, J. Biol. Chem., 272: 33140-33144).

The components of the assay are:

The Cbl N-terminal domain (amino acids 1-357; Lupher et al., 1996, J. Biol. Chem., 271: 24063-24068; OWL accession no. P22681), and

Zap-70 (amino acids 284-299, $NH_3$-IDTLNSDGYTPEPARI-COOH; Lupher et. al., 1996, supra; OWL accession no. P43403)

**Example 4 - Use of a polypeptide comprising a natural binding domain as a phosphorylation reporter**

Assay 4

This assay involves the following component-

c-Src (residues 86-536; Xu et al., 1997, Nature. 385: 595-602; GenBank Accession No. K03218).

As stated above, Src is a member of a family of non-receptor tyrosine kinases involved in the regulation of responses to extracellular signals. Association of src with both the plasma membrane and intracellular membranes is mediated by myristoylation at the N-terminus. The enzyme has four regions which are conserved throughout the family, the SH2 domain, the SH3 domain, the kinase or SH1 domain and the C-terminal tail. In addition there is a unique region which does not have homology between family members (Brown and Cooper, 1996, Biochim. Biophys. Acta, 1287: 121-149).

The SH2 domain binds tightly to specific tyrosine phosphorylated sequences. This affinity plays a role in the interaction between src and other cellular proteins and also in the regulation of the kinase by phosphorylation. The C-terminal tail of src can be phosphorylated on $Tyr_{530}$, which phosphorylation leads to almost complete inhibition of kinase activity. There is strong evidence that this inhibition is achieved by the interaction of the C-terminal tail with the SH2 domain. This interaction is thought to promote a conformational change to the 'closed' conformation which is further stabilized by the participation of the SH3 and kinase domains in intramolecular contacts.

The assay is diagrammed as follows:

$$\text{Src-SH2-F1--------Tail-F2} + \text{ATP} \rightarrow \text{Src-(SH2-F1)(PTail-F2)} + \text{ADP}$$

$$\text{No FRET} \qquad\qquad\qquad \text{FRET}$$

where F1 is the donor fluorophore, F2 is the acceptor fluorophore and P denotes the addition of a phosphate group to the target tyrosine residue.

There are several potential sites for labelling in this structure. Some examples of target residues are shown below:

| C-Terminal tail | SH2 domain |
| --- | --- |
| E527 | D195, K198 |
| C-Terminal extension (eg. Gly-Gly-Lys) | R220, K235, |

When a fluorescent protein is used in an assay such as this, using an intramolecular interaction to follow chemical modification, it is appropriate to place GFP between domains using a flexible linker to preserve protein domain interactions. This allows the GFP variants to approach more closely and increase the efficiency of the FRET achieved, but must be balanced by the need to achieve a good distance between variants in the 'No FRET' state. If sufficient spacing between donor and acceptor fluorophores or, alternatively, between a fluorophore or other label and a quencher therefor, is not achieved in this manner, other candidate locations for fluorescent protein fusion include, but are not limited to, the C-terminus and the region between the SH2 domain and the SH2-kinase linker.

**Example 5 - Solution FRET assay for *Yersinia* Tyrosine Phosphatase (YOP) using a natural binding partner labelled with a fluorescent protein and a synthetic peptide labelled with a chemical fluorophore.**

A FRET partnership is formed between the SH2 domain of ZAP-70 and a peptide based on the TCRζ chain, providing both partners are labelled with suitable fluorophores, which bind when the peptide is in a phosphorylated state. Formation of FRET is followed in real-time just by adding the two binding partners together. Disruption of FRET can be achieved with the use of a phosphatase, which removes the phosphate required for the interaction of the partners.

**Methods:**

TCR peptide sequence

Peptide 1. Phosphorylated TCRζ chain:
RCKFSRSAEPPAYQQGQNQLY(p)NELNLGRREEY(p)DVLD

Peptide labelling

Peptide 1 is labelled with rhodamine under mild conditions using thiol directed chemistry. 230$\mu$M peptide is labelled in 20mM TES buffer pH 7 in the presence of a three-fold excess of rhodamine-6-maleimide (Molecular Probes). Dialysis is utilised to remove excess dye from the peptide. Labelling verified by MALDI-TOF MS.

ZAP-GFP cloning and purification

DNA constructs

**ZAP-GFP**: Primers are designed based on the published ZAP-70 DNA sequence (Genbank accession number L05148). The SH2 domain (amino acids 1-259) of ZAP70 is cloned by PCR using the following oligo-nucleotides:

    Forward primer GGGATCCATATGCCAGACCCCGCGGCGCACCTG
    Reverse Primer GGAATTCGGGCACTGCTGTTGGGGCAGGCCTCC

The resultant PCR fragment is digested with *BamH*I and *EcoR*I and inserted into pET28a (Novagen) to generate vector pFS45. DNA encoding GFP in the vector pQB125-FNI (Quantum) is digested with *Mlu*I and the resultant 5' overhang is "filled in" using T4 DNA polymerase (NEB). After the polymerase is denatured by heat treatment the DNA is further digested with *EcoR*I and the resultant 850 bp band is gel purified. The vector pFS45 is digested with *Hind*III and the resultant 5' overhang is "filled in" with T4 DNA polymerase and then further digested with *EcoR*I. After the digested vector is gel purified it is ligated with the purified DNA encoding GFP to generate pFS46 which is designed to express a ZAP70-GFP fusion protein in bacteria.

Expression and purification procedure

Fresh transformants of ZAP-GFP pET-28a in BRL(DE3) are used to inoculate 3 ml LB/kanamycin (100 μg/ml). The starter cultures are incubated overnight at 37°C with constant shaking. From these starter cultures 1ml is used to inoculate 400 ml Terrific Broth/kanamycin (100 μg/ml) in a 2L, baffled flask. Cultures are incubated at 37°C at 200rpm for approximately 5hrs until the OD600nm reached 0.5Abs units. At this point cultures are induced by adding IPTG to a concentration of 1mM. The cultures are then left incubating at room temperature overnight with gentle shaking on a benchtop rotator.

Bacteria are harvested by centrifugation at 3000 rpm for 20mins. The bacterial pellet is resuspended in 25 ml lysis buffer (50 mM phosphate buffer pH 7.0, 300 mM NaCl, 2% Proteinase inhibitor cocktail (Sigma), 0.75 mg/ml Lysozyme). Lysis of the resuspended cells is initiated by gentle stirring for lhr at room temperature. The partially lysed mixture is subjected to 2 cycles of freeze thawing in liquid nitrogen. Finally the cells are sonicated on ice using a 10 mm probe at high power. Sonication is performed on a pulse setting for a period of 3 min. The crude lysate is then centrifuged at 15000 rpm for 30 mins to remove cell debris. Hexa-His tagged proteins are purified from the clear lysate using TALON® resin (Clontech). Proteins are bound to the resin in a batchwise manner by gentle shaking at room temperature for 30 min. Non-His tagged proteins are removed by washing the resin at least twice with 10x bed volume of wash buffer (50 mM sodium phosphate pH 7.0, 300 mM NaCl, 5 mM fluorescence-blank imidazole). The washed resin is loaded into a 2 ml column and the bound proteins released with elution buffer (50mM sodium phosphate pH 7.0, 300 mM NaCl, 150 mM florescence-blank Imidazole). Elution is normally achieved after the first 0.5 ml and within 2-3 ml in total. Proteins are stored at -80°C after snap freezing in liquid nitrogen in the presence of 10% glycerol.

Formation of the FRET partnership

ZAP-GFP is diluted to 0.5μM in YOP assay buffer (50mM, HCR Tris-Hcl pH7.2, 10 mM β-mercaptoethanol, 0.5mg/ml BSA, 0.015% Brij 35). 98 μl of this solution is used per assay. Initial readings of the fluorescence of the ZAP-GFP construct are made using 485 nm excitation wavelength and 520 nm emission wavelength. Rhodamine labelled peptide (2 μl of a 115 μM peptide solution) is added to the ZAP-GFP and formation of FRET followed in real-time by measuring the decrease in fluorescence emission of ZAP-GFP at 520nm.

YOP source and assay conditions

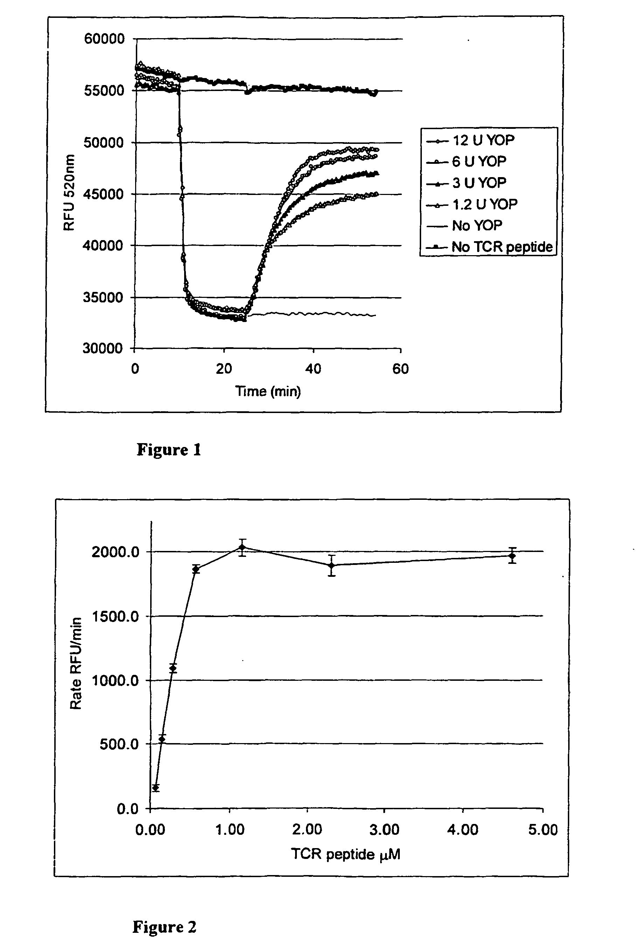

*Yersinia* protein tyrosine phosphatase (YOP) is purchased from Upstate Biotechnology. YOP, 0-12 units, is added to the ZAP-GFP/peptide FRET partnership and the increase in fluorescence emission at 520m, is followed in real-time as the partnership is disrupted (Figure 1). Dependence of YOP activity on TCR peptide concentration and Km determination can be measured using different concentrations of rhodamine labelled TCRζ peptide and 3 units of YOP (Figure 2).

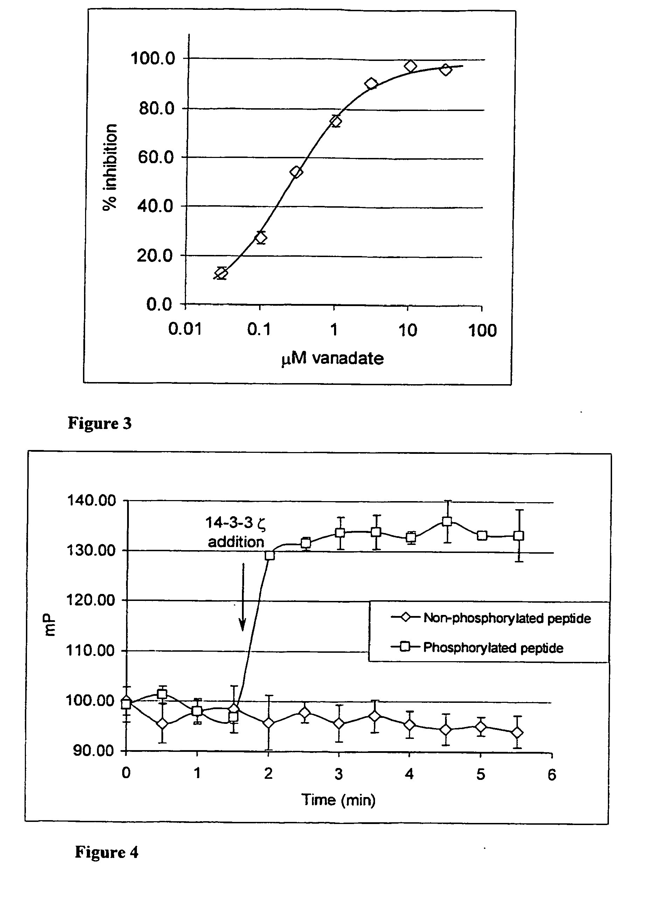

The solution FRET assay for YOP is also used to determine the $IC_{50}$ of orthovanadate, a general protein phosphatase inhibitor. The YOP assay is performed as above, using 3 units of YOP and incubating the enzyme with the FRET partnership at 30°C. Dephosphorylation is followed in real-time by measuring the increase in fluorescence at 520nm. Sodium orthovanadate is added to the reaction mix prior to enzyme addition to give final concentrations of (0.03-30 μM). Results indicate an $IC_{50}$ for vanadate of 0.25 μM and are shown in Figure 3.

**Example 6 - Assay of Chkl kinase using a solution phase FP assay with fluorescein labelled CDC25 derived peptide substrate and 14-3-3ζ binding partner.**

Chk1 protein kinase modifies the activity of CDC25 phosphatase via serine phosphorylation. Phosphorylation of CDC25 results in the binding of different isoforms of the 14-3-3 protein and subsequent inhibition of CDC25 phosphatase activity. A peptide derived from CDC25 (Chktide) and labelled with a chemical fluorophore binds to 14-3-3 isoforms zeta and epsilon when phosphorylated by Chk1 kinase. The activity of Chk1 is monitored by following the fluorescence polarisation change when fluorescein labelled Chktide binds to 14-3-3 protein.

**Methods**

Reagent sources

14-3-3 ζ protein, Chk1 enzyme and Ckhtide are from Upstate Biotechnology. Chktide , Chk1 substrate peptide sequence:
KKKVSRSGLYRSPS[216]MPENLNRPR

Chktide labelling with fluorescein

Chktide is labelled by incubating for 2 hours at a concentration of 0.185mM in 100mM NaHCO$_3$ pH 8.3, and 0.37mM fluorescein-5EX (Molecular Probes) at room temperature. The labelled peptide is then dialysed against 3 changes of 50 mM Tris-HCl pH 7.4, 150 mM NaCl (200 ml) for a total of 18 hours.

Phosphorylation of Chktide by Chk1 protein kinase.

A peptide substrate for Chk1 (Chktide) is labelled with fluorescein. The labelled peptides are then phosphorylated by incubating at a concentration of 30μM for 30 min at 30°C in 20 mM MOPS pH 7.2, 10 mM MgCl$_2$, 25 mM β-slycerophosphate, 5 mM EGTA, 1 mM sodium orthovanadate, 1 mM DTT, 0.1 mM ATP, and 62 mU Chk1. Non phosphorylated control samples are prepared by incubation of the labelled peptides under identical conditions in the absence of Chk1. The peptides are then incubated at 5 μM concentration in 20 mM MOPS pH 7.2 at 30°C, in a total volume of 30 μl on a half area 96 well plate.

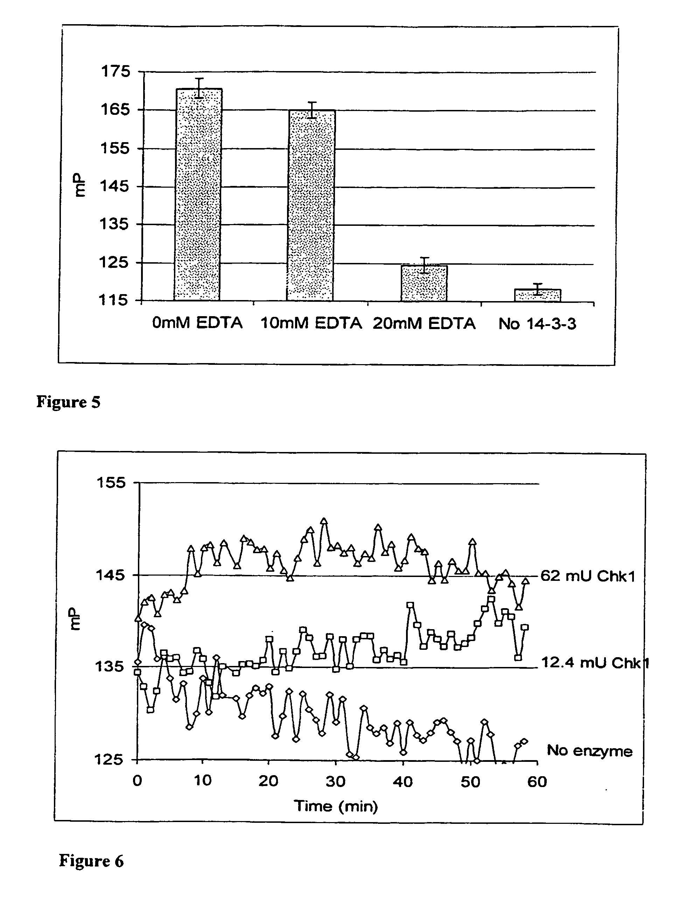

The fluorescence polarisation of the samples is measured at 520 nm (excitation 485 nm) following a 5min equilibration. 14-3-3 ζ (5 μM) is added to the peptide samples, and the fluorescence polarisation at 520nm (excitation 485nm) is monitored over time as shown in Figure 4. Inhibition of Chk1 activity by EDTA is measured by following the above procedure and adding EDTA (10mM or 20 mM) prior to enzyme addition, as shown in Figure 5.

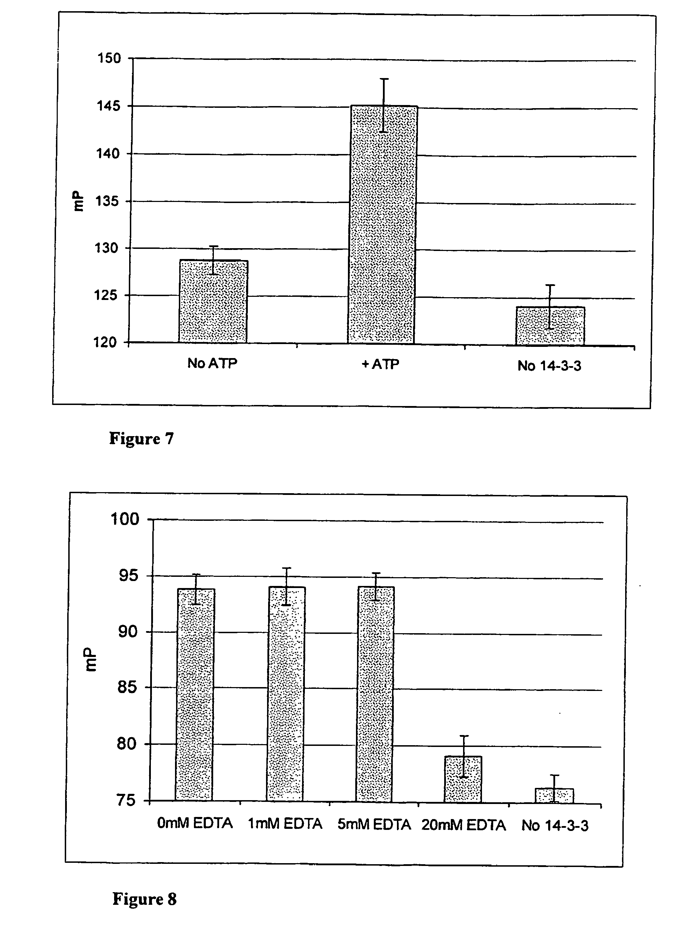

Activity of Chk1 can be followed in real time as follows. Fluorescein labelled Chktide is incubated at 30°C in a total volume of 30 μl (on a half area 96 well plate) in 20 mM MOPS pH 7.2, 10 mM MgCl$_2$, 25 mM β-glycerophosphate, 5mM EGTA, 1 mM sodium orthovanadate, 1 mM DTT, and 0.1 mM ATP in the presence of μM 14-3-3 ζ. The samples are allowed to equilibrate for 5 min, then Chk1 is added (or an equal volume of H$_2$O for control samples) and the fluorescence polarisation at 520 nm (excitation 485 nm) is monitored over time as shown in Figure 6. Dependence of the increased fluorescence polarisation signal on Chk1 activity and 14-3-3 ζ binding is shown by the lack of binding when ATP or 14-3-3ζ protein are omitted as shown in Figure 7. Inhibition of Chk1 activity by EDTA is measured by following the above procedure and adding EDTA (1 mM, 5mM or 20 mM) prior to the addition of 21 mU of Chk1 to start the reaction, as shown in Figure 8. Fluorescence polarisation for each sample is determined at the end of the linear part of the reaction.

Chk1 activity can also be measured by monitoring the binding of phosphorylated Chktide peptide to an alternate isoform of the 14-3-3 protein, 14-3-3ε.

Production of 14-3-3 ε

Under the control of the T7 promoter, the vector FS121 contains DNA encoding the 14-3-3ε (Genbank accession number U54778) protein in-frame to DNA encoding an amino terminal hexa-His tag. Fresh transformants of pFS121 in BRL(DE3) pLysS are used to inoculate 3 ml LB/ampicillin (100 μg/ml). The starter cultures is incubated overnight at 37°C with shaking. From these starter cultures 1ml is used to inoculate 400 ml Terrific Broth/ampicillin (100 μg/ml) in a 2L, baffled flask. Cultures are incubated at 37°C at 200 rpm for approximately 4 hr until the OD$_{600nm}$ has reached 0.5Abs units. At this point cultures are induced by adding IPTG to a concentration of 1mM and further incubated at 37°C for 4 hrs.

Bacteria are harvested by centrifugation at 3000 rpm for 20 min. The bacterial pellet is resuspended in 25 ml lysis buffer (50 mM Phosphate pH 7.0, 300 mM NaCl, 2% Proteinase inhibitor cocktail (Sigma), 0.75mg/ml Lysozyme). Lysis of the resuspended cells is initiated by gentle stirring for 30 min at room temperature. Nonidet P-40 is added to a final concentration of 1 % and lysis is continued for a further 20 min at room temperature. The partially lysed mixture is subjected to 3 cycles of freeze thawing in liquid nitrogen. Finally the cells are sonicated on ice using a 10 mm probe at high power. Sonication is performed on a pulse setting for a period of 4 min. The crude lysate is centrifuged at 15000 rpm for 30 min to remove cell debris. Hexa-His tagged proteins are purified from the cleared lysate using TALON® resin (Clontech). Proteins are bound to the resin in a batchwise manner by gentle shaking at room temperature for 30 min. Non-His-tagged proteins are removed by washing the resin at least twice with 10x bed volume of wash buffer (50 mM sodium phosphate pH 7.0, 300 mM NaCl, mM fluorescence-blank imidazole). The washed resin is loaded into a 2 ml column and the bound proteins are released with elution buffer (50 mM sodium phosphate pH 7.0, 300 mM NaCl, 150 mM florescence-blank imidazole). Elution is normally achieved within 5 ml. Purified proteins are stored at -80°C after snap freezing in liquid nitrogen in the presence of 10% glycerol.

End point assay of Chk1 kinase by 14-3-3ε binding to phosphorylated Chktide

A peptide substrate for Chk1 (Chktide) is labelled with fluorescein. The labelled peptides are then phosphorylated by incubating at a concentration of 30 μM for 30min at 30°C in 100 μl of 20mM MOPS pH 7.2, 10 mM MgCl$_2$, 25 mM β-glycerophosphate, 5 mM EGTA, 1 mM sodium orthovanadate, I mM DTT, 0.1 mM ATP, and 62 mU Chk1. Non-

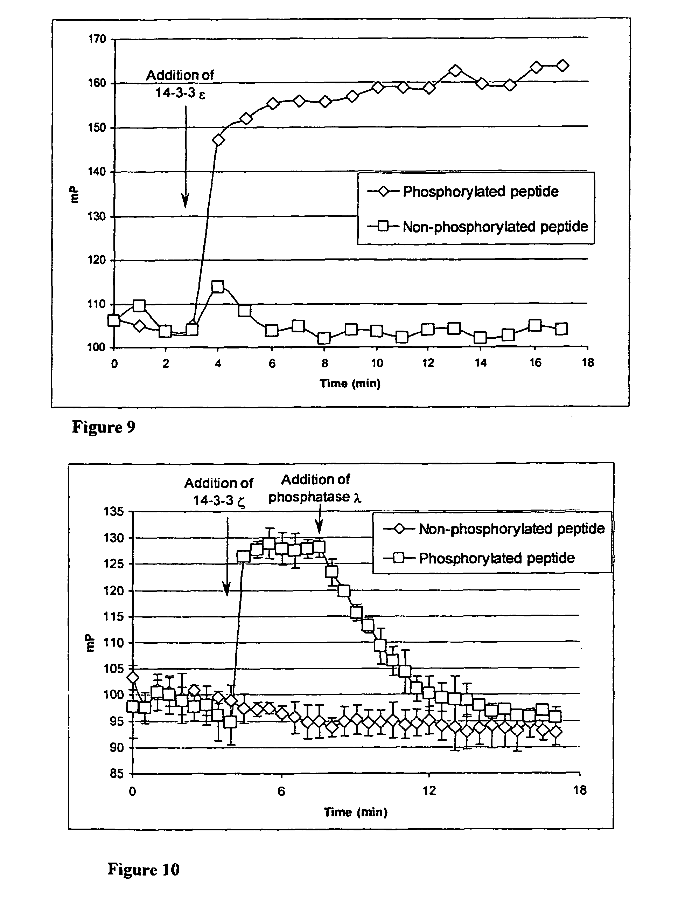

phosphorylated control samples are prepared by incubation of the labelled peptides under identical conditions in the absence of Chk1. The peptides are then incubated at 7.5 $\mu$M concentration in 20mM MOPS pH 7.2 at 30°C, in a total volume of 30 $\mu$l on a half area 96 well plate. The fluorescence polarisation of the samples is measured at 520 nm (excitation 485 nm) following a 5min equilibration. 14-3-3 $\epsilon$ (4 $\mu$l) is added to the peptide samples, and the fluorescence polarisation at 520 nm (excitation 485 nm) is monitored over time as shown in Figure 9.

**Example 7 - Solution phase FP assay for the detection of phosphatase $\lambda$ using a fluorescein labelled CDC25 derived peptide substrate and 14-3-3$\zeta$ binding partner.**

Reagents obtained and prepared as in Example 6 above.

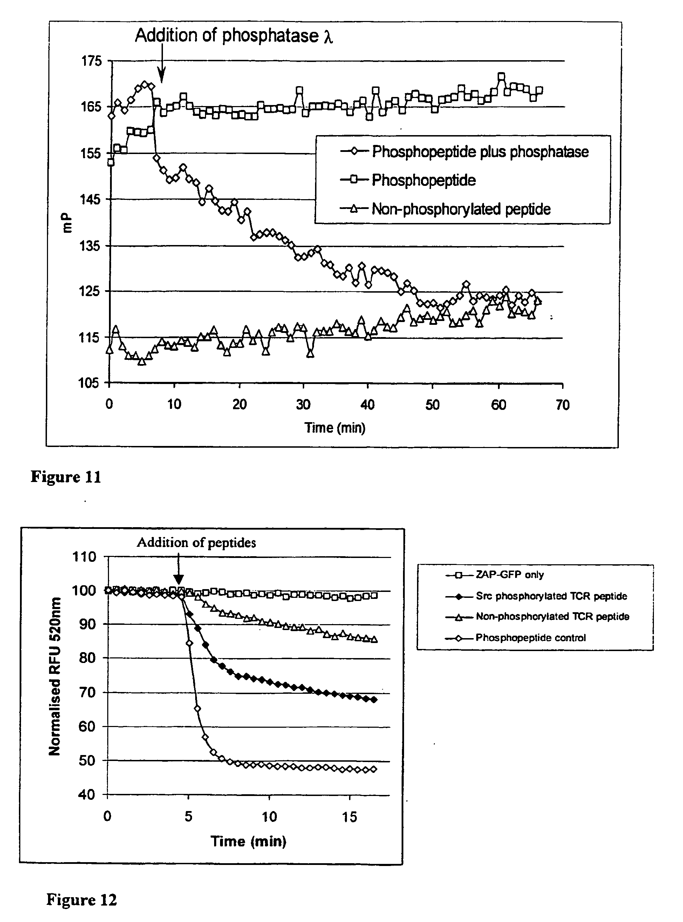

Fluorescein labelled Chktide is phosphorylated by incubating at a concentration of 30 $\mu$M for 30 min at 30°C in 20 mM MOPS pH 7.2, 10 mM MgCl$_2$, 25 mM $\beta$-glycerophosphate, 5 mM EGTA, 1 mM sodium orthovanadate, 1 mM DTT, 0.1 mM ATP, and 62mU Chk1. Non-phosphorylated control samples are prepared by incubation of the labelled peptides under identical conditions in the absence of Chk1. The peptides are then incubated at 7.5$\mu$M concentration in 20 mM MOPS pH 7.2, 2 mM MnCl$_2$, 1 mM DTT at 30°C, in a total volume of 30 $\mu$l on a half area 96 well plate. The fluorescence polarisation of the samples is measured at 520 nm (excitation 485 nm) following a 5 min equilibration. 14-3-3 $\zeta$ (5 $\mu$M) is added to the peptide samples, and the fluorescence polarisation at 520nm (excitation 485 nm) is monitored over time as shown in Figure 10. Alternatively 14-3-3 $\epsilon$ (4$\mu$l of purified protein prepared as in Example 2) is added to the peptide samples, and after the fluorescence polarisation has stabilised, phosphatase $\lambda$ (200U) is added. The fluorescence polarisation at 520 nm (excitation 485 nm) is monitored over time as shown in Figure 11.

**Example 8 - FRET assay of Src using ZAP70-GFP binding partner and synthetic rhodamine labelled TCR$\zeta$ substrate.**

A FRET partnership can be formed between the SH2 domain of ZAP-70 and a peptide based on the TCR$\zeta$ chain, providing both binding partners are labelled with suitable fluorophores, which will bind in a phosphorylation dependent manner. Src is used to phosphorylate the TCR$\zeta$ chain derived substrate.

**Methods:**

TCR peptide sequence

Peptide 1. Phosphorylated TCR$\zeta$ chain:
RCKFSRSAEPPAYQQGQNQLY$_{(p)}$NELNLGRRREEY$_{(p)}$DVLD
Peptide 2. Unphosphorylated TCR$\zeta$ chain:
RCKFSRSAEPPAYQQGQNQLYNELNLGRRREEYDVLD

Peptide labelling

Peptides 1 & 2 are labelled with rhodamine under mild conditions using thiol directed chemistry. 230 $\mu$M peptide is labelled in 20 mM TES pH 7 in the presence of a three-fold excess of rhodamine-6-maleimide (Molecular Probes). Dialysis is utilised to remove excess dye from the peptide. Labelling verified by MALDI-TOF MS.

ZAP-GFP cloning and purification

As Example 5

Phosphorylation of Peptide 2

Peptide 2 labelled with rhodamine is phosphorylated using 3 units of Src kinase (Upstate Biotechnology) in a 40 $\mu$l reaction containing 115$\mu$M peptide, 50 mM Tris-HCl pH 7.2, 1 mM ATP, 10 mM MgCl$_2$, 10 mM $\beta$-mercaptoethanol, 0.1mg/ml BSA and 0.015%(v/v) Brij 35, over a period of 2 hours at 37°C. Non-phosphorylated control samples are prepared under identical conditions in the absence of the kinase.

Formation of the FRET partnership

ZAP-GFP is diluted to 0.5$\mu$M in assay buffer (50 mM Tris-HCl pH7.2, 10 mM $\beta$-mercaptoethanol, 0.5 mg/ml BSA, 0.015% Brij 35). 98 $\mu$l of this solution is used per assay. Initial readings of the fluorescence of the ZAP-GFP construct are made using 485 nm excitation wavelength and 520nm emission wavelength. Rhodamine labelled peptide (2 μl of above phosphorylation reactions) is added to the ZAP-GFP solution and formation of FRET followed in real-time by measuring the decrease in fluorescence emission of ZAP-GFP at 520 nm as shown in Figure 12.

**Example 9 - Solution phase FRET assay of Src kinase activity using a natural binding partner, SHP-2 labelled with a fluorescent protein.**

Interaction between the tandem SH2 domain of SHP2 and a synthetic peptide based on SHPS-1 is mediated by the state of phosphorylation of the peptide. A FRET partnership can be established between phosphorylated peptide and the SH2 domain providing both partners are labelled with suitable fluorophores which are brought into close proximity upon interaction of binding partners.

**Methods:**

SHP2 peptide sequence and fluorescent labelling
SHPS-1 peptide sequence.
BiotinKQDTNDITYADLNLPKGKKPAPQAAEPNNHTEYASIQTSC

The SHPS-1 is labelled using thiol directed chemistry. 200μM peptide is reacted with 600 μM rhodamine-6-maleimide (Molecular Probes) in 20mM TES pH7 over at least two hours at room temperature. Excess label is removed using dialysis and the labelling verified by MALDI-TOF MS.

SHP2-GFP cloning and purification

Primers are based on the published SHP-2 DNA sequence (Genbank accession number L03535. The SH2 domain (amino acids 1-225) of SHP-2 is cloned by PCR using the following oligo-nucleotides:

5' primer - GGGGATCCTCTAGAATGACATCGCGGAGATGGTTTCACCC

3' primer - GGGGAATTCTTTCAGCAGCATTTATACGAGTCG

The resultant PCR fragment is digested with *Xba*I and *EcoR*I, gel purified and ligated into pET28a (Novagen) to generate vector pFS 114. The validity of the construct is confirmed by sequence analysis. DNA encoding GFP in the vector pFS46 is isolated by digestion with the restriction enzymes *EcoR*I and *Xho*I and the resultant 860bp band is gel purified and ligated into pFS 114 to generate a bacterial expression vector for production of the fusion protein SHP2-GFP (pFS115).

The hexa-His tagged SHP2-GFP fusion protein is expressed and purified using TALON® resin according to standard procedures.

Phosphorylation of peptide SHPS-1 peptide

SHPS-1 peptide labelled with rhodamine is phosphorylated using 3 units of Src kinase (Upstate Biotechnology) in a 40 μl reaction containing 100 μM peptide, 50 mM Tris-HCl pH7.2, 1 mM ATP, 10 mM MgCl$_2$, 10 mM β-mercaptoethanol, 0.1 mg/ml BSA and 0.015%(v/v) Brij 35, over a period of 2 hours at 37°C. Non-phosphorylated control samples are prepared under identical conditions in the absence of the kinase.

Formation of the FRET partnership

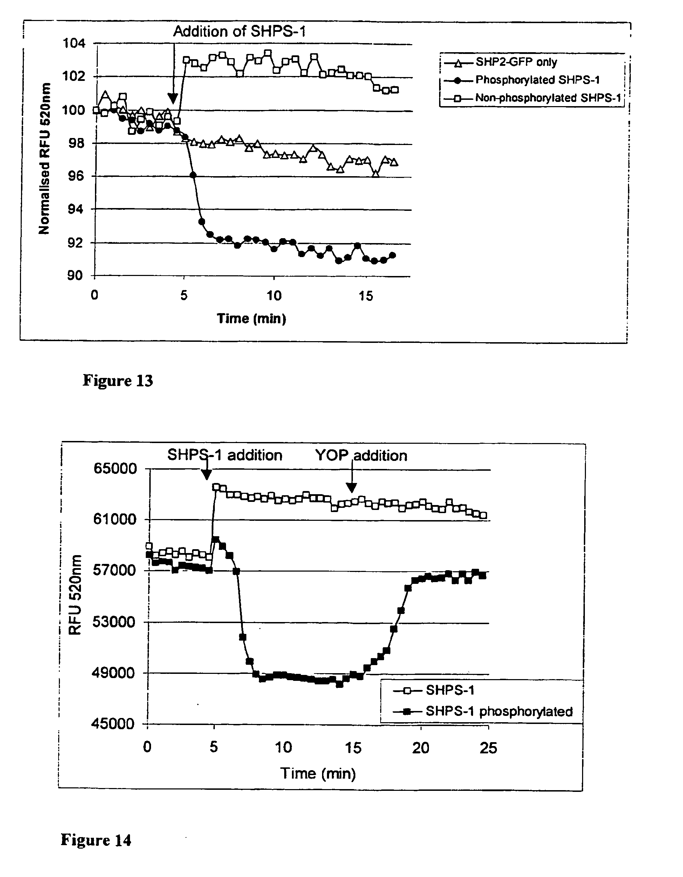

SHP2-GFP is diluted to 0.5 μM in assay buffer (50 mM Tris-HCl pH7.2, 10 mM β-mercaptoethanol, 0.5mg/ml BSA, 0.015% Brij 35). 98 μl of this solution is used per assay. Initial readings of the fluorescence of the SHP2-GFP construct are made using 485nm excitation wavelength and 520nm emission wavelength. Rhodamine labelled peptide (2μl of above phosphorylation reactions) is added to the SHP2-GFP solution and formation of FRET followed in real-time by measuring the decrease in fluorescence emission of SHP2-GFP at 520 nm as shown in Figure 13.

Disruption of the FRET partnership

The FRET partnership described in the above section is disrupted easily using the tyrosine phosphatase enzyme, YOP (Upstate Biotechnology). FRET partnership is formed as in the above example, using 4 $\mu$l of phosphorylated or control non-phosphorylated SHPS-1 peptide. Addition of 3 units of YOP to the FRET partnership results in disruption as the phosphates required for the formation of the partnership are removed as shown in Figure 14.

Inhibition by Staurosporine

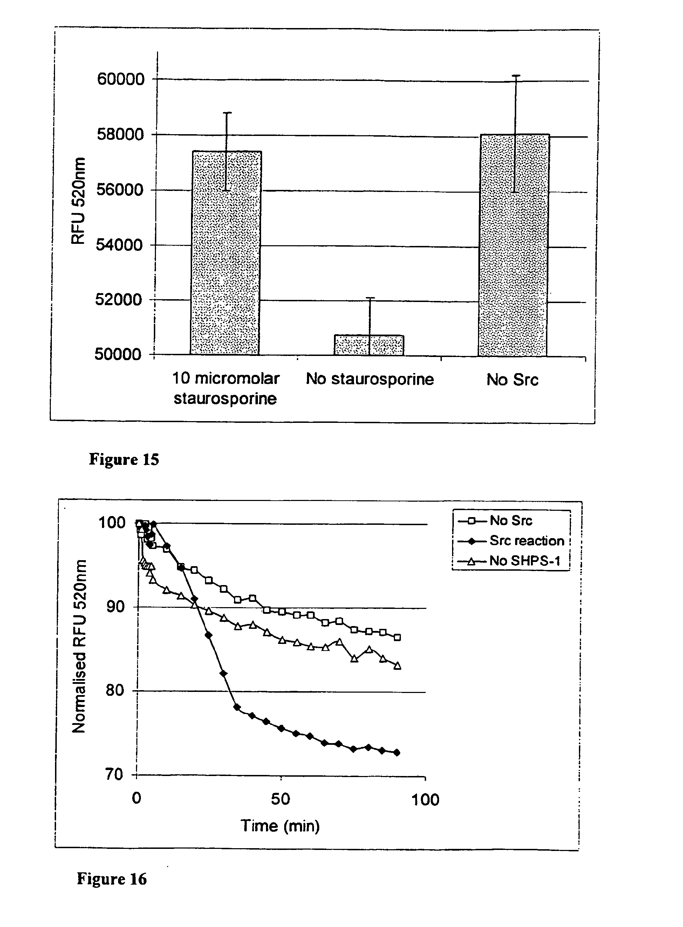

Phosphonlation of the peptide is prevented by inhibiting the enzyme, Src, with the potent kinase inhibitor staurosporine. Phosphorylation of the rhodamine labelled SHPS-1 peptide is performed as described above in the presence and absence of 10 $\mu$M staurosporine added to the reaction prior to Src enzyme. Reactions carried out in the presence of the inhibitor fail to form a FRET partnership as shown in Figure 15.

Assay of Src phosphorylation of SHPS-1 peptide in real-time

Phosphorylation of the rhodamine labelled SHPS-1 peptide and formation of the FRET partnership with SHP2-GFP can be followed in real-time by measuring the decrease in fluorescence emission at 520 nm. Reactions containing 0.5 $\mu$M SHP2-GFP, 50 mM Tris-HCl pH7.2, I mM ATP, 10 mM MgCl$_2$, 10 mM $\beta$-mercaptoethanol, 0.5 mg/ml BSA. 0.015% Brij 35 and 40 $\mu$M peptide are set up in a black microtitre plate. An initial equilibrium measurement is made before adding 6 units of Src kinase, or buffer to control wells. The decrease in fluorescence emission at 520 nm is followed in real-time at 37°C as shown in Figure 16.

USES

The invention is useful in monitoring the activity of a protein-modifying enzyme, whether the protein is isolated, partially-purified, present in a crude preparation or present in a living cell. The invention is further useful in assaying a cell or cell extract for the presence- or level of activity of a protein modifying enzyme. The invention is additionally useful in assaying the activity of naturally-occurring (mutant) or non-natural (engineered) isoforms of known protein modifying enzymes or, instead, that of novel (natural or non-natural) enzymes. The invention is of use in assaying the efficacy of candidate modulators of the activity of a protein modifying enzyme in inhibiting or enhancing the activity of that enzyme; moreover, is useful to screen potential therapeutic drugs for activity against cloned and/or purified enzymes that may have important clinical pathogenicities when mutated. The invention is further of use in the screening of candidate bioactive agents (e.g., drugs) for side effects, whereby the ability of such an agent to modulate the activity of a protein modifying enzyme may be indicative a propensity toward provoking unintended side-effects to a therapeutic or other regimen in which that agent might be employed.

**Claims**

1. A method for monitoring activity of an enzyme comprising performing a detection step to detect binding of a natural binding domain and a binding partner therefor as a result of contacting one or both of the natural binding domain and the binding partner with the enzyme, in which the natural binding domain includes a site for post-translational modification and binds the binding partner in a manner dependent upon modification of the site and in which detection of binding of the natural binding domain and the binding partner as a result of the contacting is indicative of enzyme activity, in which at least one of the natural binding domain and the binding partner is labelled with a fluorescent label.

2. A method for monitoring activity of an enzyme comprising performing a detection step to detect dissociation of a natural binding domain from a binding partner therefor as a result of contacting one or both of the natural binding domain and the binding partner with the enzyme, in which the natural binding domain includes a site for post-translational modification and binds the binding partner in a manner dependent upon modification of the site and in which detection of dissociation of the natural binding domain from the binding partner as a result of the contacting is indicative of enzyme activity, in which at least one of the natural binding domain and the binding partner is labelled with fluorescent label.

3. A method according to Claim 1 or 2, in which the enzyme is one of the following enzymes: a carbohydrate transferase, a ubiquitin activating enzyme E1, a ubiquitin conjugating enzyme E2, a ubiquitin conjugating enzyme Ubc9, a ubiquitin protein ligase E3, a poly (ADP-ribose) polymerase, a fatty acyltransferase, a kinase, a phosphatase and an NAD:

Arginine ADP ribosyltransferase.

4. A method according to Claim 1,2 or 3, which further comprises the step, prior to or after the detection step, of contacting the natural binding domain and the binding partner with an agent which modulates the activity of the enzyme.

5. A method of screening for a candidate modulator of enzymatic activity of one or more of the following enzymes: a carbohydrate transferase, a ubiquitin activating enzyme E1, a ubiquitin conjugating enzyme E2, a ubiquitin conjugating enzyme Ubc9, a ubiquitin protein ligase E3, a poly (ADP-ribose) polymerase, a fatty acyl transferase and an NAD:Arginine ADP ribosyltransferase, a kinase or a phosphatase, the method comprising

    (a) contacting a natural binding domain, a binding partner therefor and an enzyme with a candidate modulator of the enzyme, in which the natural binding domain includes a site for post-translational modification and binds the binding partner in a manner that is dependent upon modification of the site by the enzyme and in which at least one of the natural binding domain and the binding partner comprises a fluorescent label, and
    (b) monitoring the binding of the natural binding domain to the binding partner, in which binding or dissociation of the natural binding domain and the binding partner as a result of the contacting is indicative of modulation of enzymatic activity by the candidate modulator of the enzyme.

6. A method of screening for a candidate modulator of enzymatic activity of one or more of the following enzymes: a carbohydrate transferase, a ubiquitin activating enzyme E1, a ubiquitin conjugating enzyme E2, a ubiquitin conjugating enzyme Ubc9, a ubiquitin protein ligase E3, a poly (ADP-ribose) polymerase, a fatty acyl transferase and an NAD:Arginine ADP ribosyltransferase, a kinase or a phosphatase, the method comprising

    (a) contacting an assay system with a candidate modulator of enzymatic activity of a the enzyme, and
    (b) monitoring binding of a natural binding domain and a binding partner therefor in the assay system, in which the natural binding domain includes a site for post-translational modification and binds the binding partner in a manner that is dependent upon modification of the site by at least one the enzyme in the assay system,

in which at least one of the natural binding domain and the binding partner comprises a fluorescent label, and in which binding or dissociation of the natural binding domain and the binding partner as a result of the contacting is indicative of modulation of enzymatic activity by the candidate modulator of a the enzyme.

7. A method according to any preceding claim, in which the method further comprises exciting the or each fluorescent label and monitoring fluorescence emission.

8. A method according to any preceding claim, in which the detection step detects a change in signal emission by the or each fluorescent label.

9. A method according to any preceding claim, in which the method comprises real-time observation of association of the natural binding domain and its binding partner.

10. A method according to any preceding claim, in which the monitoring or detection step comprises measuring a change in energy transfer between a fluorescent label present on the natural binding domain and a fluorescent label present on the binding partner.

**Patentansprüche**

1. Verfahren zum Überwachen der Aktivität eines Enzyms, welches umfaßt, daß man eine Detektionsstufe durchführt zum Feststellen einer Bindung einer natürlichen Bindungsdomäne und eines Bindungspartners dafür als eine Folge des Inkontaktbringens von einem oder beiden der natürlichen Bindungsdomäne und des Bindungspartners mit dem Enzym, wobei die natürliche Bindungsdomäne eine Stelle für posttranslationale Modifikation enthält und den Bindungspartner in einer Art und Weise bindet, die von einer Modifikation der Stelle abhängig ist, und wobei eine Detektion von Bindung der natürlichen Bindungsdomäne und des Bindungspartners als eine Folge des Inkontaktbringens ein Hinweis für Enzymaktivität ist, wobei wenigstens eines der natürlichen Bindungsdomäne und des Bindungspartners mit einer Fluoreszenzmarkierung markiert ist.

**2.** Verfahren zur Überwachung von Aktivität eines Enzyms, welches umfaßt, daß man eine Detektionsstufe durchführt zum Feststellen von Dissoziation einer natürlichen Bindungsdomäne von einem Bindungspartner dafür als eine Folge des Inkontaktbringens von einem oder beiden der natürlichen Bindungsdomäne und des Bindungspartners mit dem Enzym, wobei die natürliche Bindungsdomäne eine Stelle für posttranslationale Modifikation umfaßt und den Bindungspartner in einer Art und Weise bindet, die von einer Modifikation der Stelle abhängig ist, und wobei eine Detektion von Dissoziation der natürlichen Bindungsdomäne von dem Bindungspartner als eine Folge des Inkontaktbringens ein Hinweis auf Enzymaktivität ist, wobei wenigstens eines von der natürlichen Bindungsdomäne und dem Bindungspartner mit einer Fluoreszenzmarkierung markiert ist.

**3.** Verfahren nach Anspruch 1 oder 2, wobei das Enzym eines der folgenden Enzyme ist: eine Carbohydrattransferase, ein Ubiquitin aktivierendes Enzym E1, ein Ubiquitin konjugierendes Enzym E2, ein Ubiquitin konjugierendes Enzym Ubc9, eine Ubiquitin-Proteinligase E3, eine Poly-(ADP-Ribose)-Polymerase, eine Fettacyltransferase, eine Kinase, eine Phosphatase und eine NAD:Arginin-ADP-Ribosyltransferase.

**4.** Verfahren nach Anspruch 1, 2 oder 3, welches weiterhin vor oder nach der Detektionsstufe die Stufe umfaßt, bei der man die natürliche Bindungsdomäne und den Bindungspartner mit einem Mittel in Kontakt bringt, welches die Aktivität des Enzyms moduliert.

**5.** Verfahren zur Durchmusterung eines Kandidaten für einen Modulator von enzymatischer Aktivität unter einem oder mehreren der folgenden Enzyme: einer Carbohydrattransferase, einem Ubiquitin aktivierenden Enzym E1, einem Ubiquitin konjugierenden Enzym E2, einem Ubiquitin konjugierenden Enzym Ubc9, einer Ubiquitin-Proteinligase E3, einer Poly-(ADP-Ribose)-Polymerase, einer Fettacyltransferase und einer NAD:Arginin-ADP-Ribosyltransferase, einer Kinase oder einer Phosphatase, wobei das Verfahren folgendes umfaßt

(a) Inkontaktbringen einer natürlichen Bindungsdomäne, eines Bindungspartners dafür und eines Enzyms mit einem Kandidaten für einen Modulator des Enzyms, wobei die natürliche Bindungsdomäne eine Stelle für posttranslationale Modifikation umfaßt und den Bindungspartner in einer Art und Weise bindet, die von einer Modifikation der Stelle durch das Enzym abhängig ist, und wobei wenigstens eines der natürlichen Bindungsdomäne und des Bindungspartners eine Fluoreszenzmarkierung enthält, und

(b) Überwachen der Bindung der natürlichen Bindungsdomäne an den Bindungspartner, wobei eine Bindung oder Dissoziation der natürlichen Bindungsdomäne und des Bindungspartners als eine Folge des Inkontaktbringens ein Hinweis für eine Modulation von enzymatischer Aktivität durch den Kandidaten für einen Modulator des Enzyms ist.

**6.** Verfahren zur Durchmusterung eines Kandidaten für einen Modulator von enzymatischer Aktivität unter einem oder mehreren der folgenden Enzyme: einer Carbohydrattransferase, einem Ubiquitin aktivierenden Enzym E1, einem Ubiquitin konjugierenden Enzym E2, einem Ubiquitin konjugierenden Enzym Ubc9, einer Ubiquitin-Proteinligase E3, einer Poly-(ADP-Ribose)-Polymerase, einer Fettacyltransferase und einer NAD:Arginin-ADP-Ribosyltransferase, einer Kinase oder einer Phosphatase, wobei das Verfahren folgendes umfaßt

(a) Inkontaktbringen eines Testsystems mit einem Kandidaten für einen Modulator von enzymatischer Aktivität des Enzyms und

(b) Überwachen von Bindung einer natürlichen Bindungsdomäne und eines Bindungspartners dafür in dem Testsystem, wobei die natürliche Bindungsdomäne eine Stelle für posttranslationale Modifikation umfaßt und den Bindungspartner in einer Art und Weise bindet, die von einer Modifikation der Stelle durch wenigstens ein Enzym in dem Testsystem abhängig ist,

wobei wenigstens eines der natürlichen Bindungsdomäne und des Bindungspartners eine Fluoreszenzmarkierung enthält und wobei eine Bindung oder Dissoziation der natürlichen Bindungsdomäne und des Bindungspartners als eine Folge des Inkontaktbringens ein Hinweis auf eine Modulation von enzymatischer Aktivität durch den Kandidaten für einen Modulator des Enzyms ist.

**7.** Verfahren nach einem der vorangegangenen Ansprüche, wobei das Verfahren weiterhin das Anregen der oder jeder Fluoreszenzmarkierung und das Überwachen von Fluoreszenzemission umfaßt.

**8.** Verfahren nach einem der vorangegangenen Ansprüche, wobei die Detektionsstufe eine Veränderung in der Signalemission durch das oder jede Fluoreszenzmarkierung feststellt.

9. Verfahren nach einem der vorangegangenen Ansprüche, wobei das Verfahren eine Echtzeitbeobachtung von einer Assoziation der natürlichen Bindungsdomäne und deren Bindungspartner umfaßt.

10. Verfahren nach einem der vorangegangenen Ansprüche, wobei die Überwachung oder die Detektionsstufe das Messen einer Veränderung in der Energieübertragung zwischen einer Fluoreszenzmarkierung, die an der natürlichen Bindungsdomäne vorhanden ist, und einer Fluoreszenzmarkierung, die an dem Bindungspartner vorhanden ist, umfaßt.

**Revendications**

1. Méthode pour contrôler l'activité d'une enzyme, comprenant la mise en oeuvre d'une étape de détection pour détecter la liaison d'un domaine de liaison naturel et d'un partenaire de liaison pour celui-ci en résultat de la mise en contact d'une ou des deux entités consistant en le domaine de liaison naturel et le partenaire de liaison avec l'enzyme, dans laquelle le domaine de liaison naturel comprend un site pour la modification en posttraduction et se lie au partenaire de liaison d'une manière dépendant de la modification du site, et dans laquelle la détection de la liaison du domaine de liaison naturel et du partenaire de liaison en résultat de la mise en contact est une indication de l'activité enzymatique, au moins une des entités consistant en le domaine de liaison naturel et le partenaire de liaison étant marquée avec un marqueur fluorescent.

2. Méthode pour contrôler l'activité d'une enzyme, comprenant la mise en oeuvre d'une étape de détection pour détecter la dissociation d'un domaine de liaison naturel d'un partenaire de liaison pour celui-ci en résultat de la mise en contact d'une des, ou des deux, entités consistant en le domaine de liaison naturel et le partenaire de liaison avec l'enzyme, dans laquelle le domaine de liaison naturel comprend un site pour la modification en posttraduction et se lie au partenaire de liaison d'une manière dépendant de la modification du site, et dans laquelle la détection de la dissociation du domaine de liaison naturel du partenaire de liaison en résultat de la mise en contact est une indication de l'activité enzymatique, au moins une des entités consistant en le domaine de liaison naturel et le partenaire de liaison étant marquée avec un marqueur fluorescent.

3. Méthode suivant la revendication 1 ou 2, dans laquelle l'enzyme est une des enzymes suivantes : une glucide-transférase, une enzyme d'activation d'ubiquitine E1, une enzyme de conjugaison d'ubiquitine E2, une enzyme de conjugaison d'ubiquitine Ubc9, une ubiquitine-protéine-ligase E3, une poly(ADP-ribose)polymérase, une acyle gras-transférase, une kinase, une phosphatase et une NAD:arginine-ADP-ribosyltransférase.

4. Méthode suivant la revendication 1, 2 ou 3, qui comprend en outre l'étape, avant ou après l'étape de détection, de mise en contact du domaine de liaison naturel et du partenaire de liaison avec un agent qui module l'activité de l'enzyme.

5. Méthode pour sélectionner un modulateur candidat d'activité enzymatique d'une ou plusieurs des enzymes suivantes : une glucide-transférase, une enzyme d'activation d'ubiquitine E1, une enzyme de conjugaison d'ubiquitine E2, une enzyme de conjugaison d'ubiquitine Ubc9, une ubiquitine-protéine-ligase E3, une poly(ADP-ribose) polymérase, une acyle gras-transférase et une NAD:arginine-ADP-ribosyltransférase, une kinase ou une phosphatase, méthode comprenant :

(a) la mise en contact d'un domaine de liaison naturel, d'un partenaire de liaison pour celui-ci et d'une enzyme avec un modulateur candidat de l'enzyme, dans laquelle le domaine de liaison naturel comprend un site pour la modification en posttraduction et se lie au partenaire de liaison d'une manière qui dépend de la modification du site par l'enzyme, et dans laquelle au moins une des entités consistant en le domaine de liaison naturel et le partenaire de liaison comprend un marqueur fluorescent, et

(b) le contrôle de la liaison du domaine de liaison naturel au partenaire de liaison, dans lequel la liaison ou la dissociation du domaine de liaison naturel et du partenaire de liaison en résultat de la mise en contact est une indication de la modulation de l'activité enzymatique par le modulateur candidat de l'enzyme.

6. Méthode pour sélectionner un modulateur candidat d'activité enzymatique d'une ou plusieurs des enzymes suivantes : une glucide-transférase, une enzyme d'activation d'ubiquitine E1, une enzyme de conjugaison d'ubiquitine E2, une enzyme de conjugaison d'ubiquitine Ubc9, une ubiquitine-protéine-ligase E3, une poly(ADP-ribose) polymérase, une acyle gras-transférase, et une NAD:arginine-ADP-ribosyltransférase, une kinase ou une phosphatase, méthode comprenant :

(a) la mise en contact d'un système analytique avec un modulateur candidat d'activité enzymatique de l'enzyme, et

(b) le contrôle de la liaison d'un domaine de liaison naturel et d'un partenaire de liaison pour celui-ci dans le système analytique, dans lequel le domaine de liaison naturel comprend un site pour la modification en posttraduction et se lie au partenaire de liaison d'une manière qui dépend de la modification du site par au moins une des enzymes dans le système analytique,

au moins une des entités consistant en le domaine de liaison naturel et le partenaire de liaison comprenant un marqueur fluorescent, et la liaison ou la dissociation du domaine de liaison naturel et du partenaire de liaison en résultat de la mise en contact étant une indication de la modulation de l'activité enzymatique par le modulateur candidat de l'enzyme.

7. Méthode suivant l'une quelconque des revendications précédentes, qui comprend en outre l'excitation du ou de chaque marqueur fluorescent et le contrôle de l'émission de fluorescence.

8. Méthode suivant l'une quelconque des revendications précédentes, dans laquelle l'étape de détection détecte une variation d'émission de signal par le ou chaque marqueur fluorescent.

9. Méthode suivant l'une quelconque des revendications précédentes, qui comprend l'observation en temps réel de l'association du domaine de liaison naturel et de son partenaire de liaison.

10. Méthode suivant l'une quelconque des revendications précédentes, dans laquelle l'étape de contrôle ou de détection comprend la mesure d'une variation de transfert d'énergie entre un marqueur fluorescent présent sur le domaine de liaison naturel et un marqueur fluorescent présent sur le partenaire de liaison.

**Figure 1**

**Figure 2**

**Figure 3**

**Figure 4**

**Figure 5**

**Figure 6**

**Figure 7**

**Figure 8**

**Figure 9**

**Figure 10**

**Figure 11**

**Figure 12**

**Figure 13**

**Figure 14**

**Figure 15**

**Figure 16**

FIG. 17

FIG. 18

EXCIMER CONFIGURATION OF PYRENE PAIRS

FIG. 19